# EUROPEAN PATENT SPECIFICATION

(11) **EP 1 660 884 B1**
(45) Date of publication and mention of the grant of the patent: **23.05.2012**
(21) Application number: 03770230.5
(22) Date of filing: 04.08.2003
(51) Int. Cl.: C07D 273/00, C07H 21/00, C09B 57/10, C09B 69/00, G01N 33/58, G01N 33/84, G01N 33/53, A61K 49/00

(54) **CROWN ETHER DERIVATIVES**
KRONENETHER-DERIVATE
DÉRIVÉS D'ÉTHER COURONNE

(43) Date of publication of application: 31.05.2006
(73) Proprietor: Life Technologies Corporation, Carlsbad, CA 92008 (US)
(72) Inventor: GEE, Kyle, Springfield, OR 97478 (US); MARTIN, Vladimir, Eugene, OR 97402 (US)
(74) Representative: Wells, Andrew
(86) International application number: PCT/US2003/024662
(87) International publication number: WO 2005/016874

(56) References cited:
- US-A- 4 843 158
- US-A- 5 096 831
- US-A- 5 134 232
- US-A- 5 405 975
- US-A- 5 773 227
- US-A- 6 124 135
- US-A1- 2002 164 616
- US-A1- 2004 096 978

## Description

### INTRODUCTION

### Field of the Invention

The invention relates to crown ether chelating compounds, including chromophoric and fluorescent compounds that are useful for chelating metal cations. Where the chelator is labeled with a fluorophore, it is an indicator useful for the detection, discrimination and quantification of metal cations. The chelators are optionally substituted one or more times with a chemically reactive group or a conjugated substance, such as a biological or nonbiological polymer, or a lipid.

The present invention is defined in the appended claims. Subject-matters which are not encompassed by the scope of the claims do not form part of the present invention.

### Background of the invention

Metal ions play an important role in biological systems. Cells utilize metal ions for a wide variety of functions, such as regulating enzyme activity, protein structure, cellular signaling, as catalysts, as templates for polymer formation and as regulatory elements for gene transcription. Metal ions can also have a deleterious effect when present in excess of bodily requirements or capacity to excrete. A large number of natural and synthetic materials are known to selectively or non-selectively bind to or chelate metal ions. Ion chelators are commonly used in solution for in vivo control of ionic concentrations and detoxification of excess metals, and as in vitro buffers. When bound to a fluorophore, ion chelators are typically used as optical indicators of ions and are useful in the analysis of cellular microenvironments or dynamic properties of proteins, membranes and nucleic acids.

Such indicators are also useful for measuring ions in extracellular spaces; in vesicles; in vascular tissue of plants and animals; biological fluids such as blood and urine; in fermentation media; in environmental samples such as water, soil, waste water and seawater; and in chemical reactors. Optical indicators for ions are important for qualitative and quantitative determination of ions, particularly in living cells. Fluorescent indicators for metal cations also permit the continuous or intermittent optical determination of these ions in living cells, and in solutions containing the ions.

A variety of fluorescent indicators that are useful for the detection of biologically relevant soluble free metal ions (such as Ca²⁺, Mg⁺ and Zn²⁺) have been described that utilize oxygen-containing anionic or polyanionic chelators to bind to metal ions. In particular, fluorescent indicators utilizing a polycarboxylate BAPTA chelator have been previously described (U.S. Patent No.: 4,603,209 to Tsien et al. (1986); U.S. Patent No. 5,049,673 to Tsien et al. (1991); U.S. Patent No. 4,849,362 to DeMarinis et al. (1989); U.S. Patent No. 5,453,517 to Kuhn et al. (1995); U.S. Patent No. 5,501,980 to Malekzadeh et al. (1996); U.S. Patent No. 5,459,276 to Kuhn et al. (1995); U.S. Patent No. 5,501,980 to Katerinopoulos et al. (1998); U.S. Patent No. 5,459,276 to Kuhn et al. (1995). Some fluorescent indicators selective for Li⁺, Na⁺ and K⁺ in aqueous or organic solution have also been described, based on the chemical modification of crown ethers (U.S. Patent No. 5,134,232; and U.S. Patent No. 5,405,975; Gromov et al, Russian Chemical Bulletin (1999) 48:6 p. 1190-1192; Lockhart et al, J.C.S. Perkin I (1977) p 202-204).

US2002/0164616 discloses crown ether chelators including chromic and fluorescent derivatives, that are useful for chelating metal cations. The compounds of this document differ from the compounds of the present application in one nitrogen atom in the heterocyclic ring, and in additional fused benzo moiety.

In general, a useful property for metal ion indicators is the ability to detect and/or quantify a selected metal ion in the presence of other metal ions. Discrimination of Ca²⁺, Na⁺ and K⁺ ions in the presence of other metal ions is particularly useful for certain biological or environmental samples. For most biological applications, it is essential that the indicators be effective in aqueous solutions. It is also useful that indicators for biological applications be relatively insensitive to pH changes over the physiological range (pH 6-8) and sensitive to ion concentrations in the physiological range (for sodium, a K_{d} of about 5 mM to about 20 mM). It is also beneficial if the indicator absorbs and emits light in the visible spectrum where biological materials have low intrinsic absorbance or fluorescence.

Also useful are chelators that possess a chemically reactive functional group, so that the chelating group can be attached to polymers for use in remote sensing of ions or enhancing the solubility or localization of the optical sensor. Many chelators bind to intracellular proteins, altering the chelator's metal binding properties. In addition, due to their relatively small size, they are readily sequestered non-selectively in intracellular vesicles, further limiting their effectiveness. One means of circumventing these problems is to attach the desired crown ether to a large, water-soluble polysaccharide, such as dextran or FICOL, by means of modification of the polysaccharide to allow covalent attachment of the indicator. Dextrans and FICOLs are especially suitable for this application, as they are low cost, optically transparent above about 250 nm and available in multiple ranges of molecular weights. Furthermore, polysaccharides and their conjugates are reasonably compatible with most biological materials and do not interact significantly with intracellular components. Although fluorescent polysaccharides have been previously described, as have indicator conjugates of dextrans, none possess the advantageous properties of the indicator conjugates of the current invention.

The crown ether chelators of the invention show significant ability to discriminate between metal ions under physiological conditions, particularly Ca²⁺, Na⁺ and K⁺ ions. This selectivity can be tailored by careful selection of crown ether substituents. The compounds of the invention are typically soluble in aqueous solutions.

The compounds of the invention that act as indicators for target ions absorb and emit light in the visible spectrum and possess significant utility as a means of detecting and quantifying certain metal ion levels in living cells, biological fluids or aqueous solutions. Upon binding the target ion in the chelating moiety of the indicator, the optical properties of the attached fluorophore are generally affected in a detectable way, and this change is correlated with the presence of the ion according to a defined standard. Compounds having relatively long wavelength excitation and emission bands can be used with a variety of optical devices and require no specialized (quartz) optics, such as are required by indicators that are excited or that emit at shorter wavelengths. These indicators are suitable for use in fluorescence microscopy, flow cytometry, fluoroscopy, or any other application that currently utilize fluorescent metal ion indicators.

### SUMMARY OF THE INVENTION

The present invention provides metal chelating compounds as defined in the present claim that are crown ether chelating compounds that bind many metal cations including physiological relevant levels of metal cations such as sodium. These metal chelating compounds find utility in detecting, quantitating and monitoring cations such as Na⁺, Li⁺, K⁺, Ca²⁺, Zn²⁺ and Rb⁺. A particular useful application is the binding of physiological levels of sodium ions in living cells wherein the compounds of the present invention provide for the detection, quantitation and monitoring of the intracellular sodium ions.

According to a first aspect of of the present invention, there is provided a crown ether chelating compound having the formula: 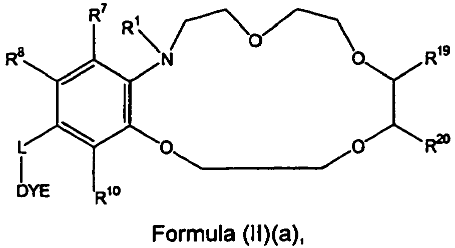 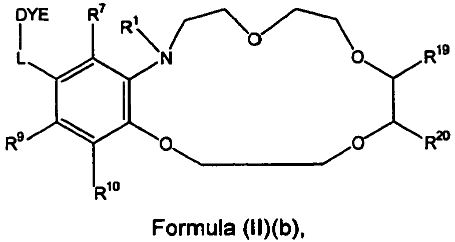 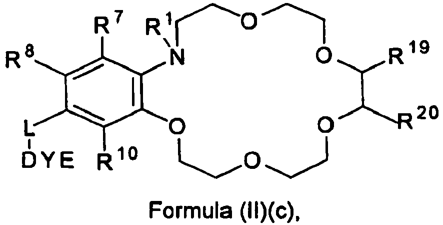 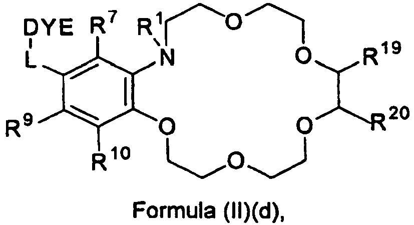 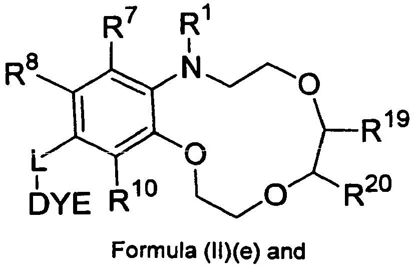 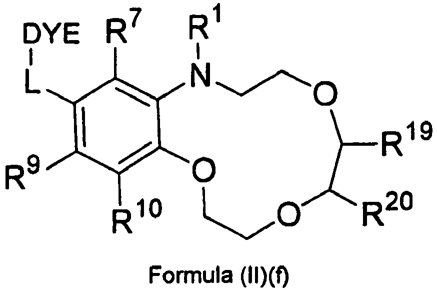 wherein
R¹ is C₁-C₈ alkyl that is substituted one or more times by amino (-NR¹⁷R¹⁸),(C=O)-O-R¹⁸ or -(C=O)-NR¹⁷R¹⁸;
R⁷, R⁸, R⁹, and R¹⁰, when present, are H;
R¹⁹ and R²⁰ are both H;
R¹⁸ is selected from the group consisting of H, C₁-C₈ alkyl, benzyl, a biologically compatible esterifying group, a biologically compatible salt, -L-R_{X}, -L-S_{C} and -L-DYE;
R¹⁷ and R¹⁸ are independently selected from the group consisting of H, C₁-C₆ alkyl, C₁-C₆ carboxyalkyl, alpha-acyloxyalkyl, trialkylsilyl, a biologically compatible salt, -L-R_{X}, -L-S_{C} and -L-DYE; or R¹⁷ and R¹⁸ taken in combination form a 5- or 6-membered aliphatic ring that optionally incorporates an oxygen atom;
each L is independently a covalent linkage;
each R_{X} is independently a reactive group;
each S_{C} is independently a conjugated substance; and
each DYE is independently a reporter molecule.

Crown ether compounds that contain an oxygen and nitrogen atom ortho to the benzo moiety find particular use in binding sodium ions and in generating a detectable signal when bound by an -L-DYE moiety at one of the benzo substitutents.

Rₓ is a reactive group that is capable of forming a covalent bond with another substance containing an appropriate reactive group to form a conjugated substance (S_{c}). Particularly useful reactive groups of the metal chelating compounds include carboxylic acid and activated esters of carboxylic acid for labeling amines and alcohols of biomolecules. Conjugated substances are intended to mean any biomolecule or non-biomolecule that contains a moiety capable of forming a covalent linkage with another moiety or is modified to contain such a reactive group. Particularly useful conjugated substances include proteins, peptides and non-biomolecule polymers. The covalent linkage (L) can be a single covalent bond or a series of stable bonds containing 1-20 non-hydrogen atoms including P, C, N. O and S.

A particularly preferred R¹ is represented by methyl or ethyl that is substituted by -(C=O)-OR¹⁶ wherein R¹⁶ is a methyl group. These particular substitutents appear to play a role in stabilizing the metal ion in the chelating ring of the present compounds.

When a lipiphilic group such as an AM ester substitutes the DYE moiety the present compounds find use in binding *in vivo* metal cations. This is a particulary useful aspect of the present invention wherein certain embodiments of the compounds, such as compounds containing only one nitrogen atom and one benzo moiety, enter cells and bind target ions with better results compared to similar compounds.

The compounds of the present invention are useful for binding physiological relevant levels of cations, particularly sodium ions. When the present compounds comprise a DYE moiety the compounds find use to monitor, detect and quantitate such cations. Furthermore, compounds comprising a lipophilic group such as an AM or acetate ester group provide compounds that are cell permeable but are well retained in the cell after the lipophilic group is cleaved by nonspecific esterases resulting in a charged molecule.

According to a second aspect of the present invention, there is provided a composition comprising:
a. a compound according to the first aspect; and,
b. a metal ion that is capable of being chelated by said compound.

According to a third aspect of the present invention, there is provided a method for binding and detecting target ions in a live cell, comprising:
a) contacting a sample of live cells with a crown ether compound according to the first aspect with the proviso that said compound comprise a DYE moiety and at least one lipohilic group;
b) incubating said sample and said crown ether chelate compound for sufficient time to allow said compound to chelate said target metal ion; and,
c) illuminate said sample with an appropriate wavelength whereby said target ion is detected in a live cell.

According to a fourth aspect of the present invention, there is provided a kit for binding a metal ion in a sample, comprising:
a compound according to the first aspect; and, comprising one or more components selected from the group consisting of a calibration standard of a metal ion, an ionophore, a fluorescent standard, an aqueous buffer solution and an organic solvent.

According to a fifth aspect of the present invention, there is provided a use of a compound of the first aspect as a metal ion chelator.

### BRIEF DESCRIPTION OF THE FIGURES

**Figure 1****:** Shows by way of background the Na⁺-dependent fluorescence excitation spectra of Compound **51** (which is outside the scope of the present invention) in a series of solutions containing 0 to 1000 mM free Na⁺, with fluorescence emission monitored at 510 nm (as described in Example 2).

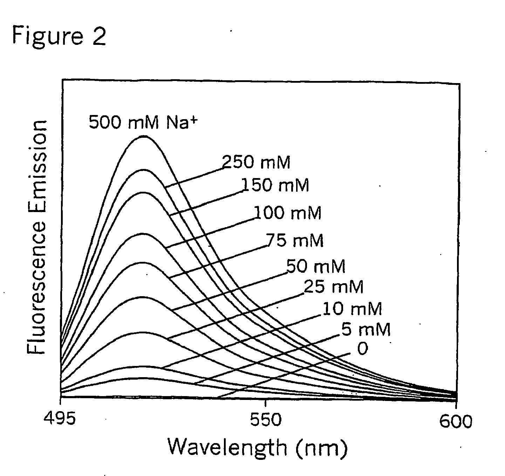

**Figure 2****:** Shows by way of background the Na⁺-dependent fluorescence emission spectra of Compound **22** (which is outside the scope of the present invention) in a series of solutions containing 0 to 500 mM free Na⁺, with excitation at 488 nm (as described in Example 2).
**Figure 3****:** Shows by way of background a comparison of the intracellular sodium response of SODIUM GREEN tetraacetate indicator (Molecular Probes, Inc.) or Compound 25 (which is outside the scope of the present invention). At every intracellular Na⁺ concentration, Compound **25** demonstrates stronger fluorescence intensity than SODIUM GREEN sodium indicator at the same concentration (as described in Example 3).
**Figure 4****:** Shows the Na+ (Fig. 4A) and K+ (Fig. 4B)-dependent fluorescent emission spectra of Compound **86** in a series of solutions containing 0 to 1000mM free Na+ and 0 to 5M of free K+ ions, with excitation at 488nm. *See,* Example 2.

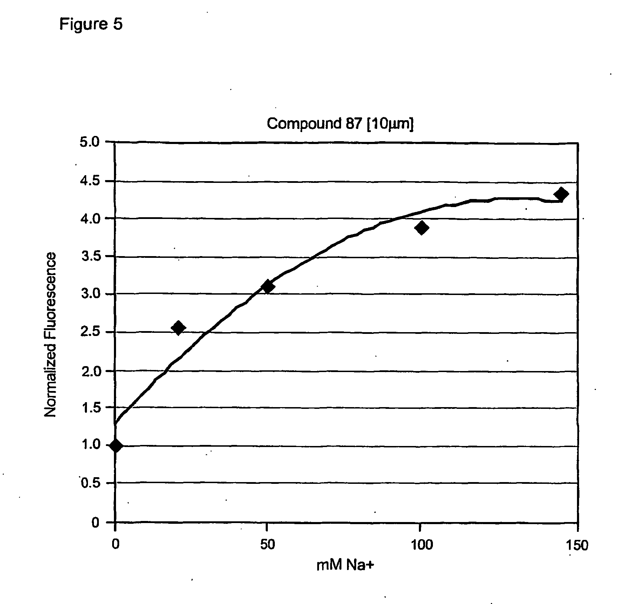

**Figure 5****:** Shows intracellular detection of Na+ ions with Compound **87.** *See,* Example 3.

### DETAILED DESCRIPTION OF THE INVENTION

### I. DEFINITIONS

Before describing the present invention in detail, it is to be understood that this invention is not limited to specific compositions or process steps, as such may vary. It must be noted that, as used in this specification and the appended claims, the singular form "a", "an" and "the" include plural referents unless the context clearly dictates otherwise. Thus, for example, reference to "a fusion protein" includes a plurality of proteins and reference to "a fluorescent compound" includes a plurality of compounds and the like.

Unless defined otherwise, all technical and scientific terms used herein have the same meaning as commonly understood by one of ordinary skill in the art to which this invention is related. The following terms are defined for purposes of the invention as described herein.

The term "affinity" as used herein refers to the strength of the binding interaction of two molecules, such as a metal chelating compound and a metal ion or a positively charged moiety and a negatively charged moiety.

The term "alkyl" as used herein refers to a straight, branched or cyclic hydrocarbon chain fragment containing between about one and about twenty five carbon atoms (e.g. methyl, ethyl and the like). Straight, branched or cyclic hydrocarbon chains having eight or fewer carbon atoms will also be referred to herein as "lower alkyl". In addition, the term "alkyl" as used herein further includes one or more substitutions at one or more carbon atoms of the hydrocarbon chain fragment. Such substitutions include, but are not limited to: aryl; heteroaryl; halogen; alkoxy; amine (-NR'R"); carboxy and thio.

The term "amino" or "amine group" refers to the group -NR'R" (or NRR'R") where R, R' and R" are independently selected from the group consisting of hydrogen, alkyl, substituted alkyl, aryl, substituted aryl, aryl alkyl, substituted aryl alkyl, heteroaryl, and substituted heteroaryl. A substituted amine being an amine group wherein R' or R" is other than hydrogen. In a primary amino group, both R' and R" are hydrogen, whereas in a secondary amino group, either, but not both, R' or R" is hydrogen. In addition, the terms "amine" and "amino" can include protonated and quaternized versions of nitrogen, comprising the group -NRR'R" and its biologically compatible anionic counterions.

The term "aryl" as used herein refers to cyclic aromatic carbon chain having twenty or fewer carbon atoms, e.g., phenyl, naphthyl, biphenyl, and anthracenyl. One or more carbon atoms of the aryl group may also be substituted with, e.g., alkyl; aryl; heteroaryl; a halogen; nitro; cyano; hydroxyl, alkoxyl or aryloxyl; thio or mercapto, alkyl-, or arylthio; amino, alkylamino, arylamino, dialkyl-, diaryl-, or arylalkylamino; aminocarbonyl, alkylaminocarbonyl, arylaminocarbonyl, dialkylaminocarbonyl, diarylaminocarbonyl, or arylalkylaminocarbonyl; carboxyl, or alkyl- or aryloxycarbonyl; aldehyde; aryl- or alkylcarbonyl; iminyl, or aryl- or alkyliminyl; sulfo; alkyl- or alkylcarbonyl; iminyl, or aryl- or alkyliminyl; sulfo; alkyl- or arylsufonyl; hydroximinyl, or aryl- or alkoximinyl. In addition, two or more alkyl or heteroalkyl substituents of an aryl group may be combined to form fused aryl-alkyl or aryl-heteroalkyl ring systems (e.g., tetrahydronaphthyl). Substituents including heterocyclic groups (e.g., heteroaryloxy, and heteroaralkylthio) are defined by analogy to the above-described terms.

The term "arylalkyl" as used herein refers to an aryl group that is joined to a parent structure by an alkyl group as described above, e.g., benzyl, α-methylbenzyl, phenethyl, and the like.

The term "attachment site" as used herein refers to a site on a moiety or a molecule, e. g. a metal chelating compound, a fluorescent dye, a peptide or a protein, to which is covalently attached, or capable of being covalently attached, to a linker or another moiety.

The term "aqueous solution" as used herein refers to a solution that is predominantly water and retains the solution characteristics of water. Where the aqueous solution contains solvents in addition to water, water is typically the predominant solvent.

The term "cell permeable" as used herein refers to compounds of the present invention that are able to cross the cell membrane of live cells. Lipophilc groups that are covalently attached to the present compounds, typically on the DYE moiety, facilitate this permeability and live cell entry. Once inside the cells, the lipophilic groups are hydrolyzed resulting in charged molecules that are well retained in living cells. Particularly useful lipophilic groups include acetoxymethyl (AM) ester and acetate esters wherein once inside the cells the groups are cleaved by nonspecific esterases resulting in charged molecules.

The term "complex" as used herein refers to the association of two or more molecules, usually by non-covalent bonding.

The term "detectable response" as used herein refers to a change in or an occurrence of, a signal that is directly or indirectly detectable either by observation or by instrumentation and the presence or magnitude of which is a function of the presence of a target metal ion in the test sample. Typically, the detectable response is an optical response resulting in a change in the wavelength distribution patterns or intensity of absorbance or fluorescence or a change in light scatter, fluorescence quantum yield, fluorescence lifetime, fluorescence polarization, a shift in excitation or emission wavelength or a combination of the above parameters. The detectable change in a given spectral property is generally an increase or a decrease. However, spectral changes that result in an enhancement of fluorescence intensity and/or a shift in the wavelength of fluorescence emission or excitation are also useful. The change in fluorescence on ion binding is usually due to conformational or electronic changes in the indicator that may occur in either the excited or ground state of the fluorophore, due to changes in electron density at the ion binding site, due to quenching of fluorescence by the bound target metal ion, or due to any combination of these or other effects. Alternatively, the detectable response is an occurrence of a signal wherein the fluorophore is inherently fluorescent and does not produce a change in signal upon binding to a metal ion or biological compound.

The term "DYE" as used herein refers to a reporter molecule that is inherently fluorescent or demonstrates a change in fluorescence upon binding to a biological compound or metal ion, i.e., fluorogenic. Numerous fluorophores are known to those skilled in the art and include, but are not limited to, coumarin, acridine, furan, indole, borapolyazaindacene, cyanine, benzofuran, quinazolinone, benzazole, oxazine and xanthenes including fluoroscein, rhodamine, rosamine and rhodol, as well as other fluorophores described in RICHARD P. HAUGLAND, MOLECULAR PROBES HANDBOOK OF FLUORESCENT PROBES AND RESEARCH PRODUCTS (9th edition, CD-ROM, 2002). The DYE moiety may be substituted by substituents that enhance solubility, live cell permeability and alter spectra absorption and emission.

The term "heteroaryl" or "heteroaromatic ring system" as used herein refers to a 5- or 6-membered unsaturated ring that is optionally fused to an additional six-membered aromatic ring(s), or is fused to one 5- or 6-membered unsaturated ring containing one or more heteroatoms, and is optionally substituted as defined below. Each heteroaromatic ring contains at least 1 and as many as 3 heteroatoms that are selected from the group consisting of O, N or S in any combination. Specific examples of a heteroaryl ring system include, but are not limited to, substituted or unsubstituted derivatives of 2- or 3-furanyl; 2- or 3-thienyl; N-, 2- or 3-pyrrolyl; 2- or 3-benzofuranyl; 2- or 3-benzothienyl; N-, 2- or 3-indolyl; 2-, 3- or 4-pyridyl; 2-, 3- or 4-quinolyl; 1-, 3-, or 4-isoquinolyl; 2-, 4-, or 5-(1,3-oxazolyl); 2-benzoxazolyl; 2-, 4-, or 5-(1,3-thiazolyl); 2-benzothiazolyl; 3-, 4-, or 5-isoxazolyl; N-, 2-, or 4-imidazolyl; N-, or 2-benzimidazolyl; 1- or 2-naphthofuranyl; 1- or 2-naphthothienyl; N-, 2- or 3-benzindolyl; 2-, 3-, or 4-benzoquinolyl; 1-, 2-, 3-, or 4-acridinyl. Preferably the heteroaryl substituent is substituted or unsubstituted 4-pyridyl, 2-thienyl, 2-pyrrolyl, 2-indolyl, 2-oxazolyl, 2-benzothiazolyl or 2-benzoxazolyl. More preferably, the heteroaryl substituent is 2-thienyl or 2-pyrrolyl.

The term "kit" as used refers to a packaged set of related components, typically one or more compounds or compositions.

The term "Linker" or "L" as used herein refers to a single covalent bond or a series of stable covalent bonds incorporating 1-20 nonhydrogen atoms selected from the group consisting of C, N, O, S and P that covalently attach the crown ether metal chelating compounds to another moiety such as a chemically reactive group, a DYE or a conjugated substance including biological and non-biological substances. A "cleavable linker" is a linker that has one or more covalent bonds that may be broken by the result of a reaction or condition. For example, an ester in a molecule is a linker that may be cleaved by a reagent, e.g. sodium hydroxide, resulting in a carboxylate-containing fragment and a hydroxyl-containing product.

The term "metal chelator" or "metal chelating compound" as used herein refers to a chemical compound that combines with a metal ion to form a chelate ring structure.

The term "metal ion" or "target metal ion" as used herein refers to any metal cation that is capable of being chelated by the present crwon ether chelate compounds. Typically, these metal ions are physiological and or nutritional relevant metal ion such as Na⁺, K⁺, Zn²⁺ and Ca²⁺. The term metal ion used herein also refers to the metal ions Li⁺ and Rb⁺.

The terms "protein" and "polypeptide" are used herein in a generic sense to include polymers of amino acid residues of any length. The term "peptide" is used herein to refer to polypeptides having less than 250 amino acid residues, typically less than 100 amino acid residues. The terms apply to amino acid polymers in which one or more amino acid residues are an artificial chemical analogue of a corresponding naturally occurring amino acid, as well as to naturally occurring amino acid polymers.

The term "Rx" or "reactive group" as used herein refers to a group that is capable of reacting with another chemical group to form a covalent bond, i.e. is covalently reactive under suitable reaction conditions, and generally represents a point of attachment for another substance. The reactive group is a moiety, such as carboxylic acid or succinimidyl ester, on the compounds of the present invention that is capable of chemically reacting with a functional group on a different compound to form a covalent linkage. Reactive groups generally include nucleophiles, electrophiles and photoactivatable groups.

The term "reporter molecule" as used herein refers to a chemical moiety that when covalently attached to the present crown ether compound is capable of generateing a detectable reposnse. Typically the reporter moleule is a DYE moiety.

The term "sample" as used herein refers to any material that may contain target metal ions, as defined above. Typically, the sample is a live cell or a biological fluid that comprises endogenous host cell proteins. Alternatively, the sample may be a buffer solution or an environmental sample containing target metal ions. The sample may be in an aqueous solution, a viable cell culture or immobilized on a solid or semi solid surface such as a polyacrylamide gel, membrane blot or on a microarray.

### II. COMPOSITIONS AND METHODS OF USE

### A. Components of the Crown Ether Chelate Compounds

The present invention provides crown ether chelating compounds that bind a wide range of metal cations including physiological relevant levels of metal cations such as sodium. These metal chelating compounds comprise a crown ether moiety, at least one benzo moiety, substituents well known in the art including linkers, chemically reactive groups and DYE moieties that function as reporter groups. The crown either moiety contains at least four heteroatoms, one of which is required to be a nitrogen atom and is located ortho to the benzo moiety. The remaining heteratoms may be selected from the group consisting of nitrogen, oxygen and sulfur and are selected based on their ability to bind different metal cations with different affinity. Typically, in addition to the nitrogen atom there is also an oxygen atom ortho to the benzo moiety that faciliates binding of target metal ions. Furthermore, the substituents on the nitrogen atom are further used to alter the affinity for particular metal ions under different environmental conditions.

The present compounds find utility in binding target metal ions in a sample. The sample includes live cells or a biological fluid that comprises endogenous host cell proteins, buffer solutions and environmental samples. Therefore, when the present crown ether compounds comprise a DYE moiety they find utility in quantitating, monitoring and detecting target metal ions. Typically, the DYE moiety is directly attached to the benzo moiety or two of the benzo substituents when taken in combination form a fused DYE moiety. Detection of target metal ions can also be accomplished in live cells wherein the DYE moiety comprises a lipophilic group such as an AM or acetate ester that allows for entry across the live cell membrane. Once inside the cells nonspecific esterases cleave the AM or acetate ester resulting a charged molecule that is well retained in the cell. These present compounds are particularly useful for binding physiological relevant levels of sodium, potassium or calcium cations.

### 1. Chelating Moiety

The present disclosure is based on investigations carried out by the inventors in relation to compounds of Formula (I) and Formula (II): 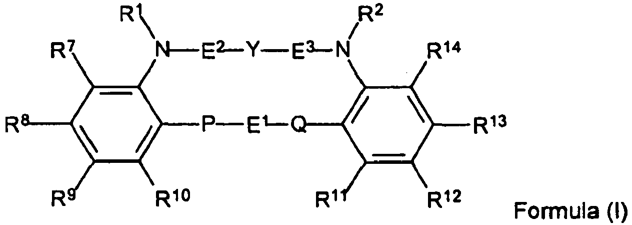 or 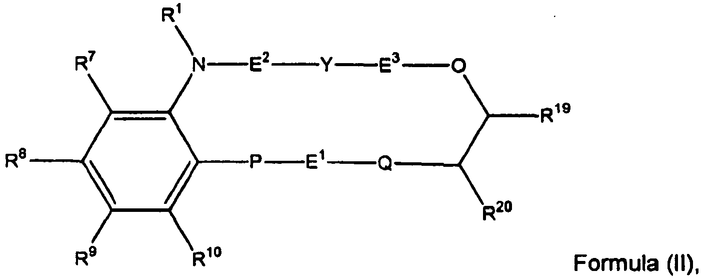 wherein the following further definitions apply.

The heteroatom Y is O, S, NR⁴ or is absent where R⁴ is H, a C₁-C₁₈ alkyl, or an aryl or heteroaryl ring system. The R⁴ alkyl or ring system substituent is optionally substituted one or more times by halogen, azido, nitro, nitroso, amino, alkylamino having 1-6 carbons, dialkylamino having 2-12 carbons, cyano, or R⁴ is substituted one or more times by a C₁-C₆ alkyl or C₁-C₆ alkoxy that is itself optionally substituted one or more times by halogen, amino, hydroxy, -(SO₂)-R¹⁵, -(SO₂)-O-R¹⁵, -(C=O)-R¹⁵, -(C=O)-O-R¹⁶, or -(C=O)-NR¹⁷R¹⁸. Alternatively, R⁴ is, or is substituted by -L-Rₓ. -L-S_{c}, or -L-DYE.

Y may be O. Y may be absent provided that E² is also absent.

R¹⁵ is independently H or C₁-C₆ alkyl. Each R¹⁸ is H, a C₁-C₈ alkyl, a benzyl, or forms an ester (e.g., R¹⁸ is an alpha-acyloxyalkyl, a trialkylsilyl, or any other biologically compatible esterifying group). Additionally, any R¹⁶ is a biologically compatible salt. R¹⁷ and R¹⁸ are independently H or C₁-C₈ alkyl or C₁-C₈ carboxyalkyl, or an alpha-acyloxyalkyl, trialkylsilyl, or any other biologically compatible esterifying group, or a biologically compatible salt. Alternatively, R¹⁷ and R¹⁸ when taken in combination form a 5- or 6-membered aliphatic ring that optionally incorporates an oxygen atom. In addition, one or more of a R¹⁵, R¹⁸, R¹⁶, or R¹⁸ is permitted to be -L-R_{X}, -L-S_{C}, or -L-DYE.

Each L is independently a covalent linkage. Each R_{X} is independently a chemically reactive group. Each S_{C} is independently a conjugated substance. Each DYE is independently a reporter molecule that is a chromophore that maximally absorbs light at a wavelength greater than 320 nm.

The heteroatoms P and Q are independently selected from O, S, or NR³, where each R³ is independently H or an alkyl having 1-6 carbons. In one aspect of the invention P and Q are both O.

In another aspect of the invention, P and Q are O, and Y is NR⁴. Typically, P, Q, and Y are each O or Y is absent and P and Q are O. Careful selection of the nature of the P, Q, and Y heteroatoms permits the moderation of the selectivity and binding affinity of the resulting crown ether compound.

E¹, E², and E³ each independently have the formula -(CR⁵₂)ₙ-, or -[C(O)CH₂]ₙ-, - (CR⁵₂)ₙO(CR⁵₂)ₙ- or E² is absent where n is 2, 3 or 4. Each R⁵ is independently H or methyl, or the R⁵ moieties on adjacent carbon atoms of each chain, when taken in combination, forms a 5- or 6-membered aliphatic ring. For a given E moiety, each R⁵ is typically H and n is 2. Where n is 2 for each E moiety, the resulting compound is known as a 15-crown-5 crown ether, having 15 atoms in the chelating ring itself, of which 5 are heteroatoms.

Alternatively, E2 is absent or a E moiety is -(CR⁵₂)ₙO(CR⁵₂)ₙ- resulting in a chelating ring with a different number of total atoms and possible heteroatoms such as oxygen.

E¹, E², and E³ may each be -(CH₂)₂- whilst Y, P and Q are each oxygen. Alternatively, E² may be absent whilst P and Q are oxygen wherein Y is absent and E¹ and E³ are each - (CH₂)₂-. Alternatively, at least one of E¹, E², or E³ is -(CR⁵₂)ₙO(CR⁵₂)ₙ wherein Y, P and Q are each oxygen and the remaining E moieties are -(CH₂)₂-.

Formula (I) contains the amine substituents R¹ and R², however Formula (II) contains only R¹, the second nitrogen having been replaced by a divalent oxygen atom. Thus, it is understood that while the amine substitutents, R1 and R2, are referred to in plural only one is intended for compounds represented by formula (II). These amine substituents are independently H, C₁-C₁₈ alkyl, or C₇-C₁₈ arylalkyl. Where R¹ or R² is alkyl or arylalkyl, it is optionally substituted one or more times by halogen, azido, nitro, nitroso, amino, hydroxy, C₁-C₈ alkylamino, C₂-C₁₂ dialkylamino, cyano, or by an aryl or heteroaryl ring system, or by - (SO₂)-R¹⁵, -(SO₂)-O-R¹⁵, -(C=O)-R¹⁵, -(C=O)-O-R¹⁶, or -(C=O)-NR¹⁷R¹⁸; or by a C₁-C₆ alkyl or C₁-C₈ alkoxy that is itself optionally substituted one or more times by halogen, amino, hydroxy, -(SO₂)-R¹⁵, -(SO₂)-O-R¹⁵, -(C=O)-R¹⁵, -(C=O)-O-R¹⁶, or -(C=O)-NR¹⁷R¹⁸. Alternatively, R¹ and R² are optionally -L-R_{X}, -L-S_{C}, or -L-DYE.

Where the R¹ and R² substituents are not -L-R_{X}, -L-S_{C}, or -L-DYE, they are typically both a lower alkyl that is substituted one or more times by carboxylic acids, by carboxylic acid esters, by carboxylic acid amides, or by cyano. Where R¹ and R² incorporate carboxylic acid esters they are typically not cleaved by esterase but instead function to stabilize the bound metal ion in the chelate ring. Thus R¹, and R² when present, are typically a methyl or ethyl that is substituted by a carboxylic acid containing group such as -(C=O)-R¹⁵ or -(C=O)-O-R¹⁸ wherein R¹⁵ and R¹⁶ are each a methyl or ethyl. Selection of the precise nature of R¹ and R² can greatly affect the binding selectivity and affinity of the resulting compound for a target ion (see, Table 1 and 2).

The nature of the R¹ and R² substituents in a large part determines the response of the indicators to particular target metal ions. For example, where the crown ether chelating compounds have the reference formula 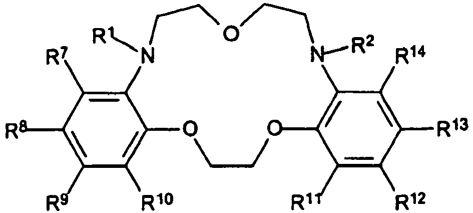 where R¹ and R² are each methoxycarbonylmethyl selectively bind sodium ions with a dissociation constant (K_{d}) of approximately 20-100 mM, and are relatively insensitive to the presence of potassium ions. In particular, the sodium ion K_{d} values typically rise less than about 10% when measured in the presence of 100 mM potassium ion.

The Formula (II) substituents R¹⁹ and R²⁰ are represented by H, halogen, azido, nitro, nitroso, amino, cyano, -L-R_{X}, -L-S_{C}, -L-DYE, C₁-C₆ alkyl and C₁-C₆ alkoxy, each of which is itself optionally substituted by halogen, amino, hydroxy, -(SO₂)-R¹⁵, -(SO₂)-O-R¹⁵, -(C=O)-R¹⁵, -(C=O)-O-R¹⁶, or -(C=O)-NR¹⁷R¹⁸. Alternatively, R¹⁹ and R²⁰ taken in combination form a fused six-membered benzo moiety that is optionally substituted by halogen, azido, nitro, nitroso, amino, cyano, -L-R_{X}, -L-S_{C}, -L-DYE, C₁-C₆ alkyl or C₁-C₆ alkoxy, each of which is itself optionally substituted by halogen, amino, hydroxy, -(SO₂)-R¹⁵, -(SO₂)-O-R¹⁵, -(C=O)-R¹⁵, -(C=O)-O-R¹⁶, or -(C=O)-NR¹⁷R¹⁸. R¹⁹ and R²⁰ may be both hydrogen. Alternatively, R¹⁹ and R²⁰ may form a benzo moiety that is optionally substituted resulting in a crown ether chelate compound with two benzo moieties wherein only one benzo moiety has a nitrogen atom that is ortho to the benzo moiety.

The benzo substituents R⁷-R¹⁰, and R¹¹-R¹⁴ when present, are independently H, halogen, azido, nitro, nitroso, amino, cyano; or -L-R_{X}, -L-S_{C}, or -L-DYE; or C₁-C₆ alkyl or C₁-C₆ alkoxy that is itself optionally substituted one or more times by halogen, amino, hydroxy, -(SO₂)-R¹⁵, -(SO₂)-O-R¹⁵, -(C=O)-R¹⁵, -(C=O)-O-R¹⁸, or -(C=O)-NR¹⁷R¹⁸.

Alternatively, two adjacent substituents of R⁷-R¹⁴, when taken in combination, form a fused six-membered benzo moiety, which is optionally substituted one or more times by a halogen, azido, nitro, nitroso, amino, cyano, -L-R_{X}, -L-S_{C}, or -L-DYE; or by a C₁-C₆ alkyl or C₁-C₆ alkoxy, which is itself optionally substituted one or more times by halogen, amino, hydroxy, - (SO₂)-R¹⁵, -(SO₂)-O-R¹⁵, -(C=O)-R¹⁵, -(C=O)-O-R¹⁸, or -(C=O)-NR¹⁷R¹⁸.

Furthermore, the compounds comprise a fused DYE wherein two adjacent substituents of R⁷-R¹⁴, when taken in combination with each other, and with the aromatic ring they are bound to, form a fused DYE. In this way the benzo moiety of the present compounds is also part of a DYE moiety.

The compounds represented by Formula (I) are substituted by at least one -L-DYE, -L-R_{X} or -L-S_{C} at one or more of R¹, R², R⁴, and R⁷-R¹⁴; or two of R⁷-R¹⁴, taken in combination, form a fused DYE. The compounds may be substituted by exactly one -L-DYE moiety, which is bound at R¹, R², an R⁴, or one of R⁷-R¹⁴, or is a fused DYE moiety at two adjacent substituents of R⁷-R¹⁴. The DYE moiety is typically bound at one of R⁷-R¹¹, preferably at R⁹ or R⁸, or is bound at R⁴ where Y is NR⁴. In one embodiment, compounds that are substituted by exactly one -L-DYE moiety are optionally further substituted by -L-R_{X} or -L-S_{C}, typically at R¹, R², R⁴, or one of R⁷-R¹⁴.

The compound may be substituted by exactly two DYE moieties, which may be the same or different, and may be bound by a covalent linkage L or fused to the crown ether chelate. In one embodiment of the invention, a first -L-DYE moiety is bound at one of R⁷-R¹⁰, while the second -L-DYE moiety is bound at one of R¹¹-R¹⁴. Typically, the first -L-DYE moiety is bound at R⁹, while the second -L-DYE moiety is bound at R¹², or a DYE moiety is fused at R⁸ and R⁹ and additionally at R¹² and R¹³.

Such investigations by the inventors led to the first aspect of the present invention.

### 2. Linkers of the crown ether chelate compounds

The crown ether chelate compounds of the present invention typically comprise a linker that is used to covalently attach a DYE moiety, conjugated substance or reactive group to the compound. When present, the linker is a single covalent bond or a series of stable bonds. Thus, the reporter molecule, conjugated substance or reactive group may be directly attached (where Linker is a single bond) to the crown ether chelate or attached through a series of stable bonds. When the linker is a series of stable covalent bonds the linker typically incorporates 1-20 nonhydrogen atoms selected from the group consisting of C, N, O, S and P. In addition, the covalent linkage can incorporates a platinum atom, such as described in U.S. Patent No. 5,714,327. When the linker is not a single covalent bond, the linker may be any combination of stable chemical bonds, optionally including, single, double, triple or aromatic carbon-carbon bonds, as well as carbon-nitrogen bonds, nitrogen-nitrogen bonds, carbon-oxygen bonds, sulfur-sulfur bonds, carbon-sulfur bonds, phosphorus-oxygen bonds, phosphorus-nitrogen bonds, and nitrogen-platinum bonds. Typically the linker incorporates less than 15 nonhydrogen atoms and are composed of any combination of ether, thioether, thiourea, amine, ester, carboxamide, sulfonamide, hydrazide bonds and aromatic or heteroaromatic bonds. Typically the linker is a single covalent bond or a combination of single carbon-carbon bonds and carboxamide, sulfonamide or thioether bonds. The bonds of the linker typically result in the following moieties that can be found in the linker: ether, thioether, carboxamide, thiourea, sulfonamide, urea, urethane, hydrazine, alkyl, aryl, heteroaryl, alkoky, cycloalkyl and amine moieties. Examples of L include substituted or unsubstituted polymethylene, arylene, alkylarylene, arylenealkyl, or arylthio.

In one embodiment, L contains 1-6 carbon atoms; in another, L comprises a thioether linkage. In another embodiment, L is or incorporates the formula -(CH₂)_{d}(CONH(CH₂)ₑ)_{z}- or -O(CH₂)_{d}(CONH(CH₂)ₑ)_{z}-, where d is an integer from 0-5, e is an integer from 1-5 and z is 0 or 1. In a further embodiment, L is or incorporates the formula -O-(CH₂)-. In yet another embodiment, L is or incorporates a phenylene or a 2-carboxy-substituted phenylene.

Any combination of linkers may be used to attach the DYE, Rx or Sc and the crown ether chelate together, typically a compound of the present invention when attached to more than one DYE, Rx or Sc will have one or two linkers attached that may be the same or different. The linker may also be substituted to alter the physical properties of the crown ether chelate compound, such as binding affinity of the chelating moiety and spectral properties of the dye.

Another important feature of the linker is to provide an adequate space between the crown ether chelate moiety and the DYE, Rx or Sc so as to prevent these substituents from providing a steric hinderance to the binding of the target metal ion for the chelating moiety of the present compounds. Therefore, the linker of the present compounds is important for (1) attaching DYE, Rx or Sc to the metal chelating moiety, (2) providing an adequate space between DYE, Rx or Sc and the metal chelating moiety so as not to sterically hinder the affinity of the chelating moiety and the zinc ions and (3) for altering the affinity of the chelating moiety for the target ions either by the choice of the atoms of the linker or indirectly by addition of substituents to the linker.

However, it is important to understand that a linker is not an essential component of the present compounds. Depending on the reporter molecule, a linker may not be necessary wherein the reporter molecule shares atoms with the metal chelating moiety, i.e. a fused DYE. A reporter molecule that exemplifies this is the dye benzofuran wherein one of the benzene rings of the dye is also one of the benzene rings of the metal chelating moiety. In addition, compounds represented for Formula (II) may not be substituted by a moiety that incorporates a linker.

### 3. DYE moiety of the crown ether chelate compounds

The DYE moiety of the present invention functions as a reporter molecule to confer a detectable signal, directly or indirectly, to the target metal ions. This results in the ability to detect, monitor and quantitate target metal ions in a sample.

The DYE moiety includes without limit a fluorophore, a chromophore, a fluorescent protein and an energy transfer pair. When the DYE moiety is a chromophore the crown ether chelate compounds are chromogenic indicators, or more preferably, the DYE moiety is a fluorophore, resulting in a compound that is a fluorogenic indicator for target ions, preferably sodium ions. Therefore, binding a sodium ion with a crown ether compound of the resent invention results in a detectable optical response that can be correlated to the presence of sodium ions.

Where the detectable response is a fluorescence response, it is typically a change in fluorescence, such as a change in the intensity, excitation or emission wavelength distribution of fluorescence, fluorescence lifetime, fluorescence polarization, or a combination thereof. Preferably, the detectable optical response upon binding a target sodium ion is a change in fluorescence intensity that is greater than approximately 10-fold, more preferably greater than 50-fold, and most preferably more that 100-fold. This large increase in fluorescent signal over baseline has not been previously observed with other sodium indicators that comprise a different metal chelating moiety. In another aspect, the detectable optical response upon binding the target metal ion is a shift in maximal excitation or emission wavelength that is greater than about 20 nm, more preferably greater than about 30 nm. Sodium and potassium indicators of the type that exhibit significant excitation and/or emission shifts have not been previously described.

The DYE moiety is any chemical moiety that exhibits an absorption maximum beyond 320 nm, that is bound to the crown ether chelate by a covalent linkage L, or that is fused to the crown ether chelate. A preferred embodiment for detecting sodium ions in live cells is a fluorogenic crown ether chelate compound wherein the DYE moiety is substituted with a lipophilic group. As described above, the covalent linkage can be a single covalent bond or a combination of stable chemical bonds. The covalent linkage binding the DYE moiety to the crown ether chelator is typically a single bond, but optionally incorporates 1-20 nonhydrogen atoms selected from the group consisting of C, N, O, P, and S.

A wide variety of chemically reactive fluorescent dyes that may be suitable for incorporation into the compounds of the invention are already known in the art (RICHARD P. HAUGLAND, MOLECULAR PROBES HANDBOOK OF FLUORESCENT PROBES AND RESEARCH PRODUCTS (*Supra*); BIOPROBES 32 (December 1999); BIOPROBES 33 (February 2000); BIOPROBES 34 (May 2000); and BIOPROBES 35 (November 2000)). The spectral properties of candidate dyes in solution or when conjugated to proteins such as IgG are known or are readily measured using an absorption spectrometer or a spectrofluorometer.

Thus, the DYE moiety of the present invention include, without limitation; a pyrene, an anthracene, a naphthalene, an acridine, a stilbene, an indole or benzindole, an oxazole or benzoxazole, a thiazole or benzothiazole, a 4-amino-7-nitrobenz-2-oxa-1,3-diazole (NBD), a carbocyanine (including any corresponding compounds in US Serial No. 09/557,275; US Publication Nos. 2002/0077487 and 2002/0064794 and US patent Nos. 6,403,807; 6,348,599; 5,486,616; 5,268,486; 5,569,587; 5,569,766; 5,627,027 and 6,048,982), a carbostyryl, a porphyrin, a salicylate, an anthranilate, an azulene, a perylene, a pyridine, a quinoline, a borapolyazaindacene (including any corresponding compounds disclosed in US Patent Nos. 4,774,339; 5,187,288; 5,248,782; 5,274,113; and 5,433,896), a xanthene (including any corresponding compounds disclosed in U.S. Patent No. 6,162,931; 6,130,101; 6,229,055; 6,339,392; 5,451,343 and US Publication No. 2002/0059684), an oxazine or a benzoxazine, a carbazine (including any corresponding compounds disclosed in US Patent No. 4,810,636), a phenalenone, a coumarin (including an corresponding compounds disclosed in US Patent Nos. 5,696,157; 5,459,276; 5,501,980 and 5,830,912), a benzofuran (including an corresponding compounds disclosed in US Patent Nos. 4,603,209 and 4,849,362) and benzphenalenone (including any corresponding compounds disclosed in US Patent No. 4,812,409) and derivatives thereof. As used herein, oxazines include resorufins (including any corresponding compounds disclosed in 5,242,805), aminooxazinones, diaminooxazines, and their benzo-substituted analogs.

Where the DYE moiety is a xanthene, the dye is optionally a fluorescein, a rhodol (including any corresponding compounds disclosed in US Patent Nos. 5,227,487 and 5,442,045), or a rhodamine (including any corresponding compounds in US Patent Nos. 5,798,276 and 5,846,737). As used herein, fluorescein includes benzo- or dibenzofluoresceins, seminaphthofluoresceins, or naphthofluoresceins. Similarly, as used herein rhodol includes seminaphthorhodafluors (including any corresponding compounds disclosed in U.S. Patent No. 4,945,171). Fluorinated xanthene dyes have been described previously as possessing particularly useful fluorescence properties (Int. Publ. No. WO 97/39064 and U.S. Patent No. 6,162,931).

Alternatively, the DYE moiety is a xanthene that is bound via an L that is a single covalent bond at the 9-position of the xanthene. Preferred xanthenes include derivatives of 3H-xanthen-6-ol-3-one bound at the 9-position, derivatives of 6-amino-3H-xanthen-3-one bound at the 9-position, or derivatives of 6-amino-3H-xanthen-3-imine bound at the 9-position.

Preferred DYE moieties of the present invention include xanthene (including rhodol, fluorescein, rhodamine), benzofuran, indole, carbocyanine, quinazolinone, a benzazole, oxazine, coumarin and borapolyazaindacene. The xanthene dyes of this invention comprise both compounds substituted and unsubstituted on the carbon atom of the central ring of the xanthene by substituents typically found in the xanthene-based dyes such as phenyl and substituted-phenyl moieties. In addition, a preferred DYE moiety includes a xanthene-based moity such as fluorescein that has a lipophilic group substited on the oxygen atom. Most preferred dyes are rhodamine, fluorescein, borapolyazaindacene, indole and benzofuran. The choice of the dye attached to the chelating moiety will determine the crown ether chelate compound's absorption and fluorescence emission properties as well as its live cell properties, i.e. substituted lipophilic groups

Typically the DYE moiety contains one or more aromatic or heteroaromatic rings, that are optionally substituted one or more times by a variety of substituents, including without limitation, halogen, nitro, sulfo, cyano, alkyl, perfluoroalkyl, alkoxy, alkenyl, alkynyl, cycloalkyl, arylalkyl, acyl, aryl or heteroaryl ring system, benzo, or other substituents typically present on chromophores or fluorophores known in the art.

In one aspect of the invention, the DYE moiety has an absorption maximum beyond 480 nm. In a particularly useful embodiment, the DYE moiety absorbs at or near 488 nm to 514 nm (particularly suitable for excitation by the output of the argon-ion laser excitation source) or near 546 nm (particularly suitable for excitation by a mercury arc lamp). The DYE moiety may be a chromophore, resulting in a compound that acts as a chromogenic indicator, or more preferably, DYE is additionally a fluorophore, resulting in a compound that is a fluorescent indicator.

Selected sulfonated DYE moieties also exhibit advantageous properties, and include sulfonated pyrenes, coumarins, carbocyanines, and xanthenes (as described in U.S. Patent Nos. 5,132,432; 5,696,157; 5,268,486; 6,130,101). Sulfonated pyrenes and coumarins are typically excited at wavelengths below about 450 nm (US Patent Nos. 5,132,432 and 5,696,157).

Fluorescent proteins also find use as DYE moieties for the crown ether chelate compounds of the present invention. Examples of fluorescent proteins include green fluorescent protein (GFP) and the phycobiliproteins and the derivatives thereof. The fluorescent proteins, especially phycobiliproteins, are particularly useful for creating tandem dye-reporter molecules. These tandem dyes comprise a fluorescent protein and a fluorophore for the purposes of obtaining a larger Stokes shift, wherein the emission spectra are farther shifted from the wavelength of the fluorescent protein's absorption spectra. This property is particularly advantageous for detecting a low quantity of a target sodium ion in a sample wherein the emitted fluorescent light is maximally optimized; in other words, little to none of the emitted light is reabsorbed by the fluorescent protein. For this to work, the fluorescent protein and fluorophore function as an energy transfer pair wherein the fluorescent protein emits at the wavelength that the acceptor fluorophore absorbs and the fluorophore then emits at a wavelength farther from the fluorescent proteins than could have been obtained with only the fluorescent protein. Alternatively, the fluorophore functions as the energy donor and the fluorescent protein is the energy acceptor. Particularly useful fluorescent proteins are the phycobiliproteins disclosed in US Patents 4,520,110; 4,859,582; 5,055,556 and the fluorophore bilin protein combinations disclosed in US Patent 4,542,104. Alternatively, two or more fluorophore dyes can function as an energy transfer pair wherein one fluorophore is a donor dye and the other is the acceptor dye including any dye compounds disclosed in US Patent Nos. 6,358,684; 5,863,727; 6,372,445; 6,221,606; 6,008,379; 5,945,526; 5,863,727; 5,800,996; 6,335,440; 6,008,373; 6,184,379; 6,140,494 and 5,656,554.

In one aspect of the invention, the compounds of the invention are fluorescent indicators having the following structure: 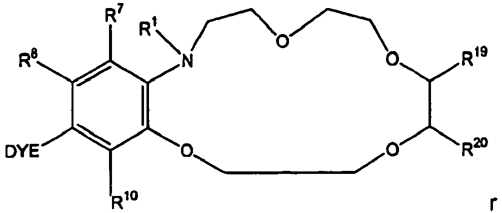 wherein the linker is a single covalent bond. These indicators typically exhibit a low fluorescence quantum efficiency in the absence of metal ions. However, in the presence of increasing metal ion concentration the fluorescence quantum efficiency rises dramatically. For example, selected indicators of this family exhibit a fluorescence signal increase of over 100-times between zero and a saturating sodium concentration. Other selected indicators of the invention exhibit a shift of the wavelength of the absorption (excitation) maximum, emission maximum, or both, upon binding the target ion. It appears that having the DYE moiety covalently attached or fused to the benzo moiety of the crown ether chelate compounds provides an additional channel, in addition to the chelate ring, to conduct electron density changes that occur upon metal binding, resulting in larger optical change. This is true for compounds of the first aspect of the present invention.

In an embodiment of the first aspect of the invention, the crown ether chelate compounds are fluorescent indicators represeted by the following structure: 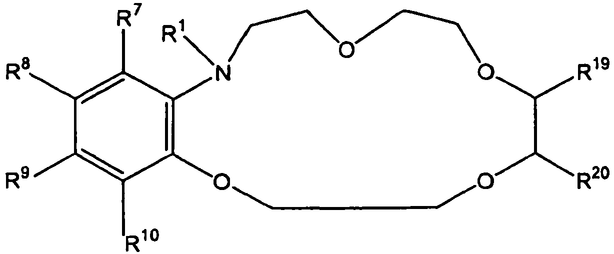 Formula (II)(a and b) wherein Y, P and Q are oxygen and R⁹ or R⁸ is represented by a -L-DYE. Preferrably R⁹ is -L-DYE wherein the linker is typically a single covalent bond and the DYE moiety is selected from the group consisting of borapolyazaindacene, xanthene and indole. Most preferred are xanthene and indole DYE moieties.

A particuary preferred is represented by the following formula: 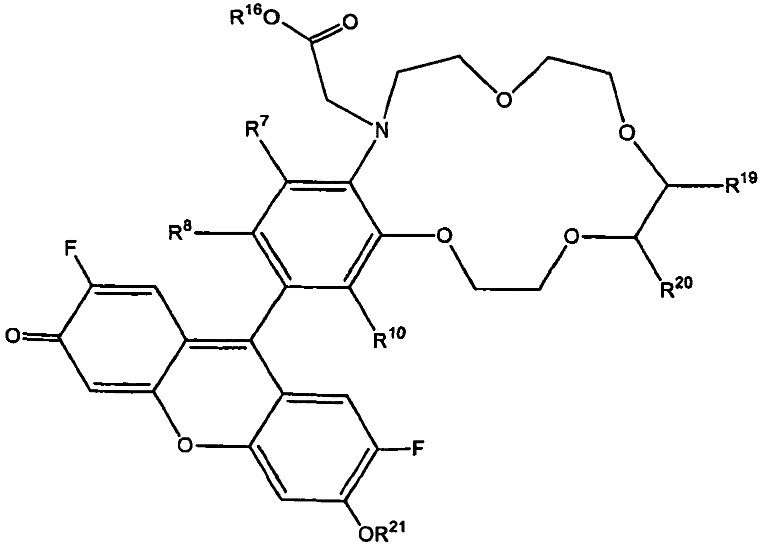 wherein R⁷, R⁸, R¹⁰, R¹⁶, R¹⁷, R¹⁸, L, Rₓ, S_{c}, DYE, R¹⁹, and R²⁰ are as defined in relation to the first aspect of the invention. R¹⁸ may be selected from the group consisting of H, C₁-C₈ alkyl, benzyl, a biologically compatible esterifying group, and a biologically compatible salt. Preferrably R¹⁶ is methyl, a biologically compatible esterifying group or a biologically compatible salt.

R²¹ is selected from the group consisting of H, C₁-C₁₆ alkyl, C₇-C₁₈ arylalkyl and lipophilic group each alkyl is optionally substituted by -(C=O)-R¹⁵, -(C=O)-O-R¹⁶, or C₁-C₈ alkoxy.

Particulary preferred compounds are compounds **86** and **115** and the corresponding cell-permeant versions, compounds **87** and **116** (See, Examples 8, 9, 34 and 35).

In an embodiment of the first aspect of the invention, the crown ether chelate compounds are represented by the formula: 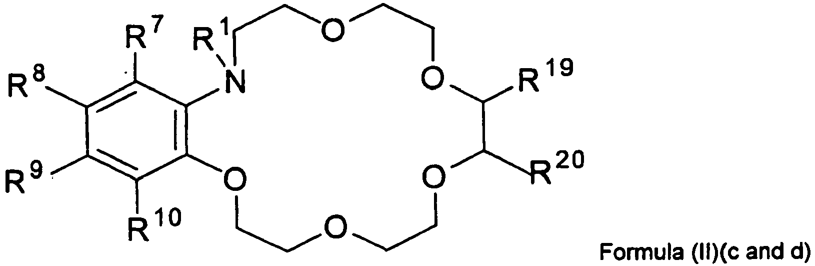 wherein R⁹ or R⁸ are -L-DYE, preferably R⁹ is -L-DYE wherein L is typically a single covalent bond and the DYE moiety is borapolyazaindacene, xanthene or indole. Most preferred are xanthene and indole DYE moieties.

A preferred compound is represented by compound **109** and the cell-permeant version; compound **110** (See, Examples 28 and 29).

In an embodiment of the first aspect of the invention, the crown ether chelate compounds are represented by the formula: 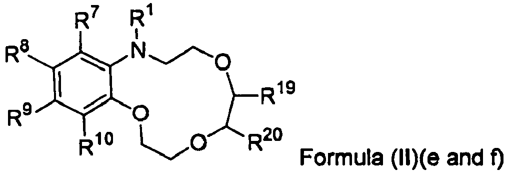 wherein R⁹ or R⁸ are -L-DYE, preferably R⁹ is -L-DYE, wherein L is typically a single covalent bond and the DYE moiety is borapolyazaindacene, xanthene or indole. Most preferred is a xanthene DYE moiety.

Typically R⁷, R⁸ or R⁹, R¹⁰, R¹⁹ and R²⁰ are hydrogen and R¹ is a methyl or ethyl group that is substituted by -(C=O)-R¹⁵ or -(C=O)-O-R¹⁶ wherien R¹⁵ and R¹⁶ are methyl or ethyl.

A preferred embodiment is represented by compound **104** (*See,* Example 23).

The family of crown ether chelate compounds of the first aspect of the invention are paticularly useful for binding physiological relevant levels of metal ions *in vivo* wherein the DYE moiety is substituted by an AM or acetate ester. In addition, this family of compounds unexpectedly cross live cell membranes easier than compounds represented by Formula (I) and are thus preferred for binding of target metal ions in live cells. Furthermore, compounds of the first aspect of the invention, wherein the second nitrogen atom has been replaced by an oxygen atom, unexpectedly resulted in a higher affinity binding of target metal ions such as sodium ions. This, in combination with their ability to effectively load into live cells, provides for unexpected advantages over compounds represented by Formula (I) for *in vivo* binding of target ions.

The family of crown ether chelate compounds according to the first aspect of the invention are also very useful for the binding and detection of target metal ions *in vitro* (See, Table 2). These compounds demonstrate a significant change in fluorescent signal after binding the target metal ions.

Table 1, shows a variety of distinct DYE moieties and crown ether substituents according to Formula (I), which is outside the scope of the claimed invention.

**Table 1: Selected reference examples for Formula (I) compounds.**

| Compound | Dissociation Constant (target ion) |
|---|---|
| | K_{d} (Na⁺) = ∼ 52 mM |
| 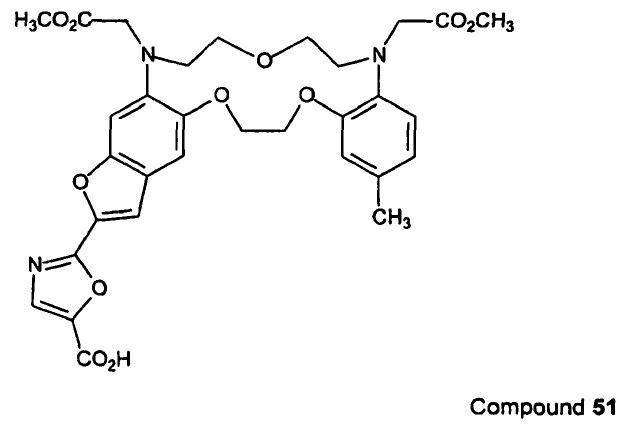 | K_{d} (K^{*}) = ∼ 330 mM |
| | I_{d} (Na⁺) = ∼ 30 mM |
| | K_{d} (K⁺) = ∼ 115mM |
| 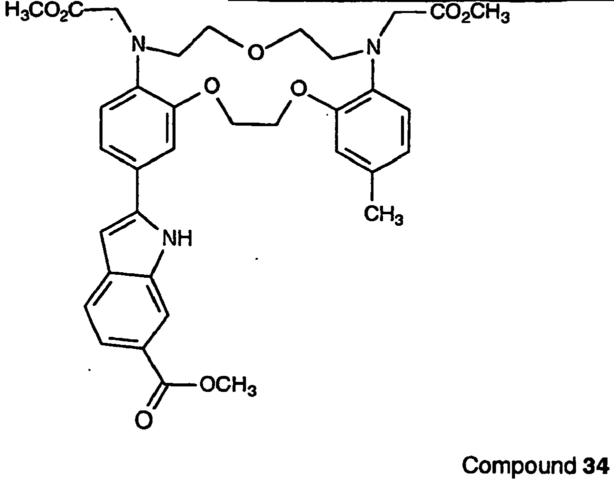 | |
| 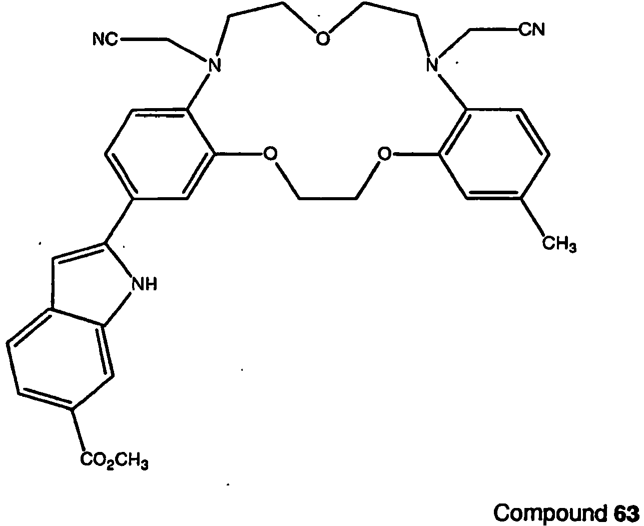 | K_{d} (Na⁺) = ∼ 220 mM |
| 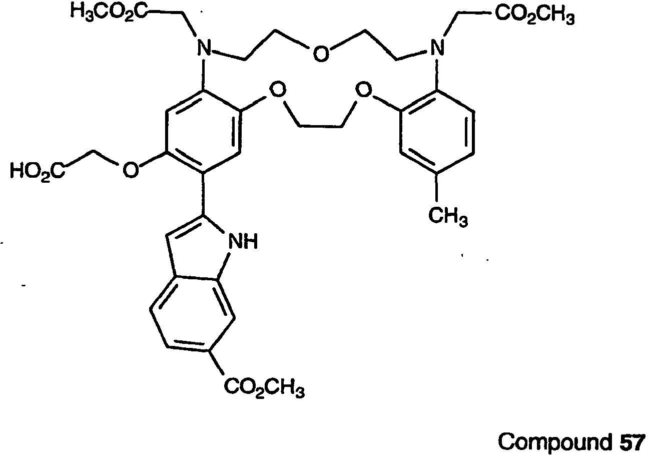 | K_{d} (Na⁺) = ∼ 52 mM K_{d} (K⁺) = ∼ 250 mM |
| 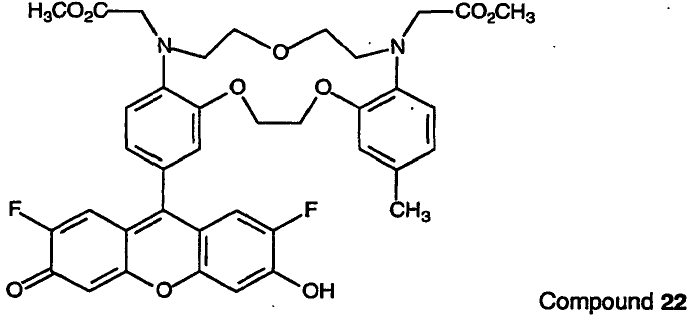 | K_{d} (Na⁺) = ∼ 60 mM K_{d}(K⁺) = ∼ 205 mM |
| 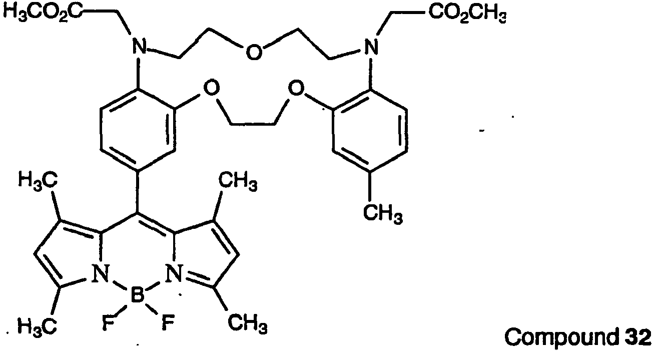 | K_{d} (Na⁺) = ∼ 42 mM |
| 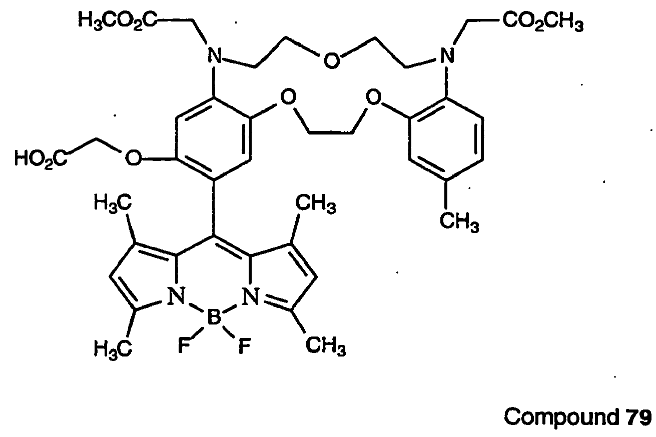 | K_{d} (Na⁺) = ∼ 28 mM K_{d} (K⁺)= ∼ 130mM |
| 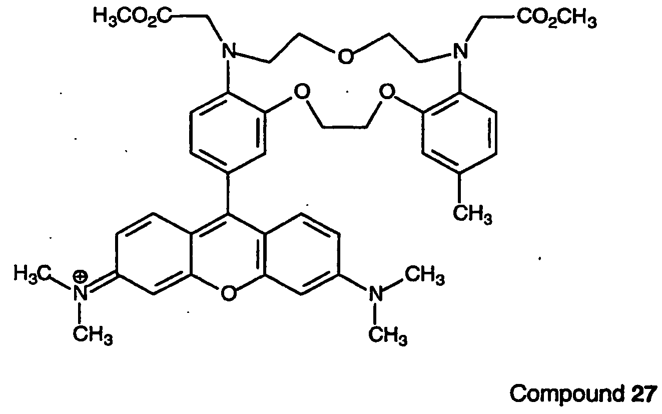 | K_{d} (Na⁺) = ∼ 95 mM K_{d} (K⁺) = ∼ 300 mM |
| 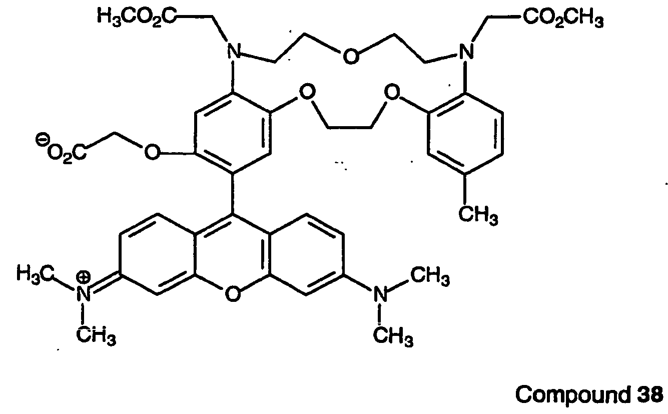 ' | K_{d} (Na⁺) = ∼ 92 mM K_{d} (K⁺) = ∼ 705 mM |
| 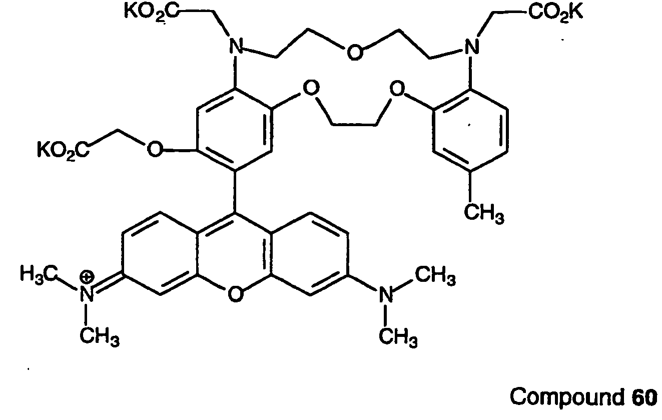 | K_{d} (Na⁺) = ∼ 70 mM K_{d} (Zn²⁺) = ∼ 100 nM |
| 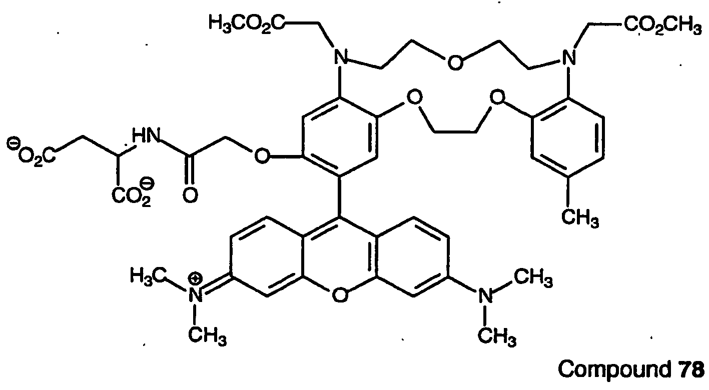 | K_{d} (Na⁺) = ∼ 85 mM K_{d} (K⁺) = ∼ 255 mM |
| 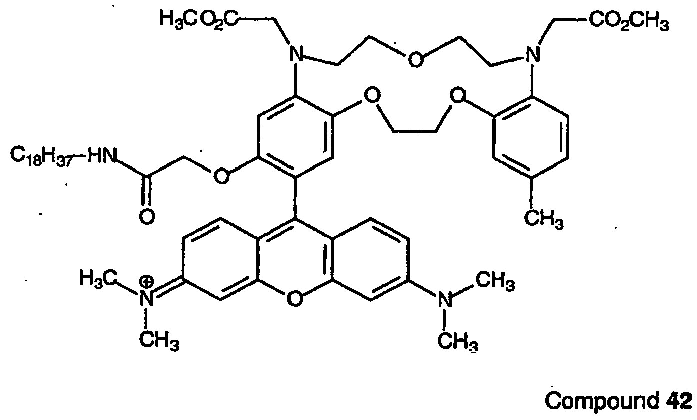 ' | K_{d} (Na⁺) = ∼ 14 mM |
| 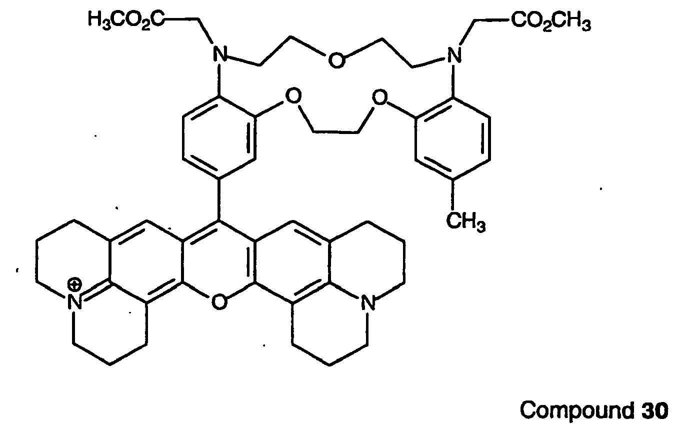 | K_{d} (Na⁺)= ∼ 103 mM K_{d} (K⁺)= ∼ 205 mM |
| 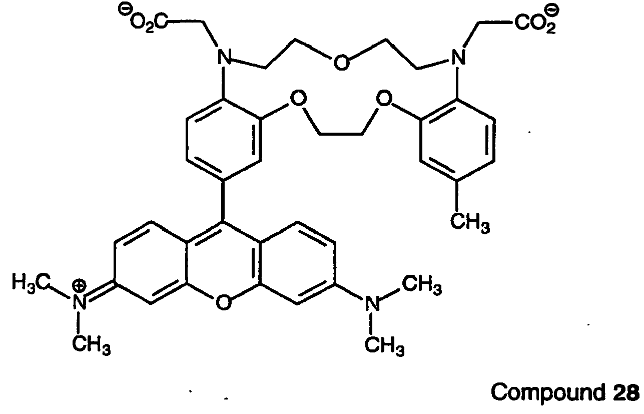 | K_{d} (Zn²⁺) = ∼ 300 nM K_{d} (Ca²⁺) = ∼ 4 µM k_{d} (Na⁺) = ∼ 38 mM K_{d} (K⁺) = ∼ 270 mM |
| 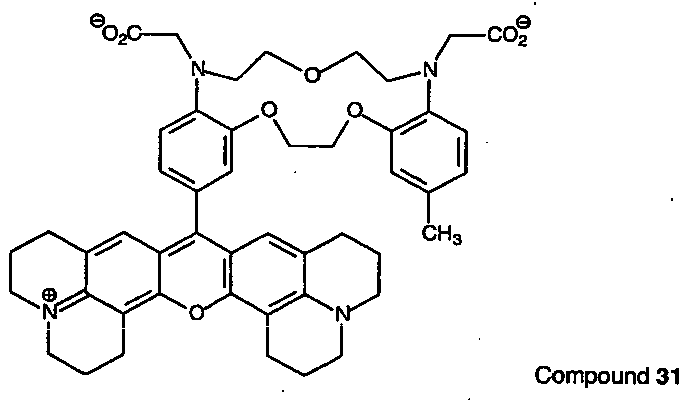 | K_{d} (Zn²⁺) = ∼ 8 µM K_{d} (Ca²⁺) = ∼ 7 µM |
| 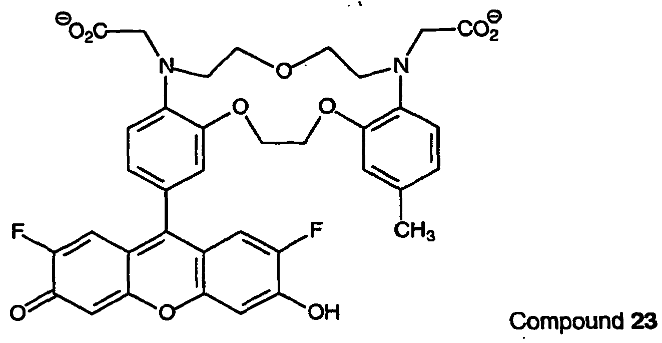 | K_{d} (Na⁺) = ∼ 36 mM K_{d} (K⁺) = ∼ 215 mM K_{d} (Zn²⁺) = ∼ 300 nM K_{d} (Ca²⁺) = ∼ 5 µM |
| 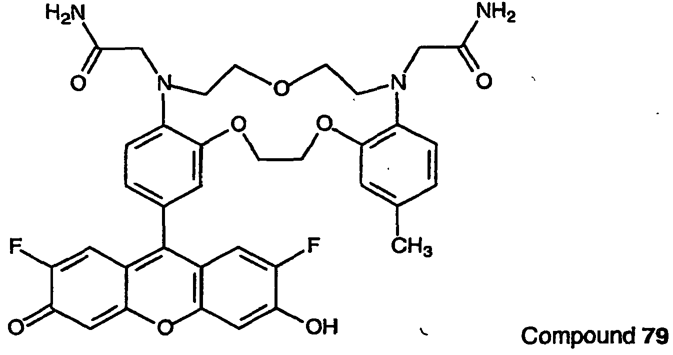 | K_{d} (Na⁺) = ∼ 2.0 M K_{d} (Zn²⁺) = ∼ 600 µM |
| 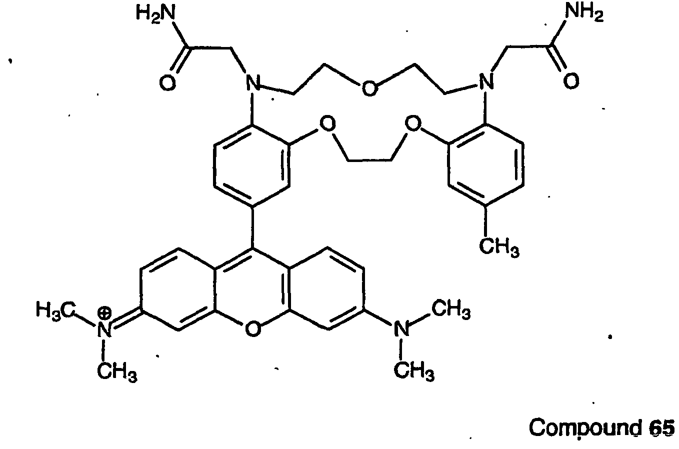 | K_{d} (Na⁺) = ∼ 500 mM K_{d} (Zn²⁺) = ∼ 650 µM |
| 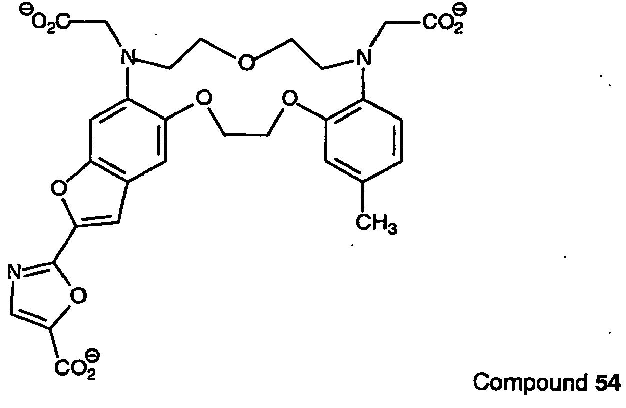 | K_{d} (Ca²⁺) = ∼ 400 nM K_{d} (Na⁺) = ∼ 160 mM |
| 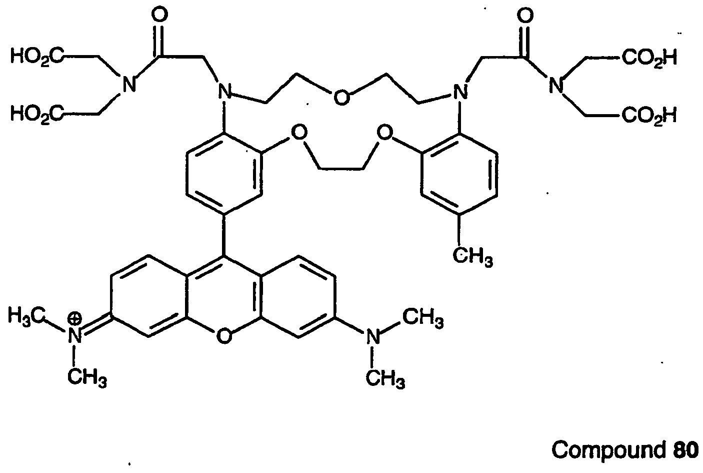 | |
| 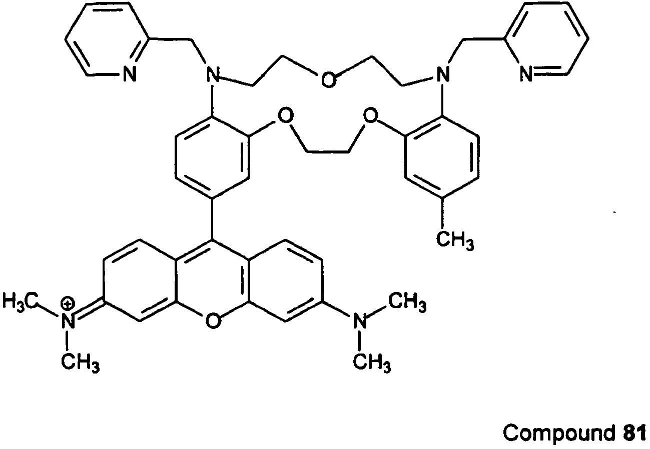 | |

Table 2 lists selected preferred compounds according to the first aspect of the invention for in solution binding of target ions

**Table 2:**

| Compound # | Dye moiety | R¹ = | Dissociation constant (K_{d}) and Response to complexation (R = F/F₀) for Target metal ions |
|---|---|---|---|
| **104** | xanthene | -CH₂COOCH₃ | K_{d} (Li⁺) = 438 mM; R (Li⁺) = 2.8 |
| | | | K_{d} (Na⁺) = 226 mM; R (Na⁺) = 19.7 |
| | | | K_{d} (K⁺) = 978 mM; R (K⁺) = 9.7 |
| | | | K_{d} (Rb⁺) = 376 mM; R (Rb⁺) = 2.9 |
| **86** | xanthene | -CH₂COOCH₃ | K_{d} (Li⁺) = 142 mM; R (Li⁺) = 6.8 |
| | | | K_{d} (Na⁺) = 82 mM; R (Na⁺) = 28.5 |
| | | | K_{d} (K⁺) = 291 mM; R (K⁺) = 6.9 |
| | | | K_{d} (Rb⁺) = 319 mM; R (Rb⁺) = 2.7 |
| **110** | xanthene | -CH₂COOCH₃ | K_{d} (Li⁺) = no sensitivity |
| | | | K_{d} (Na⁺) = 15 mM; R (Na⁺) = 1.5 |
| | | | K_{d} (K⁺) = 11 mM; R (K⁺) = 2.2 |
| | | | K_{d} (Rb⁺) = 25 mM; R (Rb⁺) = 1.5 |
| | | | K_{d} (Rb⁺) = 25 mM; R (Rb⁺) =1.5 |
| **93** | indole | -CH₂COOCH₃ | K_{d} (Na⁺) = 89 mM; R (Na⁺) = 25 |
| | | | K_{d} (K⁺) = no sensitivity |
| **89** | xanthene | -CH₂COOCH₃ | K_{d} (Na⁺) = 163 mM; R (Na⁺) = 41 |
| | | | K_{d} (K⁺) = 254 mM; R (K⁺) = 10.6 |
| **90** | borapolyazaindacene | -CH₂COOCH₃ | K_{d} (Na⁺) =100 mM; R (Na⁺) = 7.3 |
| | | | K_{d} (K⁺) = 170 mM; R (K⁺) = 2.9 |
| **127** | xanthene | -CH₂COOH | K_{d} (Na⁺) = 20 mM; R (Na⁺) = 3.6 |
| **115** | xanthene | -(CH₂)₂COCH₃ | K_{d} (Na⁺) = 78.8 mM; R (Na⁺) = 5.9 |
| | | | K_{d} (K⁺) = 269 mM; R (K⁺) = 2.8 |
| **118** | xanthene | -(CH₂)₂COCH₃ | K_{d} (Na⁺) = 150 mM; R (Na⁺) = 12 |
| **120** | indole | -(CH₂)₂COCH₃ | K_{d} (Na⁺) = 89 mM; R (Na⁺) = 2 |
| **126** | indole | -(CH₂)₂CN(CH₃)₂ | K_{d} (Na⁺) = 379 mM; R (Na⁺) = -1.2 |
| **99** | xanthene | -CH₂COOCH₃ | K_{d} (Li⁺) = 65 mM; R (Li⁺) = 3.1 |
| | | | K_{d} (Na⁺) = 59 mM; R (Na⁺) = 3.9 |
| | | | K_{d} (K⁺) =144 mM; R (K⁺) = 2.7 |
| | | | K_{d} (Rb⁺) = 157 mM; R (Rb⁺) = 1.9 |

### 4. Reactive Functional Groups and Conjugated Substances of the crown ether chelate compounds.

As described above, the compounds of the invention may be substituted one or more times by a-L-Rₓ moiety or-L-Sc moiety. L is a covalent linkage that is a single covalent bond or a seris of stable bonds comprising 1-20 nohydrogen atoms selected from the group consisting of C, O, N, P and S. Rₓ is a reactive group that functions as the site of attachment for another moiety wherein the reactive group chemically reacts with an appropriate reactive or functional group on another substance or moiety. These reactive groups are synthesized during the formation of the present compounds providing present crown ether chelate compounds that can be covalently attached to another substance, conjugated substance, facilitated by the reactive group. In this way, compounds incorporating a reactive group (Rₓ) can be covalently attached to a wide variety of biomolecules or non-biomolecules that contain or are modified to contain functional groups with suitable reactivity, resulting in chemical attachment of the conjugated substance (S_{c}), represented by -L-S_{c}. The reactive group and functional group are typically an electrophile and a nucleophile that can generate a covalent linkage. Alternatively, the reactive group is a photoactivatable group, and becomes chemically reactive only after illumination with light of an appropriate wavelength. Typically, the conjugation reaction between the reactive group and the substance to be conjugated results in one or more atoms of the reactive group R_{X} to be incorporated into a new linkage attaching the compound of the invention to the conjugated substance S_{c}. Selected examples of functional groups and linkages are shown in Table 3, where the reaction of an electrophilic group and a nucleophilic group yields a covalent linkage.

**Table 3: Examples of some routes to useful covalent linkages**

| Electrophilic Group | Nucleophilic Group | Resulting Covalent Linkages |
|---|---|---|
| activated esters* | amines/anilines | carboxamides |
| acrylamides | thiols | thioethers |
| acyl azides** | amines/anilines | carboxamides |
| acyl halides | amines/anilines | carboxamides |
| acyl halides | alcohols/phenols | esters |
| acyl nitriles | alcohols/phenols | esters |
| acyl nitriles | amines/anilines | carboxamides |
| aldehydes | amines/anilines | imines |
| aldehydes or ketones | hydrazines | hydrazones |
| aldehydes or ketones | hydroxylamines | oximes |
| alkyl halides | amines/anilines | alkyl amines |
| alkyl halides | carboxylic acids | esters |
| alkyl halides | thiols | thioethers |
| alkyl halides | alcohols/phenols | ethers |
| alkyl sulfonates | thiols | thioethers |
| alkyl sulfonates | carboxylic acids | esters |
| alkyl sulfonates | alcohols/phenols | ethers |
| anhydrides | alcohols/phenols | esters |
| anhydrides | amines/anilines | carboxamides |
| aryl halides | thiols | thiophenols |
| aryl halides | amines | aryl amines |
| aziridines | thiols | thioethers |
| boronates | glycols | boronate esters |
| carbodiimides | carboxylic acids | N-acylureas or anhydrides |
| diazoalkanes | carboxylic acids | esters |
| epoxies | thiols | thioethers |
| haloacetamides | thiols | thioethers |
| haloplatinate | amino | platinum complex |
| haloplatinate | heterocycle | platinum complex |
| haloplatinate | thiol | platinum complex |
| halotriazines | amines/anilines | aminotriazines |
| halotriazines | alcohols/phenols | triazinyl ethers |
| halotriazines | thiols | triazinyl thioethers |
| imido esters | amines/anilines | amidines |
| isocyanates | amines/anilines | ureas |
| isocyanates | alcohols/phenols | urethanes |
| isothiocyanates | amines/anilines | thioureas |
| maleimides | thiols | thioethers |
| phosphoramidites | alcohols | phosphite esters |
| silyl halides | alcohols | silyl ethers |
| sulfonate esters | amines/anilines | alkyl amines |
| sulfonate esters | thiols | thioethers |
| sulfonate esters | carboxylic acids | esters |
| sulfonate esters | alcohols | ethers |
| sulfonyl halides | amines/anilines | sulfonamides |
| sulfonyl halides | phenols/alcohols | sulfonate esters |

| | | |
|---|---|---|
| * Activated esters, as understood in the art, generally have the formula -COΩ, where Ω is a good leaving group (e.g., succinimidyloxy (-OC₄H₄O₂) sulfosuccinimidyloxy (-OC₄H₃O₂-SO₃H), -1-oxybenzotriazoly! (-OC₆H₄N₃); or an aryloxy group or aryloxy substituted one or more times by electron withdrawing substituents such as nitro, fluoro, chloro, cyano, or trifluoromethyl, or combinations thereof, used to form activated aryl esters; or a carboxylic acid activated by a carbodiimide to form an anhydride or mixed anhydride -OCOR^{a} or -OCNR^{a}NHR^{b}, where R^{a} and R^{b}, which may be the same or different, are C₁-C₆ alkyl, C₁-C₆ perfluoroalkyl, or C₁-C₆ alkoxy; or cyclohexyl, 3-dimethylaminopropyl, or N-morpholinoethyl). ** Acyl azides can also rearrange to isocyanates | | |

Choice of the reactive group used to attach the compound of the invention to the substance to be conjugated typically depends on the reactive or functional group on the substance to be conjugated and the type or length of covalent linkage desired. The types of functional groups typically present on the organic or inorganic substances (biomolecule or non-biomolecule) include, but are not limited to, amines, amides, thiols, alcohols, phenols, aldehydes, ketones, phosphates, imidazoles, hydrazines, hydroxylamines, disubstituted amines, halides, epoxides, silyl halides, carboxylate esters, sulfonate esters, purines, pyrimidines, carboxylic acids, olefinic bonds, or a combination of these groups. A single type of reactive site may be available on the substance (typical for polysaccharides or silica), or a variety of sites may occur (e.g., amines, thiols, alcohols, phenols), as is typical for proteins. A conjugated substance may be conjugated to more than one crown ether chelate compound, which may be the same or different, or to a substance that is additionally modified by a hapten, such as biotin. Although some selectivity can be obtained by careful control of the reaction conditions, selectivity of labeling is best obtained by selection of an appropriate reactive functional group.

Typically, R_{X} will react with an amine, an alcohol, an aldehyde, a ketone, or with silica. Preferably R_{X} reacts with an amine or a thiol functional group, or with silica. In one embodiment, R_{X} is an acrylamide, an activated ester of a carboxylic acid, an acyl azide, an acyl nitrile, an aldehyde, an alkyl halide, a silyl halide, an anhydride, an aniline, an aryl halide, an azide, an aziridine, a boronate, a diazoalkane, a haloacetamide, a halotriazine, a hydrazine (including hydrazides), an imido ester, an isocyanate, an isothiocyanate, a maleimide, a phosphoramidite, a reactive platinum complex, a sulfonyl halide, or a thiol group. By "reactive platinum complex" is particularly meant chemically reactive platinum complexes such as described in U.S. Patent No. 5,714,327.

Where R_{X} is an activated ester of a carboxylic acid, the resulting compound is particularly useful for preparing conjugates of proteins, nucleotides, oligonucleotides, or haptens. Where R_{X} is a maleimide or haloacetamide the resulting compound is particularly useful for conjugation to thiol-containing substances. Where R_{X} is a hydrazide, the resulting compound is particularly useful for conjugation to periodate-oxidized carbohydrates and glycoproteins, and in addition is an aldehyde-fixable polar tracer for cell microinjection. Where R_{X} is a silyl halide, the resulting compound is particularly useful for conjugation to silica surfaces, particularly where the silica surface is incorporated into a fiber optic probe subsequently used for remote ion detection or quantitation.

Preferably, R_{X} is a succinimidyl ester of a carboxylic acid, a haloacetamide, a hydrazine, an isothiocyanate, a maleimide group, an aliphatic amine, a silyl halide, or a psoralen. More preferably, R_{X} is a succinimidyl ester of a carboxylic acid, a maleimide, an iodoacetamide, or a silyl halide. In a particular embodiment R_{X} is a silyl halide or an isothiocyanate.

The compounds of the invention that possess a reactive functional group are useful for conjugation to any substance that possesses a suitable functional group for covalent attachment of the chelate. Examples of particularly useful conjugates include, among others, conjugates of antigens, steroids, vitamins, drugs, haptens, metabolites, toxins, environmental pollutants, amino acids, peptides, proteins, nucleic acids, nucleic acid polymers, carbohydrates, lipids, and non-biological polymers. Alternatively, these are conjugates of cells, cellular systems, cellular fragments, or subcellular particles. Examples include, among others, virus particles, bacterial particles, virus components, biological cells (such as animal cells, plant cells, bacteria, yeast, or protists), or cellular components.

Preferably the conjugated substance is a protein, polysaccharide, lipid, lipid assembly, non-biological polymer, or polymeric microparticle. Another class of preferred conjugated substances includes particles or fibers composed of silica or other glasses, useful for preparing optical devices for remote sensing.

Preferred nucleic acid polymer conjugates are labeled, single- or multi-stranded, natural or synthetic DNA or RNA, DNA or RNA oligonucleotides, or DNA/RNA hybrids, or incorporate an unusual linker such as morpholine derivatized phosphates (AntiVirals, Inc., Corvallis OR), or peptide nucleic acids such as *N*-(2-aminoethyl)glycine units.

In a preferred embodiment, the conjugated substance (S_{c}) is a carbohydrate that is typically a polysaccharide, such as a dextran, FICOLL, heparin, glycogen, amylopectin, mannan, inulin, starch, agarose and cellulose. Alternatively, the carbohydrate is a polysaccharide that is a lipopolysaccharide. Preferred polysaccharide conjugates are dextran, FICOLL, or lipopolysaccharide conjugates.

In another embodiment, the conjugated substance (S_{c}), is a lipid moiety (typically having 6-60 carbons), including glycolipids, phospholipids, sphingolipids, and steroids. Alternatively, the conjugated substance is a lipid assembly, such as a liposome. The lipid moiety may be used to retain the conjugated substances in cells, as described in U.S. Pat. 5,208,148.

Other conjugates of non-biological materials include conjugates of organic or inorganic polymers, polymeric films, polymeric wafers, polymeric membranes, polymeric particles, or polymeric microparticles, including, magnetic and non-magnetic microspheres, conducting and non-conducting metals and non-metals, and glass and plastic surfaces and particles. Conjugates are optionally prepared by copolymerization of a compound of the invention that contains an appropriate functionality while preparing the polymer, or by chemical modification of a polymer that contains functional groups with suitable chemical reactivity. Other types of reactions that are useful for preparing conjugates of polymers include catalyzed polymerizations or copolymerizations of alkenes and reactions of dienes with dienophiles, transesterifications or transaminations. In another embodiment, the conjugated substance is a glass or silica, which may be formed into an optical fiber or other structure.

Conjugates typically result from mixing appropriate reactive dyes and the substance to be conjugated in a suitable solvent in which both are soluble. Labeling of insoluble polymers or silica can be performed in a suspension of the insoluble polymer in a suitable solvent. For those reactive groups that are photoactivated, conjugation requires illumination of the reaction mixture to activate the reactive group.

### Synthesis

There are typically three components to the methodology used to prepare the compounds of the invention. The first involves the formation of the crown ether chelate itself, the second involves the appropriate derivatization of the secondary amine nitrogen atom(s) of the crown ether chelate, and the third, when needed, involves modification of the crown ether chelate by forming a reactive functional group, covalently attaching a conjugate, or covalently attaching a DYE moiety to form an indicator. It should be understood that the DYE moiety is typically not attached to the crown ether chelate compound but that the conjugated substance is attached in this way. Although these synthetic components are typically performed in the order given, they may be carried out in any other suitable sequence. For example, a portion of the chelate may be derivatized with a fluorescent dye prior to formation of the complete chelate ring.

Where the P and Q moieties are both oxygen, and E¹ is ethylene, the crown ether chelate is typically prepared by acylation of a bis-(2-aminophonoxy)ethane with a bis-(acid chloride), such as diglycolyl chloride, followed by reduction of the resulting bis-amide to the corresponding bis-secondary amine. Selection of the appropriate bis-acid chloride results in the particular desired crown ether.

The secondary amine nitrogen atom(s) present in the crown ether are typically derivatized with an alkylating agent. As the metal binding ability of the resulting crown ether is significantly influenced by the nature of the amine substituents, careful selection of the alkylating agent may be necessary to prepare a reporter for a particular target ion. Where the crown nitrogens are alkylated by methyl bromoacetate, the resulting bis-aza-crown ether is typically selective for sodium ions. If the alkylating agent is 2-picolyl chloride, the resulting crown ether is typically selective for zinc ions. As discussed above, the presence of esters vs. carboxylic acids on the amine nitrogen substituents may influence the relative binding affinity of selected target ions. Selection of an alkylating agent that incorporates a precursor to a reactive functional group is useful for producing chemically reactive compounds of the invention, as well as acting as a useful intermediate for preparing conjugates, as described above. Additionally, an alkylating agent that incorporates a reporter DYE results in a crown ether compound that functions as an indicator for selected target ions.

More typically, the crown ether chelate is derivatized at the benzo ring of the crown ether: As described above, typically a suitable crown ether is prepared, and then bound to a DYE moiety. In one aspect of the invention, an ortho-hydroxy aromatic aldehyde is treated with a chloromethyl heterocycle to yield a fused reporter. In another aspect of the invention, derivatization with a DYE moiety is carried out by modifying a crown ether that possesses an aldehyde or ketone functional group.

In one aspect of the invention, the crown ether is substituted by an aldehyde and the fuorophore precursors and the crown ether are combined under anaerobic or non-oxidative conditions (e.g., under nitrogen), and subsequently oxidized using a mild oxidant (e.g., a quinone oxidant, preferably DDQ or chloranil). Where xanthene fluorophore precursors are condensed under anaerobic conditions, the resulting fluorophore is the non-fluorescent dihydro species, which may be utilized without prior oxidation as a sensor for oxidative subenvironments, e.g., in cells.

In yet another aspect of the invention, the crown ether is substituted by a carboxylic acid or by an aldehyde that is converted to the carboxylic acid in the course of synthesis of the crown ether. The chelating moiety is then condensed with the fluorophore precursors to yield the resulting indicator directly.

Synthesis of conventional xanthene dyes such as fluoresceins, rhodamines and rhodols typically involve the condensation of two equivalents of resorcinol (for fluoresceins), aminophenol (for rhodamines) or a mixture of a resorcinol and an aminophenol (for rhodols) with a carbonyl-containing moiety such as a phthalic acid derivative or benzaldehyde. In the synthesis of the xanthene indicators of the invention, the desired resorcinol or aminophenol is condensed with the substituted crown ether, yielding either the reduced xanthene (where the crown ether contains an aldehyde) or the oxidized xanthene (where the crown ether contains a carboxylic acid or acyl halide) bound directly to the chelating moiety.

An oxidation step is typically required after condensation of a formyl-substituted crown ether with the fluorophore precursors. Optionally, the dihydro condensation product is isolated and subsequently oxidized with air or by standard chemical oxidants, such as chloranil. For some fluorophores, the oxidation reaction is enhanced by acidic reaction conditions. These mild oxidation reaction conditions tolerate a wide variety of substituents on the fluorophore and/or crown ether of the resulting indicators.

Unsymmetrical xanthene dyes are typically constructed by statistical methods, using a 1:1 mixture of the desired resorcinols or aminophenols in the condensation reaction, and purifying the desired product from the statistical mix of products using methods known in the art.

The synthesis of polyazaindacene dyes, particularly dipyrrometheneboron difluoride dyes, has been well documented (U.S. Patent Nos. 4,774,339; 5,187,288; 5,248,782; 5,274,113; 5,338,854 and 5,433,896). The procedure typically consists of an acid-catalyzed condensation of a benzaldehyde with a pyrrole that has a hydrogen at the 2-position, followed by *in situ* oxidation of the condensed intermediate by air, oxygen or a chemical oxidant such as 2,3-dichloro-5,6-dicyano-1,4-benzoquinone (DDQ). The condensation of two appropriately substituted pyrroles, each having a hydrogen at the 2-position, with a formyl-substituted crown ether, followed by *in situ* oxidation of the condensed intermediate and treatment with a boron trifluoride etherate yields the dipyrrometheneboron difluoride indicators of the invention. Alternatively, the indicators are formed via the direct condensation of a carboxyl- or chlorocarbonyl-substituted crown ether with two equivalents of appropriately substituted pyrroles, which may be the same of different, provided each has a hydrogen at the 2-position. The latter procedure does not require oxidation.

Post-condensation modifications of both the crown ether and the fluorophore moiety are typically strictly analogous to known methods of indicator modification; for example, the reduction of nitro substituents to amino groups, the conversion of carboxy substituents to cyano groups, and the preparation of esters of carboxylic acids, including acetoxymethyl esters. Additionally, salts and counterions of the indicators of the invention are readily converted to other salts by treatment with ion-exchange resins, selective precipitation, and basification, as is well-known in the art.

Post-condensation modifications of xanthylium dyes are well known. For instance, the xanthenone portion of the dye can be halogenated by treatment with the appropriate halogenating agent, such as liquid bromine. Xanthenes containing unsaturated fused rings can be hydrogenated to the saturated derivatives.

The reduced and oxidized versions of the xanthene indicators are freely interconvertible by well-known oxidation or reduction reagents, including borohydrides, aluminum hydrides, hydrogen/catalyst, and dithionites. Care must be exercised to select an oxidation or reducing agent that is compatible with the crown ether chelator. A variety of oxidizing agents mediate the oxidation of dihydroxanthenes, including molecular oxygen in the presence or absence of a catalyst, nitric oxide, peroxynitrite, dichromate, triphenylcarbenium and chloranil. The dihydroxanthenes are also oxidized electrochemically, or by enzyme action, including horseradish peroxidase in combination with peroxides or by nitric oxide.

Rather than condensing the DYE moiety precursors directly with a substituted crown ether, the preformed DYE moiety may be covalently bound to the crown ether via a conventional cross-linking reaction. A wide variety of chemically reactive or potentially chemically reactive and fluorescent fluorescein, rhodamine, rhodol, benzoxanthenes, dibenzoxanthene and other xanthene oxygen heterocycles that absorb maximally beyond about 490 nm are commercially available as described by Haugland, HANDBOOK OF FLUORESCENT PROBES AND RESEARCH PRODUCTS *(Supra),* as described above, or in other literature references. The nature of the bond that links the DYE moiety to the crown ether chelate appears to have an effect on the optical response of the DYE moiety to ion binding, sometimes a significant effect. Acceptability of the linking chemistry can be determined by titration of the resultant indicator with the ion of interest over the target range of response (as described in Example 2).

### B. Method of Use

The crown ether compounds of the invention are useful for any application where it is desirable to complex a target metal ion. Selected crown ether compounds of the invention may be useful as ionophores, that is, they facilitate the transport of selected target ions across cell membranes. Where the crown ether compound is bound to a conjugated substance that is a polymeric matrix, such as a microparticle, or agarose, the compounds are useful for depleting a sample solution of a selected target ion, particularly where the polymeric matrix is used to pack a chromatography column. Other crown ether compounds (those bound to a DYE moiety) are useful as colorimetric or fluorescent indicators for a selected target ion.

In order for a particular indicator of the present invention to be useful for detection purposes, it must exhibit a detectable change in spectral properties upon complexation of the desired metal ion (target ion) in the chelating moiety. Preferably the change in spectral properties is a change in fluorescence properties. More preferably, the instant indicators display an intensity increase or decrease in emission energy upon the complexation of the desired target ion.

Accordingly, a method for binding target metal ions in a sample comprises the following steps:
a) contacting said sample with a crown ether chelate compound of the present invention; and,
b) incubating said sample and said metal chelating compound for sufficient time to allow said compound to chelate said target metal ion whereby said metal ion is bound.

When the present compounds are used as indicators a DYE moiety is covalently attached to the crown ether chelate. The sample is illuminated with an appropriate wavelength whereby the target ion is detected. In such an assay the target ion can also be quantitated and monitored.

The specific indicator used in an assay or experiment is selected based on the desired affinity for the target ion as determined by the expected concentration range in the sample, the desired spectral properties, and the desired selectivity. Initially, the suitability of a material as an indicator of ion concentration is commonly tested by mixing a constant amount of the indicating reagent with a measured amount of the target ion under the expected experimental conditions.

Preferred indicators display a high selectivity, that is, they show a sufficient rejection of non-target ions. The interference of a non-target ion is tested by a comparable titration of the indicator with that ion. Although preferred target ions for most indicators of the present invention are Na⁺ and K⁺, any ion that yields a detectable change in absorption wavelengths, emission wavelengths, fluorescence lifetimes or other measurable optical property over the concentration range of interest is potentially measured using one of the indicators of this invention. Modifications to the electronic structure of the crown ether or indicator to produce an indicator having the appropriate combination of binding affinity, ion selectivity and spectral response for a wide variety of metal ions.

In one embodiment of the invention, the target ions for the indicators of the present invention are selected from Li⁺, Na⁺, K⁺, Cs⁺, Ca²⁺, Zn²⁺, Mg²⁺, Rb⁺, Tb³⁺ or Eu³⁺. In another embodiment of the invention, the target ions are selected from Li⁺, Na⁺, K⁺, Ca²⁺, Zn²⁺, and Mg²⁺. Additional target ions for selected embodiments of the present indicators also include Mn²⁺, Fe^{2+,} Fe³⁺, Co²⁺, Ni²⁺, Cu²⁺, Cu⁺, Zn²⁺, Al³⁺, Cd²⁺, Ag⁺, Au⁺, Tl⁺, Pd²⁺, Hg²⁺, Hg⁺, Sn²⁺, Pb²⁺, Sr²⁺, Ba²⁺, Mo³⁺, Ga³⁺, In³⁺, La³⁺, Eu³⁺, Tb³⁺, Dy³⁺, Ru³⁺, Sc³⁺, As³⁺, Sb³⁺, Cr³⁺, Bi³⁺, Ca³⁺, Ce⁴⁺, Pd²⁺, Pt²⁺ and Pt⁴⁺ ions. In yet another embodiment of the invention, the target ions of the instant indicators are Fe²⁺, Fe³⁺, Co²⁺, Ni²⁺, Cu²⁺, Cu⁺, Zn²⁺, Al³⁺, Cd²⁺, Hg²⁺, Pb²⁺, Ba²⁺, La³⁺, Tb³⁺ and Cr³⁺ ions. In yet another embodiment, the target ions are selected from the group consisting of Fe³⁺, Ni²⁺, Cu²⁺, Cu⁺, Hg²⁺, or Pb²⁺.

The indicator is generally prepared for use as a detection reagent by dissolving the indicator in solution at a concentration that is optimal for detection of the indicator at the expected concentration of the target ion. Modifications that are designed to enhance permeability of the indicator through the membranes of living cells, such as acetoxymethyl esters and acetates, may require the indicator to be predissolved in an organic solvent such as dimethylsulfoxide (DMSO) before addition to a cell suspension, where the indicators then readily enter the cells. Intracellular enzymes cleave the esters to the more polar acids and phenols that are then well retained inside the cells. For applications where permeability of cell-membranes is required, the indicators of the invention are typically substituted by only one fluorophore.

Therefore, in accordance with the third aspect of the invention there is provided a method for binding and detecting target ions in a live cell comprises the following steps:
a) contacting a sample of live cells with a crown ether chelating compound of the first aspect of the present invention wherein said compound comprises a DYE moiety and at least one lipohilic group;
b) incubating said sample and said crown ether chelate compound for sufficient time to allow said compound to chelate said target metal ion; and,
c) illuminate said sample with an appropriate wavelength whereby said target ion is detected in a live cell.

Typically, the lipophilic group is an AM or acetate ester that is directly attached to the DYE moiety of the crown ether chelate compound.

A specific indicator of the present invention is useful for the detection and/or quantification of a desired target ion, when the binding of the target ion in the metal ion-binding moiety of the indicator results in a detectable change in spectral properties. Preferably, the change in spectral properties is a detectable fluorescence response.

A preferred indicator for a specific target ion is an indicator that shows at least a two-fold change in net fluorescence emission intensity (either higher or lower), or a 1 nanosecond difference in fluorescence lifetime (either shorter or longer), preferably a five-fold or greater change in net fluorescence emission intensity or a 100% change in fluorescence lifetime in response to the target ion. Alternatively, an indicator that exhibits a shift in excitation or emission wavelength of at least 10 nm (either to shorter or longer wavelength) is also preferred, more preferably exhibiting a shift of 25 nm or greater.

The optical response of the indicating reagent is determined by changes in absorbance or fluorescence, preferably fluorescence. If absorbance measurements are used to determine ion concentrations, then it is usually optimal to adjust the optical density of the indicator in the sample over the range of analyte concentration to a value of approximately 0.02 to 2.5 (most preferably 0.1 to 1). For fluorescence measurements, the concentration of the indicator will depend mostly on the sensitivity of the equipment used for its detection.

If the optical response of the indicator will be determined using fluorescence measurements, samples are typically stained with indicator concentrations of 10⁻⁹ M to 10⁻² M. The most useful range of analyte concentration is about one log unit above and below the dissociation constant of the ion-indicator complex. This dissociation constant is determined by titration of the indicator with a known concentration of the target ion, usually over the range of virtually zero concentration to approximately 100 millimolar of the target ion, depending on which ion is to be measured and which indicator is being used. The dissociation constant may be affected by the presence of other ions, particularly ions that have similar ionic radii and, charge. It may also be affected by other conditions such as ionic strength, pH, temperature, viscosity, presence of organic solvents and incorporation of the sensor in a membrane or polymeric matrix, or conjugation or binding of the sensor to a protein or other biological molecule. Any or all of these effects need to be taken into account when calibrating an indicator.

The indicator is combined with a sample in a way that will facilitate detection of the target ion concentration in the sample. The sample is generally a representative cell population, fluid or liquid suspension that is known or suspected to contain the target ion. Representative samples include intracellular fluids such as in blood cells, cultured cells, muscle tissue, neurons and the like; extracellular fluids in areas immediately outside of cells; in vesicles; in vascular tissue of plants and animals; in biological fluids such as blood, saliva, and urine; in biological fermentation media; in environmental samples such as water, soil, waste water and sea water; in industrial samples such as pharmaceuticals, foodstuffs and beverages; and in chemical reactors. Detection and quantitation of the target ion in a sample can help characterize the identity of an unknown sample, or facilitate quality control of a sample of known origin.

In one embodiment of the invention, the sample contains cells, and the indicator is combined with the sample in such a way that the indicator is present within the sample cells. By selection of the appropriate chelating moiety, fluorophore, and the substituents thereon, indicators are prepared that will selectively localize in desired organelles, and provide measurements of the target ion in those organelles. Conjugates of the indicators of the invention with organelle-targeting peptides are used to localize the indicator to the selected organelle, facilitating measurement of target ion presence or concentration within the organelle (as described in U.S. Patent No. 5,773,227). Alternatively, selection of a lipophilic fluorophore, or a fluorophore having predominantly lipophilic substituents will result in localization in lipophilic environments in the cell, such as cell membranes. Selection of cationic indicators will typically result in localization of the indicator in mitochondria.

In accordance with the second aspect of the invention, there is provided a composition of matter comprising any of the compounds of the first aspect and a metal ion that is capable of being chelated by said compound. In one embodiment, the compounds of the invention, in any of the embodiments described above, are associated, either covalently or noncovalently, with a surface such as a microfluidic chip, a silicon chip, a microscope slide, a microplate well, or another solid matrix, and is combined with the sample of interest as it flows over the surface. The detectable optical response is therefore detected on the matrix surface itself, typically by use of an instrumental. This embodiment of the invention is particularly suited to high-throughput screening using automated methods.

Quantification of target ion levels in samples is typically accomplished using the indicators of the present invention by methods known in the art. For example, the ratiometric measurement of ion concentration provides accurate measurement of ion concentrations by the treatment of the fluorescence data as the ratio of excitation or fluorescence intensities at two wavelengths, rather than the absolute intensity at a single wavelength. Using the ratio method, a number of variables that may perturb the ion concentration measurements are eliminated. In particular, ion-dependent factors that affect the signal intensity, such as nonuniform intracellular dye concentrations, probe leakage, dye bleaching and cell thickness, are canceled in the ratio measurements, since these parameters have a similar effect on intensities at both wavelengths. While the ratio method can be used to determine concentrations using observation of either the excitation spectra of the indicator, the emission spectra of the indicator, or both, in the case of the indicators of the present invention, the shift in excitation energy upon binding metal ions makes observation of the excitation spectrum a more useful technique. In either case, to achieve maximal utility, the indicator must be calibrated (to compensate for variance in the dissociation constant of the indicator due to ionic strength, viscosity, or other conditions within the sample). To calibrate the indicator, ionophores such as A-23187, gramicidin, valinomycin, or ionomycin are used. Non-ratiometric analysis can also be accomplished by calibration with a second fluorescent dye present in the sample.

The optical response of the indicator to the ion can be detected by various means that include measuring absorbance or fluorescence changes with an instrument, visually, or by use of a fluorescence sensing device. Several examples of fluorescence sensing devices are known, such as fluorometers, fluorescence microscopes, laser scanners, flow cytometers, and microfluidic devices, as well as by cameras and other imaging equipment. These measurements may be made remotely by incorporation of the fluorescent ion sensor as part of a fiber optic probe. The indicator is covalently attached to the fiber optic probe material, typically glass or functionalized glass (e.g., aminopropyl glass) or the indicator is attached to the fiber optic probe via an intermediate polymer, such as polyacrylamide. The indicator solution is alternatively incorporated non-covalently within a fiber optic probe, as long as there is a means whereby the target ion can come into contact with the indicator solution.

### C. Kits of the Invention

Due to the advantageous properties and the simplicity of use of the instant crown ether compounds, they are particularly useful in the formulation of a kit for the complexation, detection, quantification or monitoring of selected target ions, comprising one or more compounds or compositions of the invention in any of the embodiments described above (optionally in a stock solution), instructions for the use of the crown ether compound to complex or detect a desired target ion, and optionally comprising additional components. In one aspect, the compounds of the invention are associated with a surface, such as a chip, microplate well, or other solid matrix, and the sample of interest flows over the surface. The detectable optical response is therefore detected on the matrix surface itself.

Therefore, in accordance with the fourth aspect of the present invention there is provided a kit for binding a target metal ion in a sample, comprising a compound of the first aspect and comprising one or more components selected from the group consisting of a calibration standard of a metal ion, an ionophore, a fluorescent standard, an aqueous buffer solution and an organic solvent.

The additional kit components may be selected from, without limitation, calibration standards of a target ion, ionophores, fluorescence standards, aqueous buffers, and organic solvents. The additional kit components are present as pure compositions, or as aqueous solutions that incorporate one or more additional kit components. Any or all of the kit components optionally further comprise buffers.

The examples below are given so as to illustrate the practice of this invention. They are not intended to limit or define the entire scope of this invention.

### EXAMPLES

### Example 1. Preparation of (2'-Nitrophenoxy)-2-chloroethane (7).

A suspension of 2-nitrophenol (50.0 g, 0.36 mol), 1-bromo-2-chloroethane (45 mL, 0.54 mol), and K₂CO₃ (100.0 g, 0.72 mol) in DMF (200 mL) was stirred at 90°C for 2 h, cooled to room temperature, poured into ice-water, filtered, washed with H₂O and dried to give Compound **7**, 70.3 g (96%) as a yellow solid.

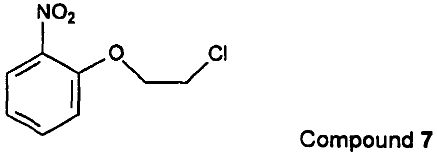

### Example 2. Determination of Na+, K+, Li+ and Tb+ indicator fluorescence response.

The fluorescence response of a selected compound of the invention as a function of ion concentration was determined by dissolving a sample of the pure compound in 3 mL of each of two solutions: solution 1 ("high") consists of 200 mM NaCl (or other appropriate ion) and 10 mM MOPS buffer at pH 7.05; solution 2 ("zero ") consists of 10 mM MOPS buffer at pH 7.05 in deionized water. A series of curves are generated by cross dilution between the two solutions to arrive at intermediate concentrations of Na⁺, K+, Li+ and Tb+. The emission of a selected indicator in solution 2 was scanned and then was repeated to cover the entire range from zero to 200 mM Na⁺. For example, the fluorescence emission of the chosen indicator was scanned while the sample was excited at that indicator's absorption maximum wavelength, and then 1/100 of the sample was removed and replaced with 1/100 of solution 1 to arrive at a Na⁺ concentration of 2 mM. This dilution was repeated to cover the entire range from zero to 1 M Na⁺ and the resulting emission intensities were plotted versus the ion concentrations. A least-squares fit was used to arrive at the concentration where the selected indicator was maximally sensitive to changes in Na⁺ concentration. This corresponded to the dissociation constant of that indicator for Na⁺ and was expressed as a concentration.

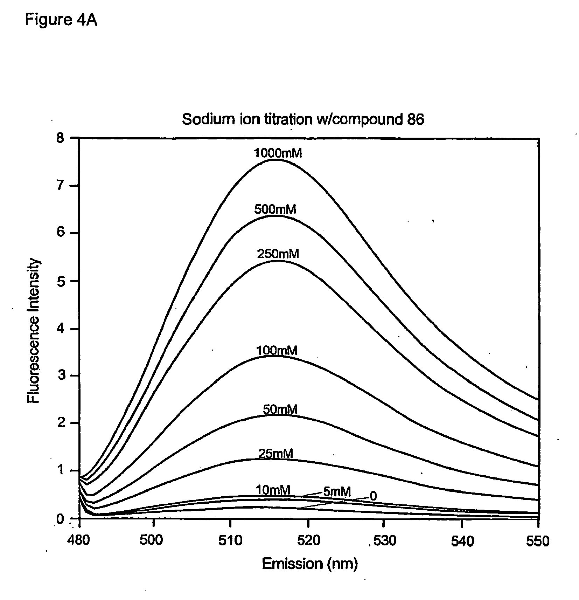

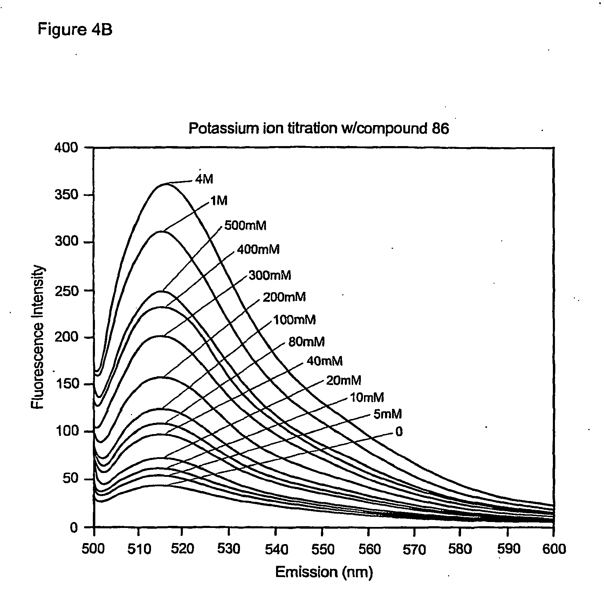

This methodology was repeated for K+, Li+ and Tb+ to obtain emission spectra and to calculate the Kd values. See, Table 2 and Figure 4

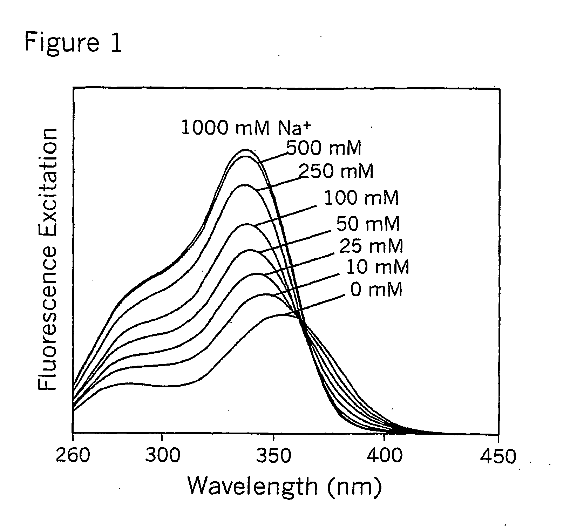

For visible wavelengths probes such as Compound **22,** Compound **27** and Compound **86** (Example 8 the indicators fluorescence emission was typically scanned from 450-650 nm while the sample was excited at the absorption maximum wavelength. For UV-excitable ratiometric probes such as Compound **51**, the excitation wavelengths of the dye in solution 2 were scanned from 260 to 450 nm while monitoring constant fluorescence emission at 510 nm (as in Figure 1).

The binding affinity of a selected indicator for sodium ions, in the presence of potassium ions, is determined using the process above, except in the presence of 100 mM K⁺ concentrations.

### Example 3: Calibration of sodium indicator fluorescence response in cells.

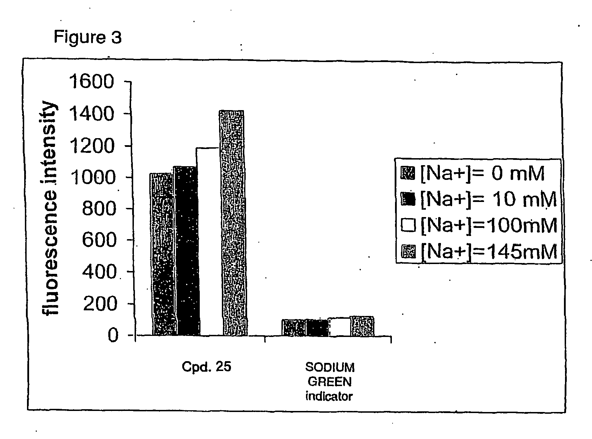

Jurkat cells were loaded with a 5 µM solution of either Compound **25** (a compound according to Formula (I) as shown below), or commercially available SODIUM GREEN tetraacetate indicator (Molecular Probes, Inc., Eugene, OR) and 10 µM for Compound **87** (Example 9) for 30 minutes at 37 °C. The use of PLURONIC dispersing agent helped dissolve the selected indicator. Intracellular sodium concentrations were then established by varying the sodium concentration of the extracellular buffer in the presence of 2 µM gramicidin (a sodium-pore forming antibiotic). The extracellular buffer was set at 0 mM, 10 mM, 20 mM, 50 mM, 100 mM, and 145 mM, respectively. Intracellular fluorescence response of the selected indicators was measured using a FACSCAN flow cytometer and associated software, with fluorescence excitation at 488 nm. Compound **25** exhibits substantially brighter intracellular fluorescence intensity than the SODIUM GREEN indicator at comparable Na⁺ concentrations, as shown in Figure 3. Similarly, as intracellular Na⁺ concentration was increased, Compound **25** exhibits a consistent increase in fluorescence intensity. Compound **87** demonstrated an increased fluorescent signal with increasing concentrations of sodium ions.

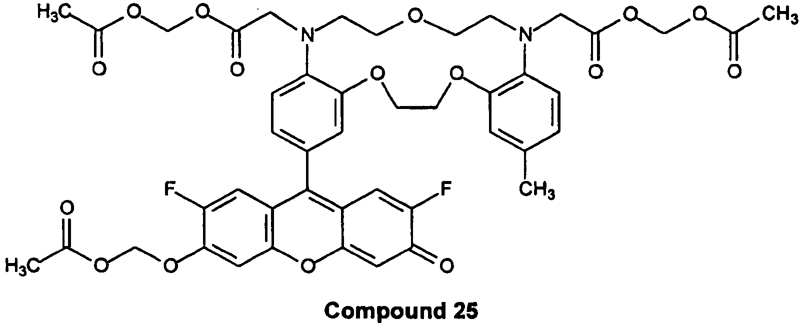

### Example 4: Preparation of 1-Aza-benzo-15-crown-5 ether (82)

To a solution of 2-aminophenol **81** (1.322 g, 12 mmol) in MeCN (1 L), powdered CsF (7.300 g, 48 mmol) is added. The mixture is stirred vigorously for 1 h and then tetraethyleneglycole ditosylate (6.100 g, 12 mmol) in MeCN (50 mL) is introduced. The mixture is refluxed under N₂ atmosphere for 24h and evaporated. The residue is dissolved in CHCl₃ (800 mL), washed with H₂O, sat. NaHCO₃, H₂O, sat. NaCl (200 mL each), filtered through paper, and evaporated. The crude product is purified by column chromatography on silica gel (12x60 cm bed column, made in CHCl₃) using CHCl₃ as eluant to give compound **82**, 1.995 g (62% yield) as a low melting solid.

### Example 5: Preparation of 1-Methoxycarbonylmethyl-1-aza-benzo-15-crown-5-ether (83).

The mixture of compound **82** (1.380 g, 5.17 mmol), DIEA (2.4 mL, 25.84 mmol), methyl bromoacetate (1.8 mL, 10.34 mmol), and Nal (0.750 g, 5.00 mmol) in MeCN (100 mL) is refluxed under N₂ atmosphere for 24 h, then cooled and evaporated. The residue is re-dissolved in in CHCl₃ (400 mL), washed with 1% AcOH (2x200 mL), H₂O (200 mL). The chloroform solution is dried over MgSO₄, filtered through paper, and evaporated. The crude product is purified by column chromatography on silica gel (3x30 cm bed column, made in CHCl₃) using 0-2.5% MeOH gradient in CHCl₃ as eluant to give compound **83**, 1.102 g (63% yield) as a yellow oil.

### Example 6: Preparation of 15-Formyl-1-methoxycarbonylmethyl-1-aza-benzo-15-crown-5-ether (84).

To a solution of the Vilsmeier reagent prepared from POCl₃ (1.0 mL, 11.0 mmol) in 5 mL DMF, compound **83** (0.750 g, 2.20 mmol) in DMF (2 mL) is introduced. The mixture is stirred under a N₂ atmosphere for 16 h, then poured into ice (20 g)/ sat. K₂CO₃ (50 mL) mixture. The mixture is extracted with CHCl₃ (50 + 5x10 mL), and the extract is dried over MagSO₄ and evaporated. The crude product is purified by column chromatography on silica gel (1.5x30 cm bed column, made in CHCl₃) using CHCl₃ as eluant to give aldehyde **84**, 0.632 g (78% yield) as an off-white low-melting solid.

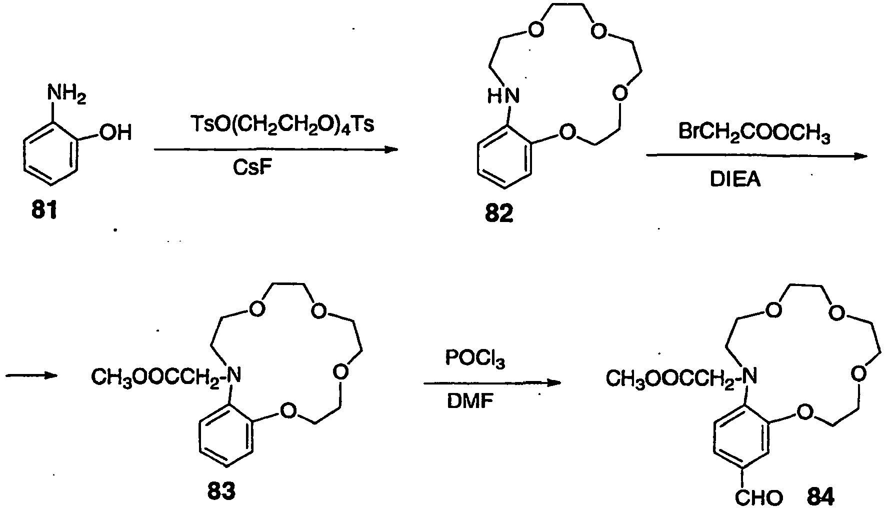

### Example 7: Preparation of compound 85 with a fluorinated xanthene as a DYE

A mixture of the aldehyde **84** (0.212 g, 0.58 mmol) and 4-fluororesorcinol (0.163 g, 1.27 mmol) in methanesulfonic acid (7 mL) is stirred for 24 h, then poured into 3N NaOAc (100 mL). The precipitated solid is filtered off, washed generously with water, and dried under vacuum to give compound **85** (0.229 g, 67%) as an off-white solid. Crude compound **85** is used in the next step without purification.

### Example 8: Preparation of a cell-impermeable metal chelating compound (Compound 86) with a fluorinated xanthene as a DYE

A mixture of the dihydro compound **85** (0.117 g, 0.20 mmol) and freshly powdered chloranil (0.246 g, 1.00 mmol) in CHCl₃/MeOH 1:1 (10 mL) is vigorously stirred upon reflux for 3 h, then cooled down, filtered from the excess oxidizer and evaporated. The residue is purified by preparative TLC on silica gel, using MeOH/AcOH 5%:2% in CHCl₃ as eluant to give the compound 86 (0.046 g, 39%) as an orange solid.

### Example 9: Preparation of a cell-permeable metal chelating compound (Compound 87) with a fluorinated xanthene as a DYE.

To a solution of compound **86** (12 mg, 0.02 mmol) and DIEA (75 µL, 0.4 mmol) in DMF (1 mL), bromomethyl acetate (20 µL, 0.2 mmol) is added. The mixture is stirred for 3 h and evaporated at 1 mm Hg vacuum. The residue is purified by preparative TLC on silica gel, using MeOH/AcOH 7%:1% in CHCl₃ as eluant to give the compound **87** (12 mg, 96%) as an orange solid.

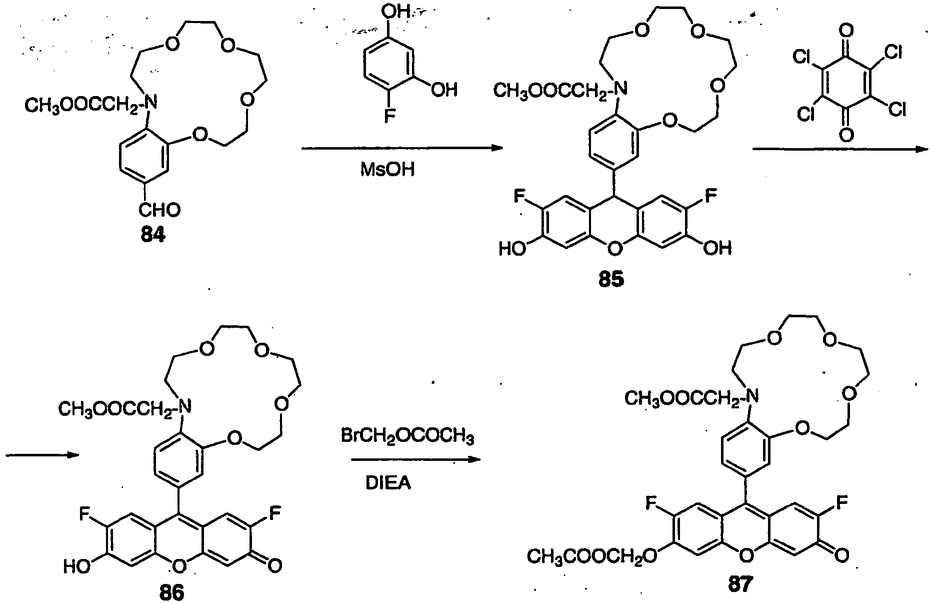

### Example 10: Preparation of metal chelating compound (Compound 89) with Tetramethylrosamine as DYE

A mixture of aldehyde **844** (0.410 g, 1.11 mmol), and 3-(N,N-dimethylamino)phenol (0.337 g, 2.46 mmol), and TsOH (22 mg) in EtCOOH (11 mL) is stirred for 16 h at 65 °C. The mixture is cooled down and poured into 3N NaOAc (100 mL). The resulting suspension is extracted with CHCl₃ (100 + 7x20 mL). The chloroform extract is filtered through paper and evaporated. The crude dihydro compound **88** is re-dissolved in MeOH/CHCl₃ 1:1 mixture (10 mL) and treated with chloranil (0.246 g, 1.00 mmol). The oxidation is continued upon vigorous stirring for 2 h, then the solvents are evaporated, the residue is re-dissolved in CHCl₃, and purified by column chromatography on silica gel (4 x 60 cm bed column, made with 5% MeOH + 1% AcOH in CHCl₃) using (5-12.5%)MeOH / (1.0-1.3%) AcOH gradient as eluant to give the crude product, which is purified further by preparative TLC on silica gel, using 12% MeOH/ 3% AcOH in CHCl₃ as eluant to give compound **89** (0.038 g, 6%) as a dark red solid.

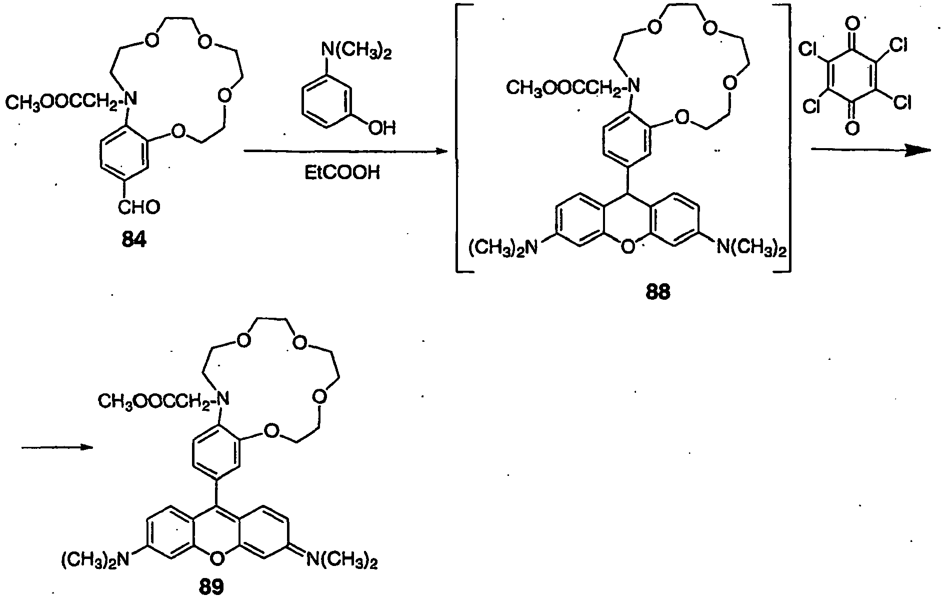

### Example 11: Preparation of a metal chelating compound (Compound 90) with a borapolyazaindacene as a DYE

To a stirred solution of aldehyde **84** (0.367 g, 1.0 mmol) in CH₂Cl₂ (40 ml) 2,4-dimethylpyrrole (0.25 mL, 2.4 mmol) is added, followed by TFA (0.09 mL, 1.2 mmol). The mixture is stirred for 20 h, diluted with CHCl₃ (200 mL) and washed with 2% Me₄NOH (2 x 200 mL), H₂O (200 mL). Tye chloroform layer is separated, filtered through paper filter and evaporated. The residue is co-evaporated with toluene (50 mL), re-dissolved in toluene (40 mL) and stirred 2h with chloranil (0.296 g, 1.2 mmol). DIEA (1.7 mL, 10 mmol) is added, followed by BF₃OEt₂ (1.04 mL, 8 mmol) and the mixture is stirred for 20 h, filtered through Cellite, and evaporated. The residue is purified by column chromatography on silica gel (4x40 cm bed, made in 2% MeOH + 1% AcOH in CHCl₃), using the same mixture as eluant to give compound **90** (0.169 g, 30%) as an orange solid.

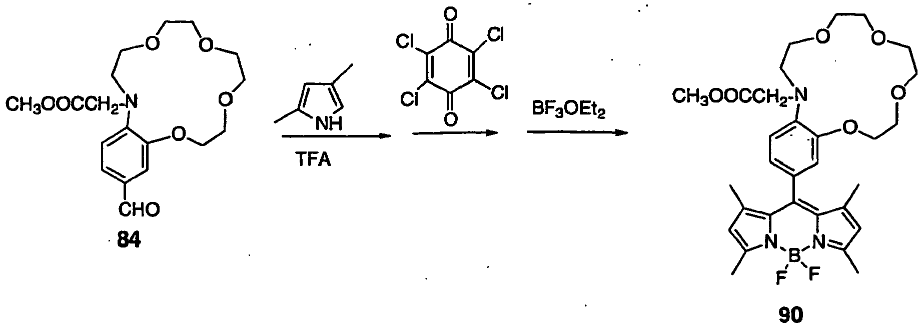

### Example 12: Synthesis of an α-nitrostilbene (Compound 92)

A mixture of aldehyde **84** (0.367 g, 1.0 mmol), Wittig salt **91** (0.804 g, 1.5 mmol), and K₂CO₃ (0.690 g, 5.0 mmol) in DMF (6 mL) is stirred for 7 h at 90 °C, then left overnight at rt. The mixture is poured into H₂O (200 mL), and the resulting suspension is extracted with CHCl₃ (200 + 10x20 mL). The extract is evaporated to dryness at 3 mm Hg and the residue is purified by column chromatography on silica gel (4 x 60 cm bed column, prepared in 4% MeOH in CHCl₃) using the same mixture of solvents as eluant to give stilbene **92** (0.530g, 97%) as a dark red low-melting solid.

### Example 13: Preparation of a metal chelating compound (Compound 93) with indole as a DYE.

A solution of stilbene **92** (0.530 g, 0.97 mmol) in P(OEt)₃ (5 mL) is heated at 125 °C for 4 h, then evaporated. The residue is purified by column chromatography on silica gel (4 x 50 cm bed column, prepared in 5% MeOH in CHCl₃) using the same mixture of solvents as eluant to give compound **93** (0.171 g, 34%).

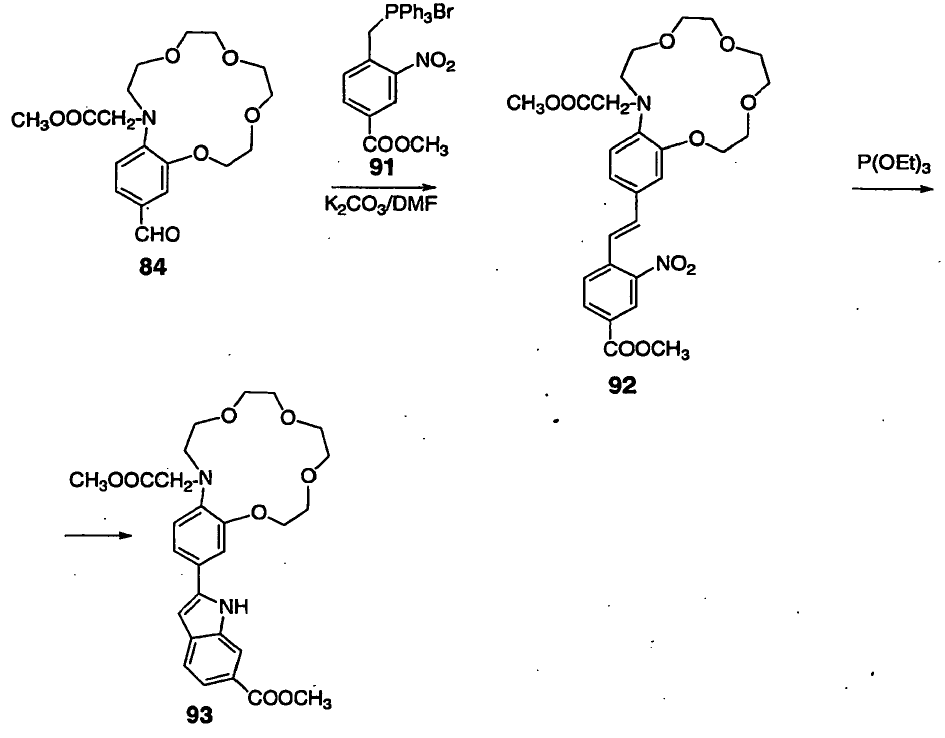

### Example 14: Preparation of 15-Nitro-1-methoxycarbonylmethyl-1-aza-benzo-15-crown-5-ether (Compound 94).

To a stirred solution of crown ether **83** (0.420 g, 1.24 mmol) in Ac₂O (10 mL), 65% HNO₃ (0.10 mL, 1.5 mmol) is introduced at 0 °C. The mixture is stirred for 2 h at 0° C and then poured into sat. K₂CO₃ (100 mL), and stirred for 1 h. The resulting solution is extracted with CHCl₃ (100 + 7x20 mL), the extract is filtered through paper and evaporated. The residue is purified by column chromatography on silica gel (4 x 60 cm bed column, prepared in CHCl₃) using CHCl₃ as eluant to give nitro derivative **94** (0.272g, 57%) as a yellow-orange solid.

### Example 15: Preparation of Azo-dye metal chelating compound (Compound 96)

A solution of crown ether **83** (0.339 g, 1.0 mmol) in dioxane (1 mL) and AcOH (1 mL) is treated with a solution of diazo compound **95**, prepared from sulfanilic acid (0.190 g, 1.1 mmol) in 6 mL H₂O. The mixture is stirred for 3 h, poured into H₂O (100 mL) and extracted with CHCl₃ (100 + 25 x 20 mL). The chloroform extract is evaporated, and the residue is purified by column chromatography on Sephadex LH-20 (3 x 35 cm bed column, prepared in H₂O) using H₂O as eluant to give azo compound **96** (0.029g, 7%) as a dark red solid.

### Example 16: Preparation of 15-Amino-1-methoxycarbonylmethyl-1-aza-benzo-15-crown-5-ether (Compound 97).

A) A sample of nitro compound **94** (0.220 g, 0.57 mmol) is hydrogenated at 50 psi in DMF (25 mL) over 10% Pc/C (40 mg, catalyst) for 7 h. The mixture is filtered, and evaporated at 1 mm Hg. The residue is purified by preparative TLC on silica gel, using 20% MeOH in CHCl₃ as eluant to give the amine **97** (128 mg, 63%).
B) A sample of azo compound **96** (4 mg, 0.01 mmoL) in 0.5 ml ethanol is treated with sodium dithionite (10 mg, 0.06 mmol) in 0.2 ml water. The solvents are evaporated and the residue is washed with water to give amine **97** (3mg, 85%), identical to that prepared in the above example.

### Example 17: Preparation of cell-permeable Compound 98.

To a stirred solution of the amine **97** (25 mg, 0.07 mmol) and DIEA (0.17 ml, 1 mmol) in CH₂Cl₂ (3 mL) an acid chloride, prepared from fluorinated xanthene acid (55 mg, 0.11 mmol) in CH₂Cl₂ (2 mL) is added dropwise. The mixture is stirred for 2 h and evaporated. The residue is purified by preparative TLC on silica gel, using 5% MeOH and 1% AcOH in CHCl₃ as eluant to give the compound **98** (32 mg, 63%) as an off-white solid.

### Example 18: Preparation of cell-impermeable Compound 99.

To a solution of the compound **98** (10 mg, 0.012 mmol) in MeOH (2 mL) an aqueous solution of NH₃ (0.5 mL) is added. The mixture is stirred for 30 min, evaporated to dryness, and the residue is suspended in water (2 mL), and centrifuged. The supernatant is discarded, and the solid is purified with preparative TLC on reverse-phase C18 plates, using 50% 2-PrOH and 0.2% TFA in H₂O as eluant to give compound **99** (4 mg, 48%), as an orange solid.

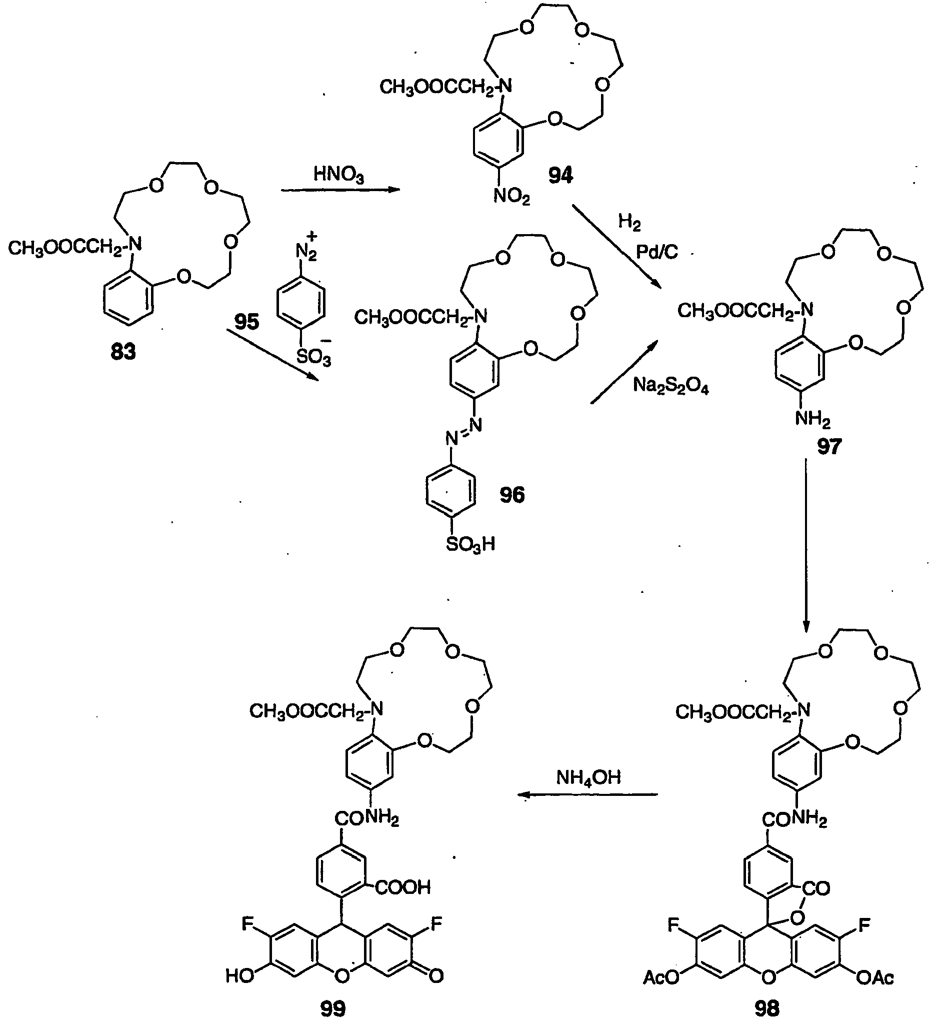

### Example 19: Preparation of 1-Aza-benzo-14-crown-4 ether (Compound 100).

To a solution of 2-aminophenol **81** (1.322 g, 12 mmol) in MeCN (1 L), powdered CsF (7.300 g, 48 mmol) is added. The mixture is stirred vigorously for 1 h and then triethyleneglycol ditosylate (5.503 g, 12 mmol) in MeCN (50 mL) is introduced. The mixture is refluxed under N₂ atmosphere for 16 h and then evaporated. The residue is dissolved in CHCl₃ (500 mL), washed with H₂O, sat. NaHCO₃, H₂O, sat. NaCl (200 mL each), filtered trough paper, and evaporated. The residue is purified by column chromatography on silica gel (12x60 cm bed column, made in CHCl₃) using CHCl₃ as eluant to give crude product, which is purified by column chromatography on silica gel (12x60 cm bed column, made in 25% EtOAc in hexanes) using the same mixture of solvents as eluant to give compound **100**, 0.370 g (14% yield) as an off-white solid.

### Example 20: Preparation of 1-Methoxycarbonylmethyl-1-aza-benzo-12-crown-4-ether (Compound 101).

The mixture of compound **100** (0.360 g, 1.61 mmol), DIEA (0.56 mL, 3.22 mmol), methyl bromoacetate (0.76 mL, 8.05 mmol), and Nal (0.241 g, 1.61 mmol; catalyst) in MeCN (30 mL) is refluxed under N₂ atmosphere for 16 h, then cooled down and evaporated. The residue is redissolved in CHCl₃ (200 mL), washed with 1% AcOH (2x200 mL), H₂O (200 mL). The chloroform solution is dried over MgSO₄, filtered through paper, and evaporated. The residue (TLC-pure crude product) is used in the next step without purification, considering it to be a 1.6 mmol quantity.

### Example 21: Preparation of 13-Formyl-1-methoxycarbonylmethyl-1-aza-benzo-12-crown-4-ether (Compound 102).

To a solution of the Vilsmeier reagent prepared from POCl₃ (0.75 mL, 8.0 mmol) in 8 mL DMF compound **101** (1.60 mmol) in DMF (2 mL) is introduced. The mixture is stirred under a N₂ atmosphere for 16 h, then poured into ice (20 g)/ sat. K₂CO₃ (50 mL) mixture. The mixture is extracted with CHCl₃ (100 + 6x10 mL); the extract is dried over MagSO₄, and evaporated. The crude product is purified by column chromatography on silica gel (1.5x30 cm bed column, made in CHCl₃) using CHCl₃ as eluant to give aldehyde **102**, 0.285 g (55% yield on two steps) as an off-white solid.

### Example 22: Preparation of compound 103.

A mixture of the aldehyde **102** (0.272 g, 0.88 mmol) and 4-fluoro resorcinol (0.248 g, 1.94 mmol) in MsOH (13 mL) is stirred for 24 h and then poured into 3N NaOAc (100 mL). The mixture is centrifuged, and the supernatant is discarded. The solid is washed with water, and dried in vacuum to give compound **103** (0.087 g, 18%) as an off-white solid. Crude compound **104** is used in the next step without purification.

### Example 23: Preparation of cell-impermeable compound 104.

A mixture of the dihydro compound **85** (0.085 g, 0.16 mmol), and freshly powdered chloranil (0.197 g, 0.80 mmol) in CHCl₃/MeOH 1:1 mixture (6 mL) is vigorously stirred during reflux for 4h, then cooled down, filtered from the excess oxidizer and evaporated. The residue is purified by preparative TLC on silica gel, using MeOH/AcOH 5%:2% in CHCl₃ as eluant to give the compound **104** (0.031 g, 36%) as an orange solid.

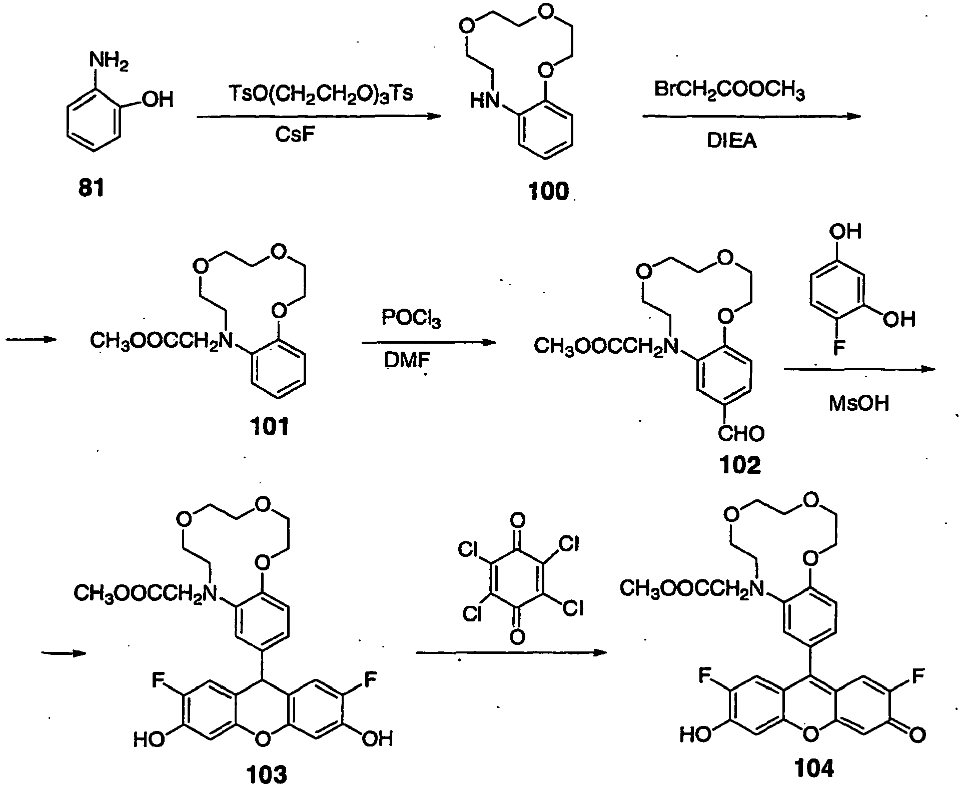

### Example 24: Preparation of 1-Aza-benzo-18-crown-6 ether (Compound 105).

To a solution of 2-aminophenol **81** (1.090 g, 10 mmol) in MeCN (1 L), powdered CsF (6.080 g, 40 mmol) is added. The mixture is stirred vigorously for 1 h and then pentaethyleneglycol ditosylate (5.500 g, 11 mmol) in MeCN (50 mL) is introduced. The mixture is refluxed under N₂ atmosphere for 70 h and then evaporated. The residue is dissolved in CHCl₃ (600 mL), washed with H₂O, sat. NaHCO₃, H₂O, and sat. NaCl (200 mL each). The chloroform solution is dried over MgSO₄, filtered trough paper, and evaporated. The residue is purified by column chromatography on silica gel (8x45 cm bed column, made in CHCl₃) using 0-1.2% MeOH gradient in CHCl₃ as eluant to give compound **105**, 0.681 g (22% yield) as a off-white solid.

### Example 25: Preparation of 1-Methoxycarbonylmethyl-1-aza-benzo-18-crown-6-ether (Compound 126).

A mixture of compound **105** (0.670 g, 2.15 mmol), DIEA (0.72 mL, 4.15 mmol), methyl bromoacetate (1.01 mL, 10.75 mmol), and Nal (0.322 g, 2.15 mmol; catalyst) in MeCN (40 mL) is refluxed under a N₂ atmosphere for 16 h, then cooled down and evaporated. The residue is redissolved in CHCl₃ (200 mL), washed with 1% AcOH (2x200 mL), H₂O (200 mL). Chloroform solution is dried over MgSO₄, filtered trough paper, and evaporated. The residue is purified by column chromatography on silica gel (3x30 cm bed column, made in CHCl₃) using 2.5 - 4% MeOH gradient in CHCl₃ as eluant to give compound **106**, 0.430 g (52% yield) as an off-white solid.

### Example 26: Preparation of 18-Formyl-1-methoxycarbonylmethyl-1-aza-benzo-18-crown-6-ether (Compound 107).

To a solution of the Vilsmeier reagent prepared from POCl₃ (1.23 mL, 13.2 mmol) in 6 mL DMF compound **106** (0.410g, 1.32 mmol) in DMF (2 mL) is introduced. The mixture is stirred under a N₂ atmosphere for 16 h, then poured into ice (40 g)/ sat. K₂CO₃ (100 mL) mixture. The mixture is extracted with CHCl₃ (5x100 mL), the extract is dried over MagSO₄, and evaporated. The crude product is treated with cold ether (10 mL), and the precipitated solid is collected to give aldehyde **107**, 0.246 g (45% yield) as a white solid.

### Example 27: Preparation of compound 108.

A mixture of the aldehyde **107** (0.236 g, 0.57 mmol) and 4-fluoro resorcinol (0.186 g, 1.45 mmol) in MsOH (8 mL) is stirred for 16 h, then poured into 3N NaOAc (100 mL). The precipitate is filtered, washed with water, and dried in vacuum to give compound **108** (0.350 g, 97%) as an off-white solid. Crude compound **108** is used in the next step without purification.

### Example 28: Preparation of Cell-impermeable compound 109.

A mixture of the dihydro compound **108** (0.350 g, 0.56 mmol), and freshly powdered chloranil (0.701 g, 2.85 mmol) in CHCl₃/MeOH 1:1 mixture (25 mL) is vigorously stirred upon reflux for 4 h, then cooled down, filtered from the excess oxidizer and evaporated. The residue is purified by column chromatography on silica gel, (4 x 40 cm bed column, made in 10% MeOH + 1% AcOH in CHCl₃) using the same mixture of solvents as eluant to give the compound **109** (0.088 g, 25%) as an orange solid.

### Example 29: Preparation of Cell-permeable Compound 110.

To a solution of compound **109** (32 mg, 0.05 mmol) and DIEA (0.17 mL, 1.0 mmol) in DMF (2 mL), bromomethyl acetate (50 microL, 0.5 mmol) is added. The mixture is stirred for 3 h and evaporated at 1 mm Hg vacuum. The residue is purified by preparative TLC on silica gel, using MeOH/AcOH 15%:1% in CHCl₃ as eluant to give the compound **110** (20 mg, 57%) as an orange solid.

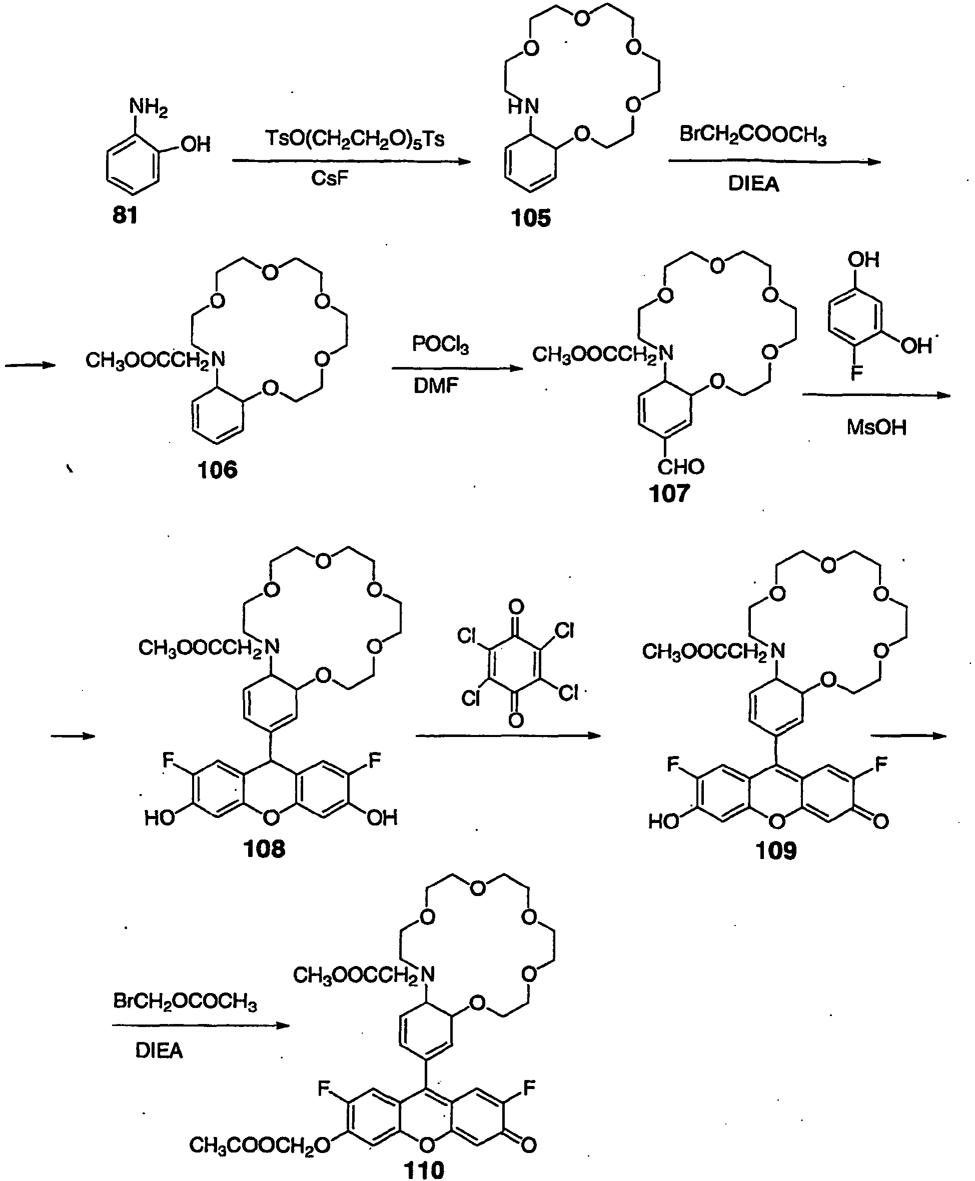

### Example 30: Preparation of 1-(2'-Methoxyacetyl)-1-aza-benzo-15-crown-5 ether (Compound 111).

To a stirred solution of the crown ether **82** (6.20 g, 23 mmol) and Et₃N (9.3 mL, 70 mmol) in CH₂Cl₂ (250 mL) a methoxyacetyl chloride (3.2 mL, 35 mml) in CH₂Cl₂ (15 mL) is introduced within 15 min. The mixture is stirred for 3 h, diluted with CHCl₃ (150 mL), and washed with H₂O, 1% AcOH, H₂O, sat. NaHCO₃, H₂O (200 mL each), filtered through paper filter and evaporated. The residue is purified by column chromatography on silica gel (6 x 45 cm bed column, made in CHCl₃), using 2.5% MeOH in CHCl₃ as eluant to give compound **111** (4.25 g, 55%) as a yellow oil.

### Example 31: Preparation of 1-(2'-Methoxyethyl)-1-aza-benzo-15-crown-5 ether (Compound 112).

A) To a stirred solution of compound **111** (4.25 g, 12.5 mmol) in dry THF (50 mL) a 1 N diborane in THF (50 mL, 50 mmol) is added. The mixture is stirred under reflux for 20 h, decomposed by dropwise addition of MeOH (50 mL), and evaporated. The residue is co-eveporated with MeOH (5 x 100 mL) to remove boron complexes, and dried in vacuo to give compound **112** (4.05 g, 99%) as a colorless oil.
B) A mixture of compound **82** (1.20g, 4.50 mmol), DEIA (1.6 mL, 9.0 mmol), 2-bromoethylmethyl, ether (4.2 mL, 45 mmol), Nal (0.68 g, 4.50 mmo, catalyst) in MeCN (200 mL) is stirred under reflux for 70h. More DIEA (1.6 mL, 9.0 mmol) and 2-bromoethylmethyl ether (4.2 mL, 45 mmol) are added and heating continued for another 40 h. The reaction mixture is evaporated, the residue is re-dissolved in CHCl₃ (300 mL), washed with 1% AcOH (3x200 mL), H₂O (200 mL), filtered through paper filter and evaporated. The residue is purified by column chromatography on silica gel (3 x 40 cm bed column, made in 2.5% MeOH in CHCl₃), using the same mixture of solvents as eluant to give compound 112 (0.080 g, 5%), identical to that prepared section A of this example.

### Example 32: Preparation of 1-(2'-Methoxyethyl)-15-formyl-1-aza-benzo-15-crown-5 ether (Compound 113).

To a solution of the Vilsmeier reagent prepared from POCl₃ (12 mL, 125 mmol) in 70 mL DMF compound **112** (4.05 g, 12.5 mmol) in DMF (10 mL) is introduced. The mixture is stirred under N₂ atmosphere for 16 h at 40 °C, then more Vilsmeier reagent from POCl₃ (12 mL, **125** mmol) in 70 mL DMF is added and the stirring continued for another 24 h. The mixture is poured into ice (400 g)/ sat. K₂CO₃ (400 mL) mixture. The mixture is extracted with CHCl₃ (300 + 6x50 mL), the extract is dried over MagSO₄, and evaporated. The crude product is purified by column chromatography on silica gel (6x50 cm bed column, made in CHCl₃) using 1.5% MeOH in CHCl₃ as eluant to give aldehyde **113**, 1.613 g (36% or, 58% based on recovery), then eluant is changed to 10% to recover starting material (1.49 g, 37%).

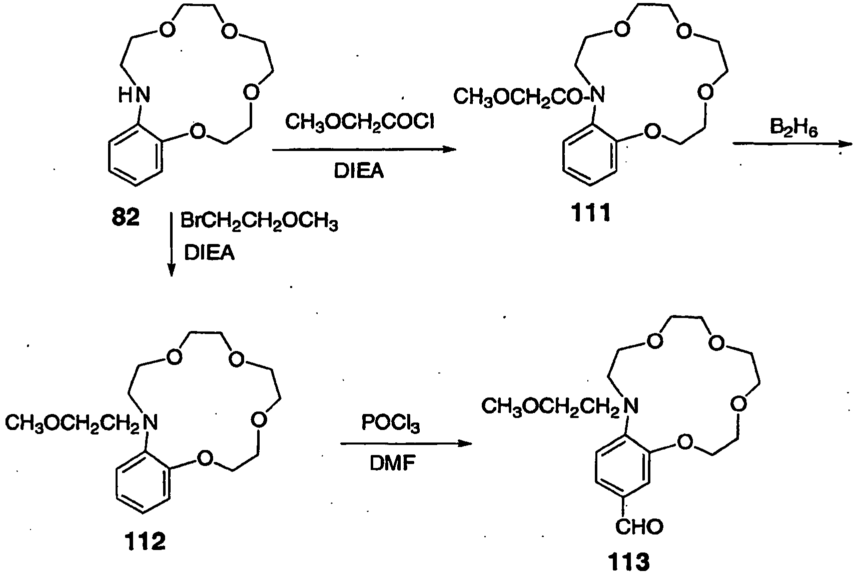

### Example 33: Preparation of compound 114.

A mixture of the aldehyde **113** (0.700 g, 1.98.mmol) and 4-fluororesorcinol (0.558 g, 4.36 mmol) in methanesulfonic acid (30 mL) is stirred for 24 h and poured into 3N NaOAc (300 mL). The mixture is extracted with n-BuOH (7 x 50 mL). The extract is evaporated to give the crude dihydro derivative **114**. Crude compound **114** is used in the next step without purification, considering it as 1.8 mmol.

### Example 34: Preparation of Call-impermeable Compound 115.

A mixture of the dihydro compound **114** (1.8 mmol), and freshly powdered chloranil (2.210 g, 9.00 mmol) in CHCl₃/MeOH 1:1 mixture (100 mL) is vigorously stirred upon reflux for 4 h, then cooled down, filtered from the excess oxidizer and evaporated. The residue is purified by column chromatography on silica gel (6 x 55 cm bed column, made in 10% MeOH + 1 % AcOH in CHCl₃), using 10-15%MeOH gradient in CHCl₃ + 1% AcOH as eluant to give the compound 115 (0.189 g, 18% for two steps) as a brown solid.

### Example 35: Preparation of Cell-permeable Compound 116.

To a solution of compound **115** (28 mg, 0.05 mmol) and DIEA (0.17, 1.0 mmol) in DMF (2 mL), bromomethyl acetate (50 µL, 0.5 mmol) is added. The mixture is stirred for 1 h and evaporated at 1 mm Hg vacuum. The residue is purified by preparative TLC on silica gel, using MeOH/AcOH 10%:2% in CHCl₃ as eluant to give the compound **116** (4 mg, 12%) as an orange solid.

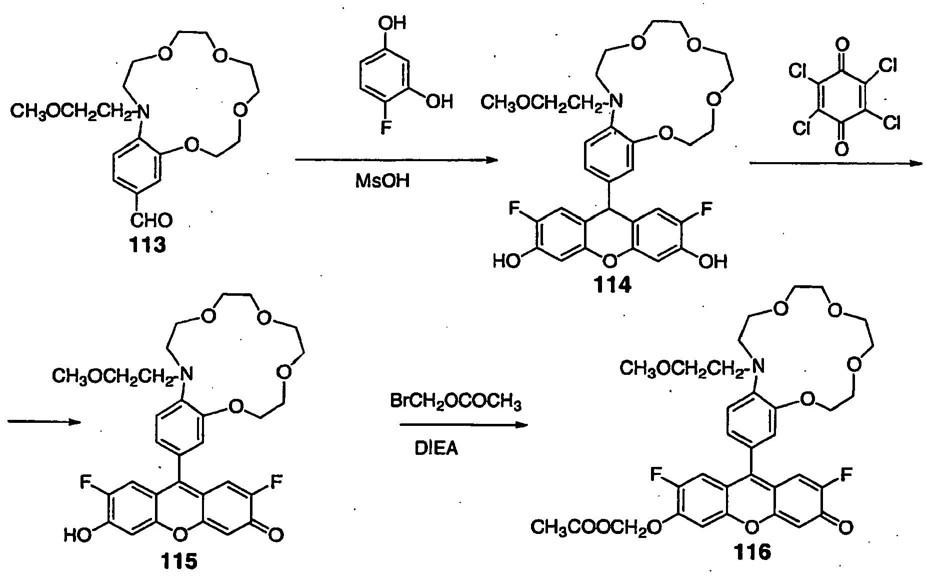

### Example 36: Preparation of Tetramethylrosamine compound 118.

A mixture of aldehyde **113** (0.900 g, 2.55 mmol), and 3-(N,N-dimethylamino)phenol (0.769 g, 5.61 mmol), and TsOH (50 mg, catalyst) in EtCOOH (25 mL) is stirred for 16 h at 65°C. The mixture is cooled down and poured into 3N NaOAc (500 mL). The resulting suspension is extracted with CHCl₃ (200 + 7x30 mL). The extract is filtered through paper and evaporated. The resulting crude dihydro compound **117** is re-dissolved in MeOH/CHCl₃ 1:1 mixture (100 mL) and treated with chloranil (0.541 g, 2.20 mmol). The oxidation is continued upon vigorous stirring for 2 h, then the solvents are evaporated, the residue is re-dissolved in CHCl₃, and purified by column chromatography on silica get (8 x 50 cm bed column, made with 5% MeOH + 1% AcOH in CHCl₃) using 5-20% MeOH gradient in CHCl₃+1.0% AcOH as eluant to give compound **118** (0.236 g, 16% on two steps) as a dark red solid.

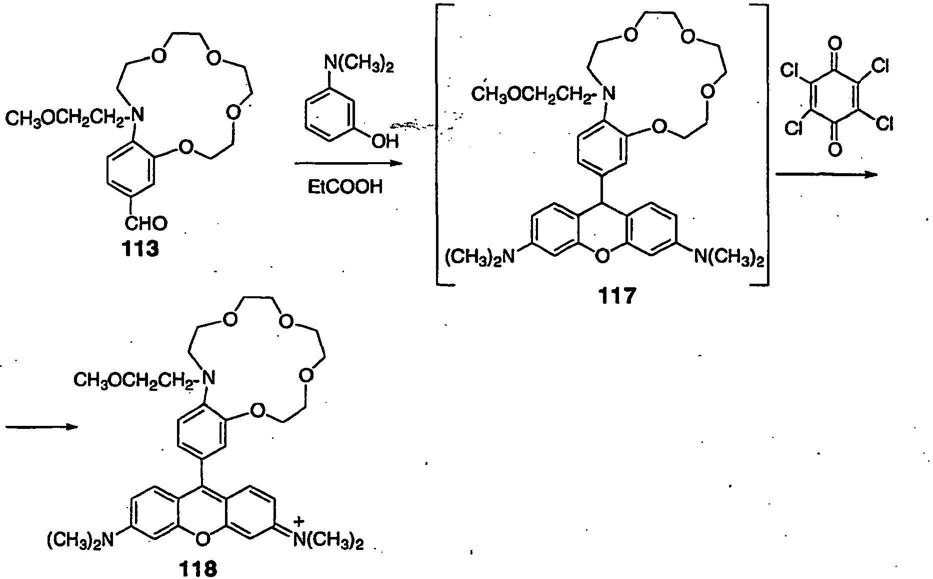

### Example 37: Synthesis of an α-nitrostilbene (Compound 119)

A mixture of aldehyde **113** (0.172 g, 0.49 mmol), Wittig salt **91** (0.392 g, 0.73 mmol), and K₂CO₃ (0.338 g, 2.45 mmol) in DMF (3 mL) is stirred for 6 h at 90°C, then left overnight at rt. The mixture is poured into H₂O (200 mL), and the resulting suspension is extracted with CHCl₃ (50 + 7x20 mL. The extract is evaporated to dryness at 3 mm Hg and the residue is purified by column chromatography on silica gel (2.5 x 30 cm bed column, prepared in 3% MeOH in CHCl₃) using the same mixture of solvents as eluant to give stilbene **119** (0.195g, 75%) as a dark red low-melting solid.

### Example 38: Preparation of Compound 120.

A solution of stilbene **119** (0.190 g, 0.36 mmol) in P(OEt)₃ (3 mL) is heated at 125 °C for 4 h, then evaporated. The residue is purified by column chromatography on silica gel (2.5 x 50 cm bed column, prepared in 5% MeOH and 1% AcOH in CHCl₃) using the same mixture of solvents as eluant to give compound **120** (0.099 g, 55%) as an off-white solid.

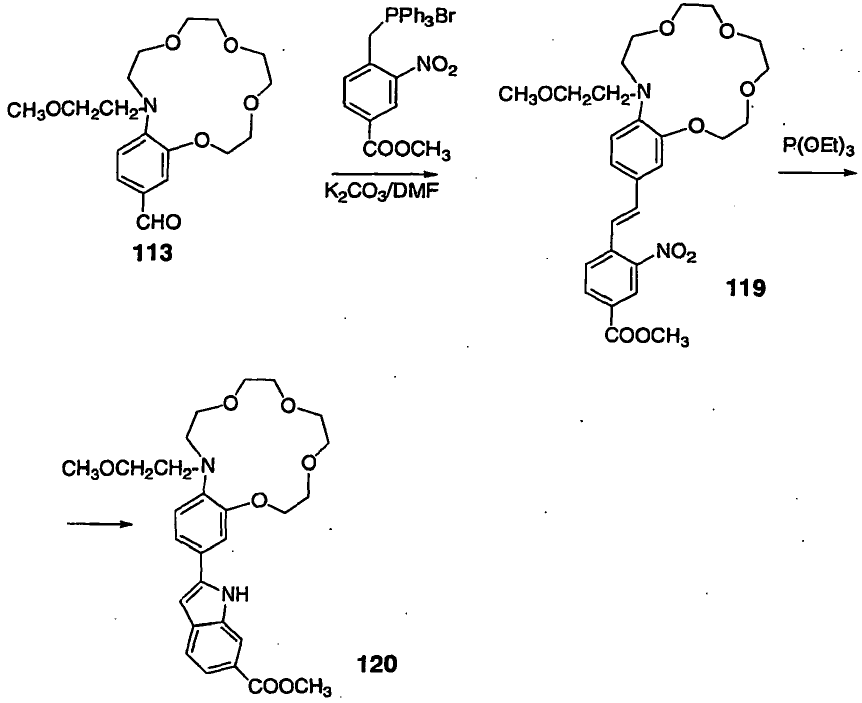

### Example 39: Preparation of 1-Ethoxyoxalyl-1-aza-benzo-15-crown-5-ether (Compound 121).

To a mixture of compound **82** (1.345 g, 5.00 mmol) and Et₃N (1.4 mL, 10.00 mmol) in CH₂Cl₂ (30 mL), ethyl oxalylchloride (0.84 mL, 7.50 mmol) in CH₂Cl₂ (5 mL) is added dropwise. The mixture is stirred for 1 h, diluted with CHCl₃ (200 mL), washed with 1% AcOH (3 x 100 mL), H₂O (100 mL), sat. NaHCO₃ (2 x 100 mL), sat. NaCl (200 mL). The chloroform fraction is dried over MgSO₄ and evaporated. The residue is purified by column chromatography on silica gel (4 x 30 cm bed, made in 30% EtOAc in hexanes), using 30-60% EtOAc gradient in hexanes as eluant to give compound **121** (1.170 g, 65% yield) as a yellow oil.

### Example 40: Preparation of 1-(N,N-Dimethylaminooxalyl)-1-aza-benzo-15-crown-5-ether (Compound 122).

To a solution of the compound **121** (0.734 g, 2.00 mmol) in MeOH (15 mL), a solution of dimethylamine (2 mL, 40 mmol) in cold MeOH (15 mL) is added upon cooling to 0 °C. The mixture is heated to 30 °C for 16 h, then cooled to 0 °C and a new portion of dimethylamine (2 mL, 40 mmol) is introduced. The mixture is stirred for 3 h at 30 °C and then evaporated and the residue is treated with cold ether. The precipitated solid is filtered off, and washed with ether to give compound **122** (0.542 g, 74% yield) as a white solid.

### Example 41: Preparation of 1-(2'-N,N-Dimethylaminoethyl)-1-aza-benzo-15-crown-5-ether (Compound 123).

To the solution of compound **122** (0.440 g, 1.20 mmol) in THF (10 ml) a solution of 1 N diborane in THF (12 mL, 12 mml) is added. The mixture is refluxed for 16 h, then decomposed by careful addition of MeOH (30 mL), evaporated, and co-evaporated with MeOH (5 x 50 mL) to remove boron complexes. The crystalline residue of the compound **123** (0.405 g, 100% yield) is used in next step without purification.

### Example 42: Preparation of 1-(2'-N,N-Dimethylaminoethyl)-15-formyl-1-aza-benzo-15-crown-5-ether (Compound 124).

To a solution of the Vilsmeier reagent prepared from POCl₃ (1.5 mL, 16.0 mmol) in 5 mL DMF, compound **123** (0.550 g, 1.62 mmol) in DMF (2 mL) is introduced. The mixture is stirred under N₂ atmosphere for 16 h, then poured into ice (20 g)/ sat. K₂CO₃ (50 mL) mixture. The mixture is extracted with CHCl₃ (100 + 7x25 mL), the extract is dried over MagSO₄, and evaporated. The crude product is purified by column chromatography on silica gel (3x35 cm bed column, made with 3% MeOH in CHCl₃) using the same mixture of solvents as eluant to give aldehyde **124**, (0.175 g 30% yield) as a yellow low-melting solid.

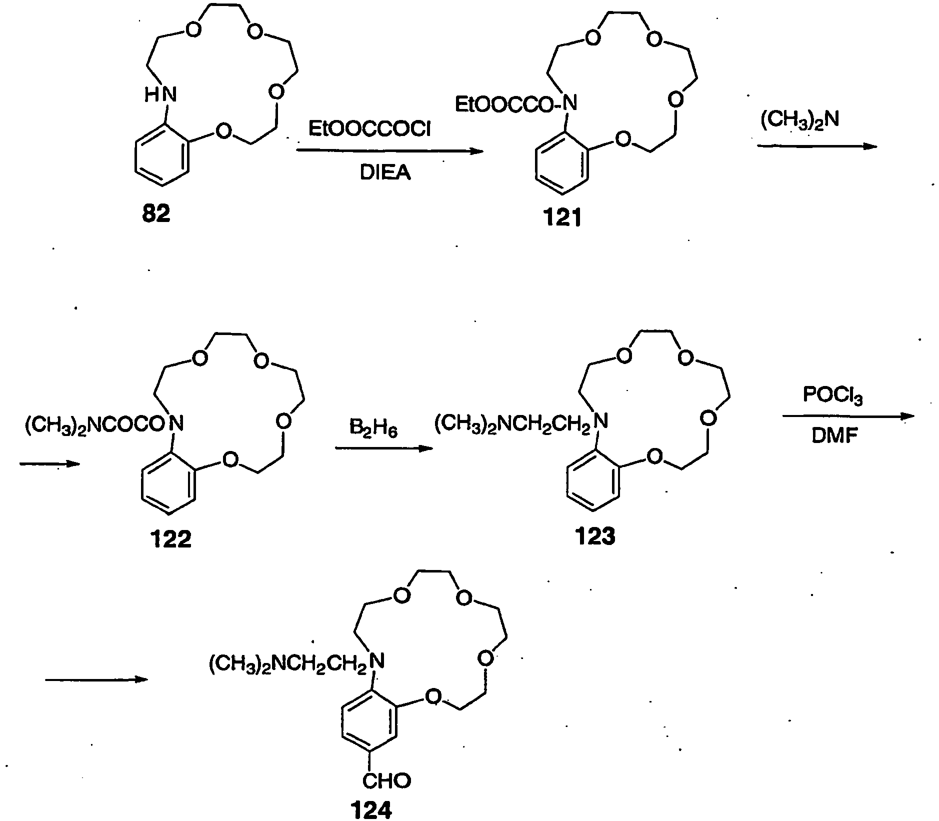

### Example 43: Synthesis of α-nitrostilbene (Compound 125).

A mixture of aldehyde 124 (0.070 g, 0.19 mmol), Wittig salt 91 (0.154 g, 0.29 mmol), and K₂CO₃ (0.131 g, 0.95 mmol) in DMF (2 mL) is stirred for 16 h at 90 °C. The mixture is diluted with CHCl₃ (10 mL), filtered from inorganic materials and evaporated to dryness at 3 mm Hg. The residue is purified by preparative TLC on silica gel using 20% MeOH and 5% AcOH in CHCl₃ as eluant to give stilbene 125 (0.060 g, 58%) as a dark red solid.

### Example 44: Preparation of Compound 126

A solution of stilbene 119 (0.055 g, 0.01 mmol) in P(OEt)₃ (2 mL) is heated at 125 °C for 14 h, then evaporated. The residue is purified by preparative TLC on silica gel using 25% MeOH and 5% AcOH in CHCl₃ as eluant to give compound **126** (0.029 g, 57%) as an off-white solid.

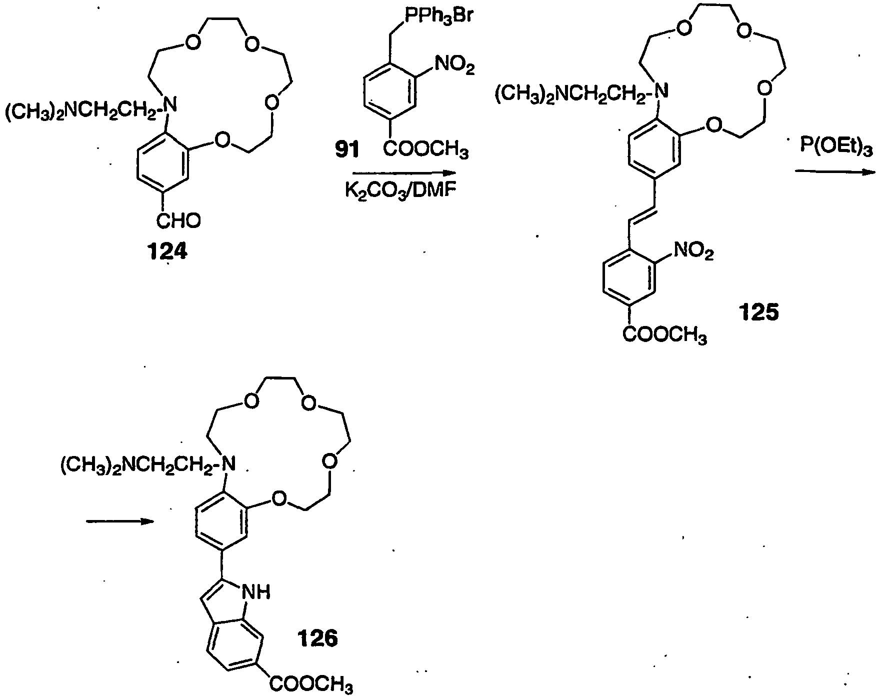

### Example 45: Hydrolysis of compound 86.

To a solution of the compound **86** (0.032 g, 0.055 mmol) in 50% MeOH (2 mL), 1 N KOH (0.16 mL, 16 mml) is added. The mixture is stirred for 16 h, diluted with H₂O, and 0.2 N HCl is added to achieve pH=8.0. The resulting solution is filtered through a nylon membrane filter and evaporated. The crude product is purified by column chromatography on Sephadex LH-20 (2.6 x 50 cm bed column, made with H₂O), using H₂O as eluant to give compound **127** (0.021 mg, 59 % yield) as a brown solid.

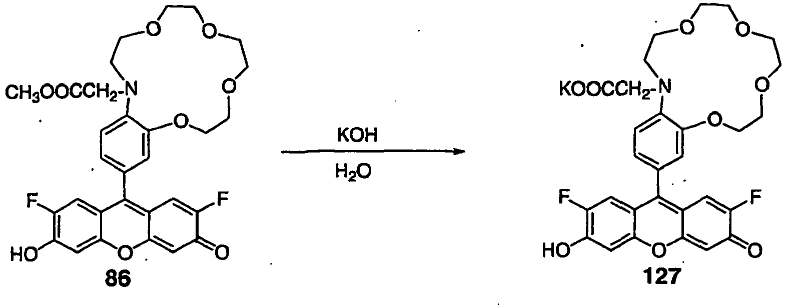

### Example 46: Preparation of Compound 138

To a solution of compound **128** in dry THF (0.5M) and two equivalents of DIEA is added dropwise benzoyl chloride (1.0 eq). After 1 h at room temperature, the volatiles are removed. The residue is partitioned between 0.1M HCl and ethyl acetate. The organic layer is washed with brine, dried over sodium sulfate, and concentrated to give amide **129**.

To a solution of **129** in THF (0.5M) is added a solution of borane-THF (1.0M, 2 eq) at room temperature. The resulting solution is stirred at room temperature over night and then methanol (2 eq) is carefully added. The volatiles are removed in vacuo, and the residue partitioned between water and ethyl acetate. The organic layer is dried over sodium sulfate and concentrated to give aniline **130**, which is purified further if necessary by flash chromatography on silica gel using ethyl acetate in hexanes.

To a solution of **130** (0.05M) in dry THF with 2 eq DIEA is slowly added a 0.1 M solution of diglycolyl chloride in dry THF with rapid stirring. After 2h, the reaction solution is concentrated in vacuo. The residue is partitioned between 0.1 M HCl and ethyl acetate. The organic layer is washed with brine, dried over sodium sulfate, and concentrated to give bisamide **131**.

To a 0.1 M solution of **131** in dry THF is slowly added borane-THF (1.0M, 5 eq). The resulting solution is stirred at room temperature overnight and then methanol (5 eq) is added. The resulting mixture is concentrated in vacuo, and the residue partitioned between water and ethyl acetate. The organic phase is washed with brine and dried over sodium sulfate, and concentrated in vacuo to give the diol **132**, which is purified further if necessary by flash chromatography on silica gel using methanol in chloroform.

A solution of **132** is dissolved in dry acetonitrile to 0.5M. The resulting solution is treated with methanesulfonyl chloride (1.2 eq) and cesium carbonate (2 eq). The resulting mixture is stirred at reflux for 6 hours, then cooled and filtered and concentrated in vacuo. The desired azacrown ether **133** is formed as a minor product, isolated from the residue by flash chromatography on silica gel using ethyl acetate in hexanes.

The crown **133** is stirred in ethyl acetate (0.5M) with 10 wt% Pd/C (cat.) under 30 psi hydrogen gas for 6 hours. After filtration, the reaction solution is concentrated in vacuo to give aniline **134**.

To a solution of **134** in DMF (0.5M) is added methyl bromoacetate (5 eq) and DIEA (3 eq). The resulting solution is stirred at 90°C overnight, then cooled and concentrated. The residue is partitioned between 0.1 M HCl and ethyl acetate. The organic layer is washed with brine and dried over sodium sulfate, then concentrated in vacuo to give crown **135**, which is purified further if necessary by flash chromatography on silica gel using ethyl acetate in hexanes.

To a solution of Vilsmeyer reagent made from 2 eq phosphorous oxychloride in DMF is added a solution of **135** in DMF. The resulting solution is stirred at room temperature overnight, then poured into aq. sodium bicarbonate. The resulting mixture is extracted with ethyl acetate. The extract is washed with water and brine, dried over sodium sulfate, and concentrated in vacuo to give aldehyde **136**, which is purified further if necessary by crystallization from methanol.

To a solution of **136** in methanesulfonic acid (0.5M) is added 4-fluororesorcinol (2 eq). The resulting solution is stirred at room temperature 15 minutes, then poured carefully into 3M aqueous NaOAc. The resulting precipitate is collected by suction filtration, rinsed with water, and dried in vacuo to give dihydro compound **137** as a pale brown powder.

A solution of **137** in methanol/chloroform (1:1, 0.5M) is treated with 2 eq of para-chloranil. The resulting mixture is stirred at room temperature overnight, then filtered and concentrated. The residue is purified by chromatography on silica gel using methanol in chloroform to give indicator **138** as a dark orange powder.

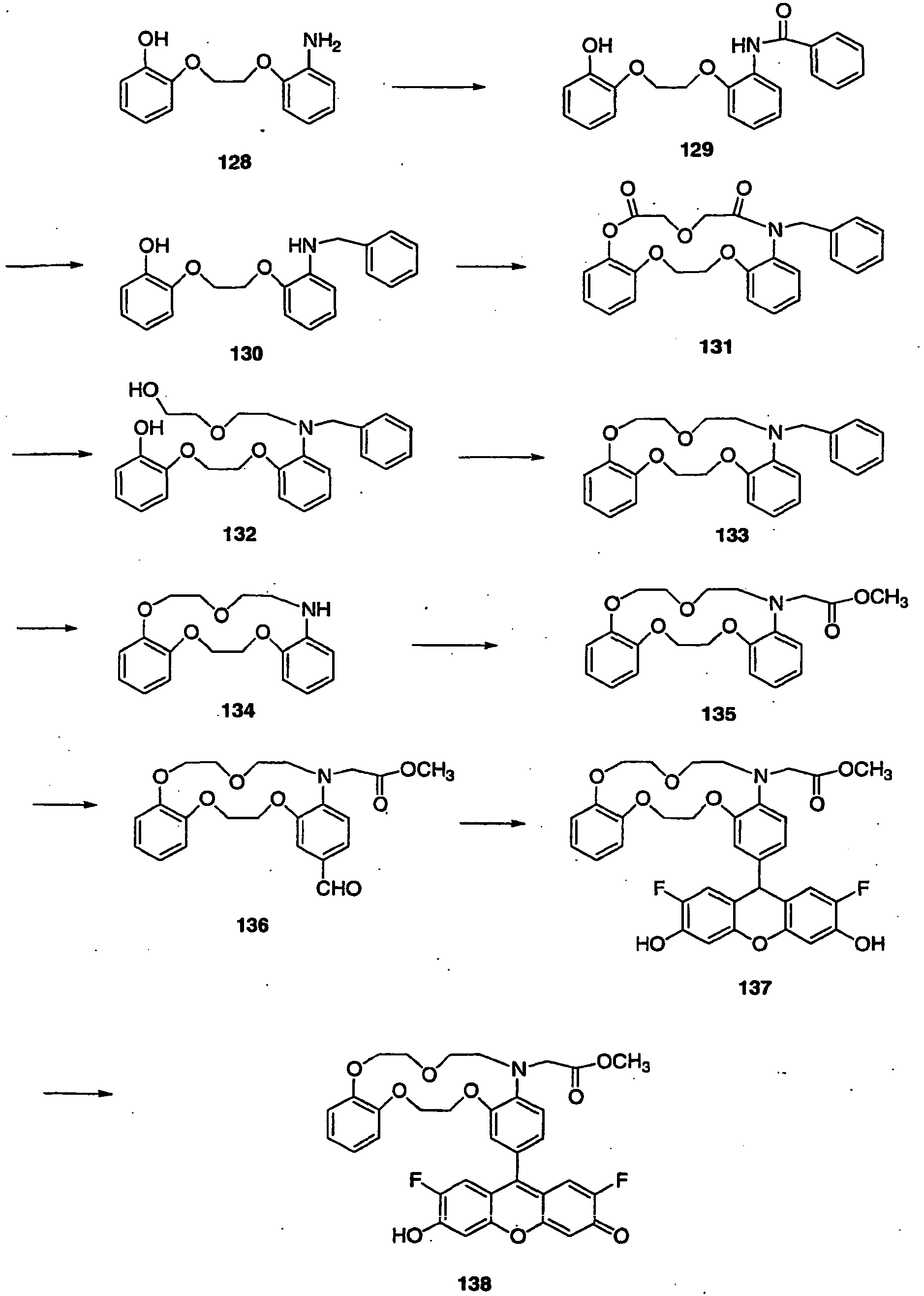

### Example 47: Syntheis of Compound 140

A 0.5M solution of compound **86** in 1:1 methanol/dioxane is treated at room temperature with 5 equivalents of tetrabutylammonium hydroxide. After 2 hours the volatiles are removed in vacuo and the residue is dissolved in water. The pH is lowered to 2.0 by dropwise addition of aqueous HCl. The resulting precipitate is collected by filtration and dried in vacuo to give carboxylic acid **139**. A 0.5M solution of compound **139** in dry DMF is treated with 1.5 equivalents of O-(N-succinimidyl)-N,N,N',N'-tetramethyluronium tetrafluoroborate. After 2 hours the reaction soluted is diluted 10x with diethyl ether. The resulting precipitate is collected by filtration to give reactive succinimidyl ester compound **140.**

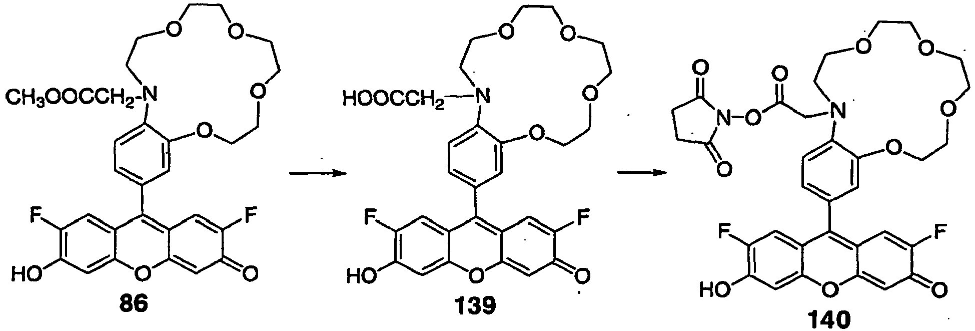

### Example 48: Synthesis of Compound 141

A 0.1 M solution of compound **140** in DMSO is added to a 0:1 M solution of an equivalent mass of aminodextran (average MW 10000) in 0.3M sodium bicarbonate solution. The resulting solution is stirred at rt overnight, then slowly diluted with 10x methanol. The resulting precipitate is collected by centrifugation to give conjugated substance **141**, which can be purified further if necessary by gel filtration chromatography using water and Sephadex G-15.

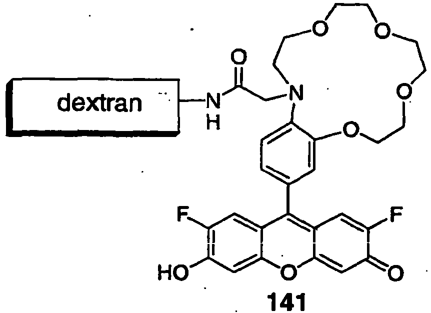

## Claims

1. A crown ether chelating compound having formula: 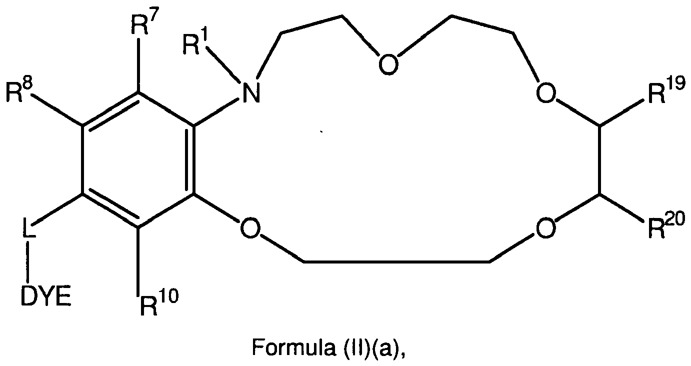 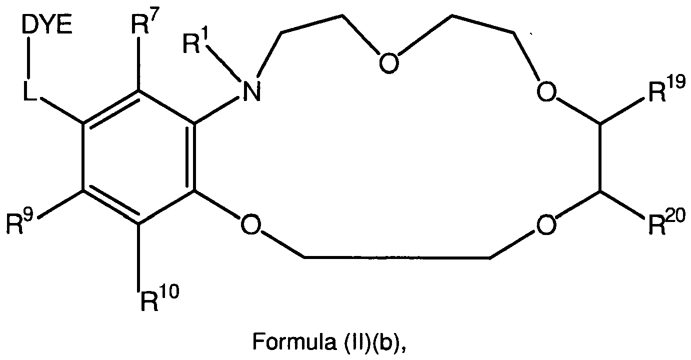 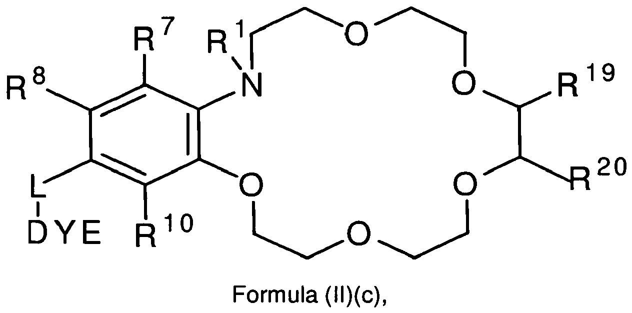 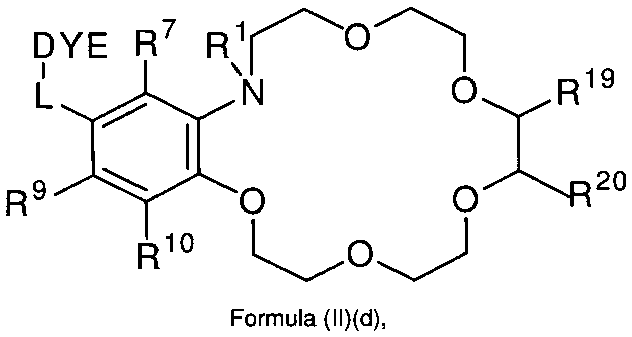 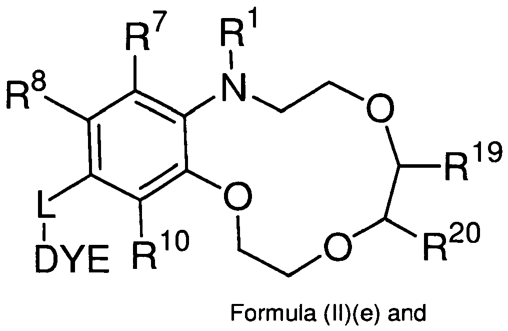 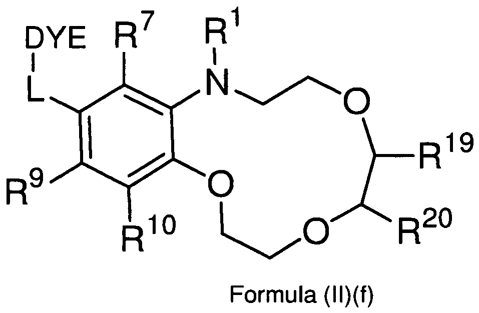 wherein
R¹ is C₁-C₆ alkyl that is substituted one or more times by amino (-NR¹⁷R¹⁸), -(C=O)-O-R¹⁶ or -(C=O)-NR¹⁷R¹⁸;
R⁷, R⁸, R⁹, and R¹⁰, when present, are H;
R¹⁹ and R²⁰ are both H;
R¹⁶ is selected from the group consisting of H, C₁-C₆ alkyl, benzyl, a biologically compatible esterifying group, a biologically compatible salt, -L-Rₓ, -L-S_{c} and -L-DYE;
R¹⁷ and R¹⁸ are independently selected from the group consisting of H, C₁-C₆ alkyl, C₁-C₆ carboxyalkyl, alpha-acyloxyalkyl, trialkylsilyl, a biologically compatible salt, -L-Rₓ, -L-S_{c} and -L-DYE; or R¹⁷ and R¹⁸ taken in combination form a 5- or 6-membered aliphatic ring that optionally incorporates an oxygen atom;
each L is independently a covalent linkage;
each Rₓ is independently a reactive group;
each S_{c} is independently a conjugated substance; and
each DYE is independently a reporter molecule.

2. The compound according to Claim 1, wherein L is a single covalent bond, or a covalent linkage that is linear or branched, cyclic or heterocyclic, saturated or unsaturated, having 1-20 nonhydrogen atoms selected from the group consisting of C, N, P, O and S; and are composed of any combination of ether, thioether, amine, ester, carboxamide, sulfonamide, hydrazide bonds and aromatic or heteroaromatic bonds.

3. The compound according to Claim 1, wherein -Rx is selected from the group consisting of an acrylamide, an activated ester of a carboxylic acid, a carboxylic ester, an acyl azide, an acyl nitrile, an aldehyde, an alkyl halide, an anhydride, an aniline, an amine, an aryl halide, an azide, an aziridine, a boronate, a diazoalkane, a haloacetamide, a halotriazine, a hydrazine, an imido ester, an isocyanate, an isothiocyanate, a maleimide, a phosphoramidite, a reactive platinum complex, a silyl halide, a sulfonyl halide, a thiol and a photoactivatable group, and optionally wherein -Rx is selected from the group consisting of carboxylic acid, succinimidyl ester of a carboxylic acid, hydrazide, amine and a maleimide.

4. The compound according to Claim 1, wherein -Sc is selected from the group consisting of an amino acid, a peptide, a protein, a polysaccharide, a nucleoside, a nucleotide, an oligonucleotide, a nucleic acid, a hapten, a psoralen, a drug, a hormone, a lipid, a lipid assembly, a synthetic polymer, a polymeric microparticle, a biological cell or a virus, and optionally wherein -Sc is selected from the group consisting of an antibody or fragment thereof, an avidin or streptavidin, a biotin, a blood component protein, a dextran, an enzyme, an enzyme inhibitor, a hormone, an igG binding protein, a fluorescent protein, a growth factor, a lectin, a lipopolysaccharide, a microorganism, a metal binding protein, a metal chelating moiety, a non-biological microparticle, a peptide toxin, a phosphotidylserine-binding protein, a structural protein, a small-molecule drug, or a tyramide.

5. The compound according to Claim 1, wherein, in the group R¹, said C₁-C₆ alkyl is methyl or ethyl.

6. The compound according to Claim 5 wherein R¹⁶ is selected from H, C₁-C₆ alkyl, benzyl, a biologically compatible esterifying group, and a biologically compatible salt.

7. The compound according to Claim 6 wherein R¹⁶ is methyl.

8. The compound according to Claim 1, wherein R¹⁷ and R¹⁸ are each methyl.

9. A compound of Claim 1 having formula: 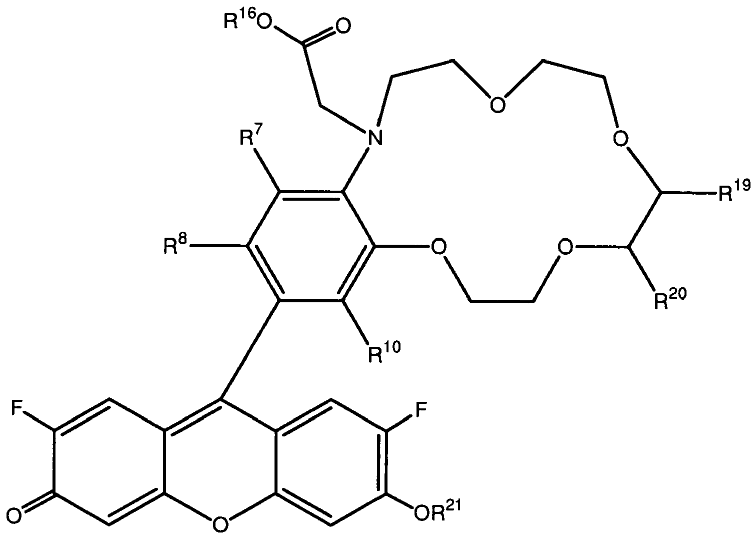 wherein
R⁷, R⁸, R¹⁰, R¹⁶, R¹⁷, R¹⁸, L, Rₓ, S_{c}, DYE, R¹⁹ and R²⁰ are as defined in Claim 1;
R²¹ is selected from the group consisting of H, C₁-C₁₈ alkyl, C₇-C₁₈ arylalkyl and a lipophilic group wherein each alkyl is optionally substituted by -(C=O)-R¹⁵, -(C=O)-O-R¹⁶, or C₁-C₆ alkoxy;
R¹⁵ is selected from the group consisting of H, C₁-C₆ alkyl, -L-Rₓ, -L-S_{C} and -L-DYE;
and optionally wherein R¹⁶ is methyl, a biologically compatible esterifying group or a biologically compatible salt.

10. The compound according to any preceding claim, wherein -DYE is selected from the group consisting of xanthene, borapolyazaindacene, carbocyanine, benzofuran, quinazolinone, indole, a benzazole, oxazine, and coumarin.

11. The compound according to Claim 10, wherein said -DYE moiety is independently substituted by a lipophilic group.

12. The compound according to Claim 11, wherein said lipophilic group is an AM or acetate ester.

13. A composition comprising:
a. a compound according to any one of Claims 1-12; and,
b. a metal ion that is capable of being chelated by said compound.

14. The composition according to claim 13, wherein the metal ion is Na⁺, Li⁺, K⁺, Ca²⁺, Zn²⁺ or Rb⁺, e.g. is Na⁺.

15. A method for binding and detecting target ions in a live cell, comprising:
a) contacting a sample of live cells with a crown ether compound according to any one of Claims 1-12 with the proviso that said compound comprises a DYE moiety and at least one lipohilic group;
b) incubating said sample and said crown ether chelate compound for sufficient time to allow said compound to chelate said target metal ion; and,
c) illuminating said sample with an appropriate wavelength whereby said target ion is detected in a live cell.

16. The method according to Claim 15, wherein said DYE moiety is substituted by a lipophilic group.

17. The method according to Claim 16 wherein said lipophilic group is an AM or acetate ester.

18. The method according to any one of claims 15 to 17, wherein the metal ion is Na⁺, Li⁺, K⁺, Ca²⁺, Zn²⁺ or Rb⁺, e.g. is Na⁺.

19. A kit for binding a metal ion in a sample, comprising:
a compound according to any one of Claims 1-12; and, comprising one or more components selected from the group consisting of a calibration standard of a metal ion, an ionophore, a fluorescent standard, an aqueous buffer solution and an organic solvent.

20. A kit according to claim 19, wherein the metal ion is Na⁺, Li⁺, K⁺, Ca²⁺, Zn²⁺ or Rb⁺, e.g. is Na⁺.

21. The use of a compound of any of Claims 1 to 12 as a metal ion chelator.

22. The use of Claim 21 wherein the compound is used as a chelator of in vivo metal ions and it comprises a DYE moiety substituted by a lipophilic group, e.g. an AM ester or an acetate ester.

23. The use according to claim 21 to 22 wherein the metal ion is Na⁺, Li⁺, K⁺, Ca²⁺, Zn²⁺ or Rb⁺, e.g. is Na⁺.

## Patentansprüche

1. Kronenether-Chelatbildnerverbindung mit der Formel: 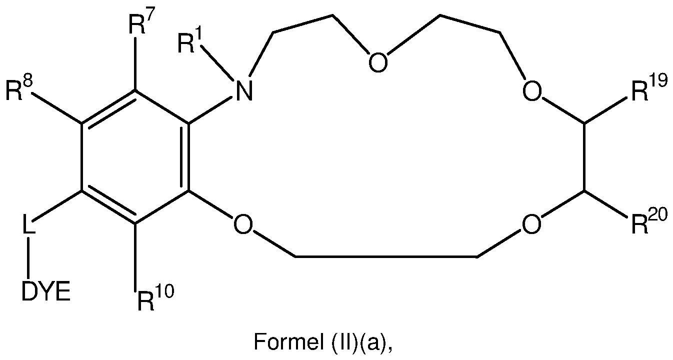 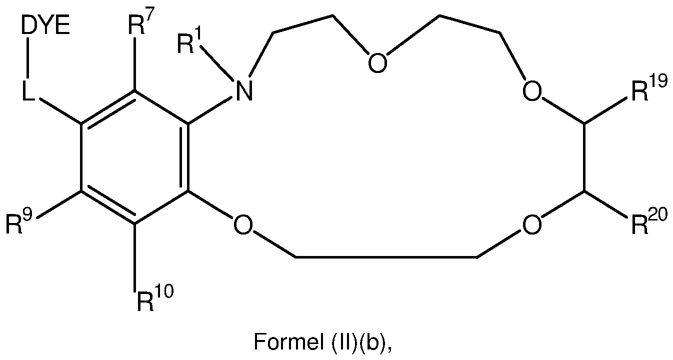 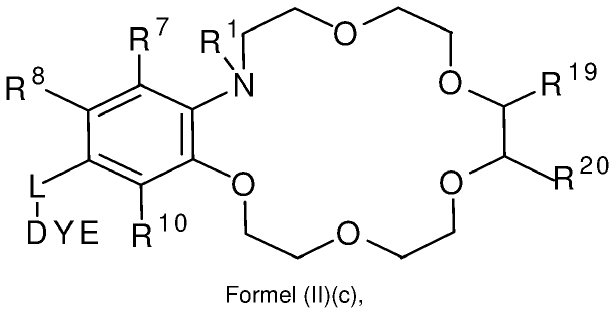 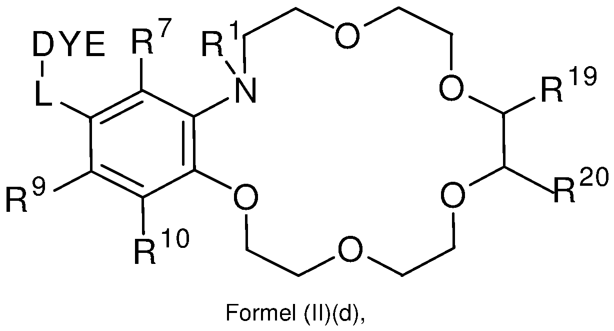 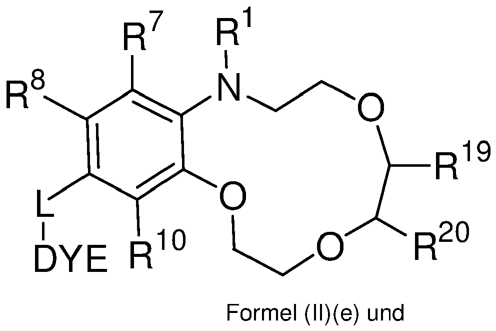 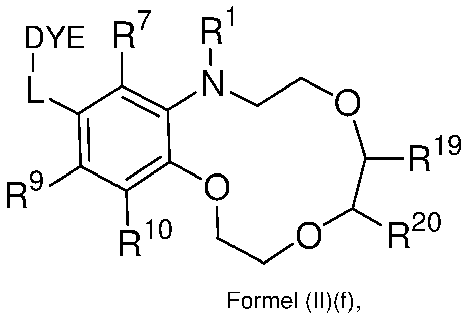 wobei
R¹ C₁-C₆-Alkyl ist, das einfach oder mehrfach durch Amino (-NR¹⁷R¹⁸), -(C=O)-O-R¹⁶ oder - (C=O)-NR¹⁷R¹⁸ substituiert ist,
R⁷, R⁸, R⁹ und R¹⁰, wenn vorhanden, H sind;
R¹⁹ und R²⁰ beide H sind;
R¹⁶ aus der Gruppe bestehend aus H, C₁-C₆-Alkyl, Benzyl, einer biologisch kompatiblen Veresterungsgruppe, einem biologisch kompatiblen Salz, -L-Rₓ, -L-S_{c} und -L-DYE ausgewählt ist;
R¹⁷ und R¹⁸ unabhängig aus der Gruppe bestehend aus H, C₁-C₆-Alkyl, C₁-C₆-Carboxyalkyl, alpha-Acyloxyalkyl, Trialkylsilyl, einem biologisch kompatiblen Salz, -L-R_{X}, -L-S_{C} und -L-DYE ausgewählt ist; oder R¹⁷ und R¹⁸ zusammengenommen einen 5- oder 6-gliedrigen aliphatischen Ring bilden, der gegebenenfalls ein Sauerstoffatom einbindet;
jedes L unabhängig eine kovalente Verknüpfung ist;
jedes Rₓ unabhängig eine reaktive Gruppe ist;
jedes S_{c} unabhängig eine konjugierte Substanz ist und
jedes DYE unabhängig ein Reportermolekül ist.

2. Verbindung nach Anspruch 1, wobei L eine einzige kovalente Bindung oder eine kovalente Verknüpfung ist, die linear oder verzweigt, cyclisch oder heterocyclisch, gesättigt oder ungesättigt ist, mit 1 - 20 Nicht-Wasserstoffatomen, die aus der Gruppe bestehend aus C, N, P, O und S ausgewählt sind; und die sich aus einer beliebigen Kombination von Ether-, Thioether-, Amin-, Ester-, Carboxamid-, Sulfonamid-, Hydrazidbindungen und aromatischen oder heteroaromatischen Bindungen zusammensetzen.

3. Verbindung nach Anspruch 1, wobei -Rₓ aus der Gruppe bestehend aus einem Acrylamid, einem aktivierten Ester einer Carbonsäure, einem Carbonsäureester, einem Acylazid, einem Acylnitril, einem Aldehyd, einem Alkylhalogenid, einem Anhydrid, einem Anilin, einem Amin, einem Arylhalogenid, einem Azid, einem Aziridin, einem Boronat, einem Diazoalkan, einem Halogenacetamid, einem Halogentriazin, einem Hydrazin, einem Imidoester, einem Isocyanat, einem Isothiocyanat, einem Maleinimid, einem Phosphoramidit, einem reaktiven Platinkomplex, einem Silylhalogenid, einem Sulfonylhalogenid, einem Thiol und einer photoaktivierbaren Gruppe ausgewählt ist und gegebenenfalls wobei -Rₓ aus der Gruppe bestehend aus Carbonsäure, Succinimidylester einer Carbonsäure, Hydrazid, Amin und einem Maleinimid ausgewählt ist.

4. Verbindung nach Anspruch 1, wobei -S_{c} aus der Gruppe bestehend aus einer Aminosäure, einem Peptid, einem Protein, einem Polysaccharid, einem Nukleosid, einem Nukleotid, einem Oligonukleotid, einer Nukleinsäure, einem Hapten, einem Psoralen, einem Wirkstoff, einem Hormon, einem Lipid, einer Lipidanordnung, einem synthetischen Polymer, einem polymeren Mikroteilchen, einer biologischen Zelle oder einem Virus ausgewählt ist und gegebenenfalls wobei -S_{c} aus der Gruppe bestehend aus einem Antikörper oder einem Fragment davon, einem Avidin oder Streptavidin, einem Biotin, einem Blutkomponentenprotein, einem Dextran, einem Enzym, einem Enzymhemmer, einem Hormon, einem IgG-Bindeprotein, einem fluoreszierenden Protein, einem Wachstumsfaktor, einem Lektin, einem Lipopolysaccharid, einem Mikroorganismus, einem Metallbindeprotein, einer Metallchelatbildnereinheit, einem nicht biologischen Mikroteilchen, einem Peptidtoxin, einem Phosphotidylserin-Bindeprotein, einem Strukturprotein, einem Kleinmolekül-Wirkstoff oder einem Tyramid ausgewählt ist.

5. Verbindung nach Anspruch 1, wobei das C₁-C₆-Methyl in der Gruppe R¹ Methyl oder Ethyl ist.

6. Verbindung nach Anspruch 5, wobei R¹⁶ aus H, C₁-C₆-Alkyl, Benzyl, einer biologisch kompatiblen Veresterungsgruppe und einem biologisch kompatiblen Salz ausgewählt ist.

7. Verbindung nach Anspruch 6, wobei R¹⁶ Methyl ist.

8. Verbindung nach Anspruch 1, wobei R¹⁷ und R¹⁸ jeweils Methyl sind.

9. Verbindung nach Anspruch 1 mit der Formel: 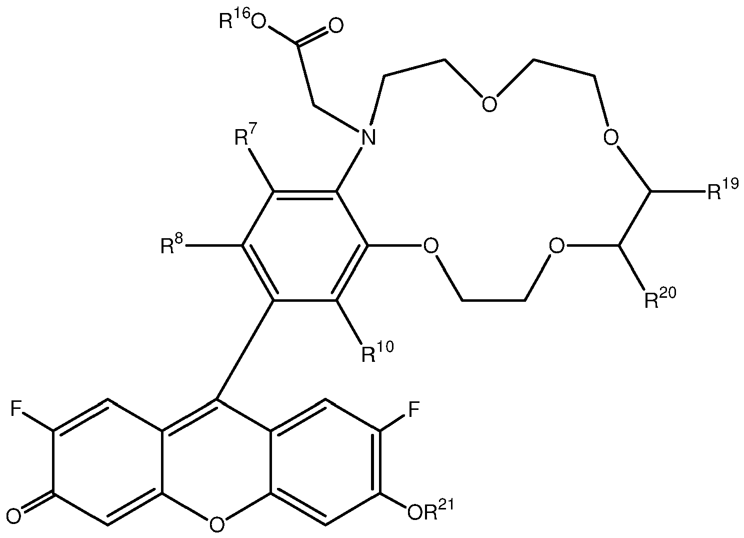 wobei
R⁷, R⁸, R¹⁰, R¹⁶, R¹⁷, R¹⁸, L, R_{X}, S_{C} DYE, R¹⁹ und R²⁰ wie in Anspruch 1 definiert sind;
R²¹ aus der Gruppe bestehend aus H, C₁-C₁₈-Alkyl, C₇-C₁₈-Arylalkyl und einer lipophilen Gruppe ausgewählt ist, wobei jedes Alkyl gegebenenfalls durch -(C=O)-R¹⁵, -(C=O)-O-R¹⁶ oder C₁-C₆-Alkoxy substituiert ist;
R¹⁵ aus der Gruppe bestehend aus H, C₁-C₆-Alkyl, -L-Rₓ, -L-S_{c} und -L-DYE ausgewählt ist
und gegebenenfalls wobei R¹⁶ Methyl, eine biologisch kompatible Veresterungsgruppe oder ein biologisch kompatibles Salz ist.

10. Verbindung nach einem der vorhergehenden Ansprüche, wobei -DYE aus der Gruppe bestehend aus Xanthen, Borapolyazaindacen, Carbocyanin, Benzofuran, Chinazolinon, Indol, einem Benzazol, Oxazin oder Cumarin ausgewählt ist.

11. Verbindung nach Anspruch 10, wobei die -DYE-Einheit unabhängig durch eine lipophile Gruppe substituiert ist.

12. Verbindung nach Anspruch 11, wobei die lipophile Gruppe ein AM- oder Acetatester ist.

13. Zusammensetzung, die Folgendes umfasst:
a. eine Verbindung nach einem der Ansprüche 1 - 12 und
b. ein Metallion, das von der Verbindung chelatiert werden kann.

14. Zusammensetzung nach Anspruch 13, wobei das Metallion Na⁺, Li⁺, K⁺, Ca²⁺, Zn²⁺ oder Rb⁺ ist, z. B. Na⁺ ist.

15. Verfahren zum Binden und Nachweisen von Zielionen in einer lebenden Zelle, wobei das Verfahren Folgendes umfasst:
a) Inkontaktbringen einer Probe von lebenden Zellen mit einer Kronenetherverbindung nach einem der Ansprüche 1 - 12 mit der Maßgabe, dass die Verbindung eine DYE-Einheit und mindestens eine lipophile Gruppe umfasst;
b) Inkubieren der Probe und der Kronenether-Chelatverbindung für einen ausreichenden Zeitraum, um zu ermöglichen, dass die Verbindung mit dem Zielmetallion chelatiert; und
c) Anstrahlen der Probe mit einer geeigneten Wellenlänge, wodurch das Zielion in einer lebenden Zelle nachgewiesen wird.

16. Verfahren nach Anspruch 15, wobei die DYE-Einheit durch eine lipophile Gruppe substituiert ist.

17. Verfahren nach Anspruch 16, wobei die lipophile Gruppe ein AM- oder Acetatester ist.

18. Verfahren nach einem der Ansprüche 15 bis 17, wobei das Metallion Na⁺, Li⁺, K⁺, Ca²⁺, Zn²⁺ oder Rb⁺ ist, z. B. Na⁺ ist.

19. Kit zum Binden eines Metallions in einer Probe, wobei das Kit Folgendes umfasst:
eine Verbindung nach einem der Ansprüche 1 - 12; und eine oder mehrere Komponenten umfasst, die aus der Gruppe bestehend aus einem Eichstandard eines Metallions, einem lonophor, einem Fluoreszenzstandard, einer wässrigen Pufferlösung und einem organischen Lösungsmittel ausgewählt sind.

20. Kit nach Anspruch 19, wobei das Metallion Na⁺, Li⁺, K⁺, Ca²⁺, Zn²⁺ oder Rb⁺ ist, z. B. Na⁺ ist.

21. Verwendung einer Verbindung nach einem der Ansprüche 1 bis 12 als ein Metallionenchelator.

22. Verwendung nach Anspruch 21, wobei die Verbindung als ein Chelator von *In-vivo-*Metallionen verwendet wird und eine DYE-Einheit umfasst, die durch eine lipophile Gruppe, z. B. einen AM-Ester oder einen Acetatester, substituiert ist.

23. Verwendung nach den Ansprüchen 21 bis 22, wobei das Metallion Na⁺, Li⁺, K⁺, Ca²⁺, Zn²⁺ oder Rb⁺ ist, z. B. Na⁺ ist.

## Revendications

1. Composé chélatant éther couronne ayant la formule : 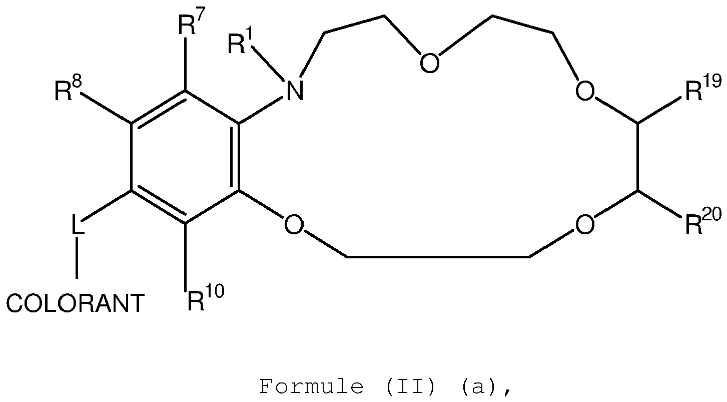 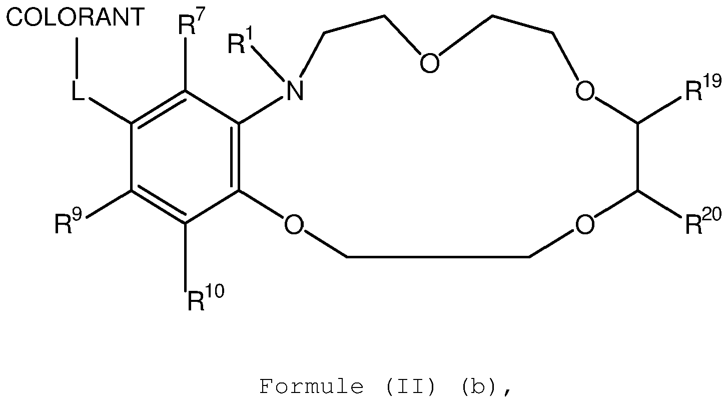 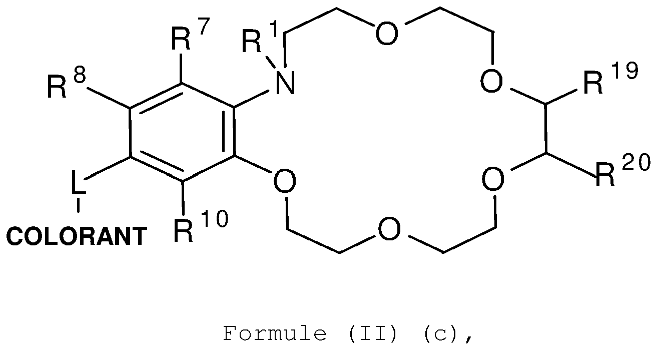 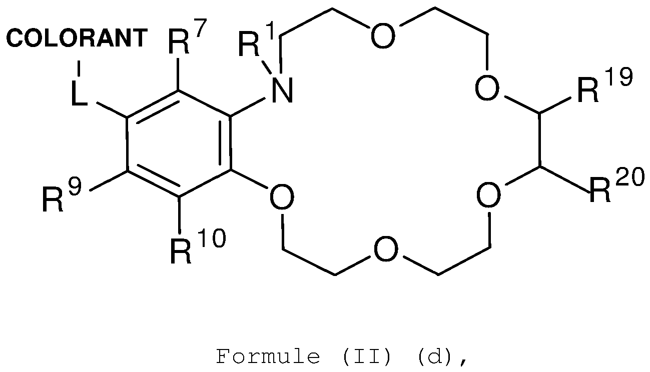 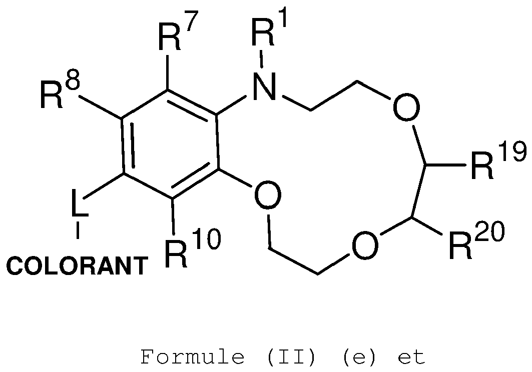 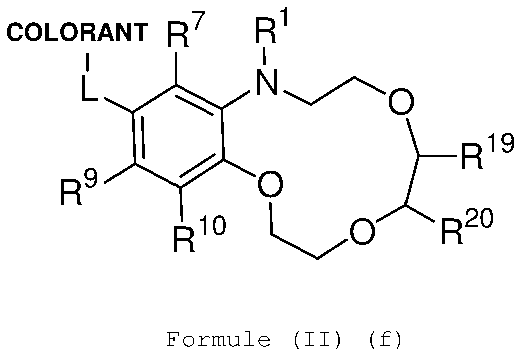 dans lesquelles
- R¹ représente alkyle en C₁-C₆ qui est substitué une ou plusieurs fois par amino (-NR¹⁷R¹⁸) , - (C=O) -O-R¹⁶ ou - (C=O) -NR¹⁷R¹⁸ _{;}
- R⁷, R⁸, R⁹ et R¹⁰, lorsqu'ils sont présents, représentent H ;
- R¹⁹ et R²⁰ représentent tous les deux H ;
- R¹⁶ est choisi dans le groupe consistant en H, alkyle en C₁-C₆, benzyle, un groupe estérifiant biologiquement compatible, un sel biologiquement compatible, -L-Rₓ, -L-S_{c} et -L-COLORANT ;
- R¹⁷ et R¹⁸ sont indépendamment choisis dans le groupe consistant en H, alkyle en C₁-C₆, carboxyalkyle en C₁-C₆, alpha-acyloxyalkyle, trialkylsilyle, un sel biologiquement compatible, -L-Rₓ, -L-S_{c} et -L-COLORANT ; ou R¹⁷ et R¹⁸ pris en combinaison forment un noyau aliphatique à 5 ou 6 chaînons qui incorpore facultativement un atome d'oxygène ;
- chaque L représente indépendamment une liaison covalente ;
- chaque Rₓ représente indépendamment un groupe réactif ;
- chaque S_{c} représente indépendamment une substance conjuguée ; et
- chaque COLORANT représente indépendamment une molécule rapporteuse.

2. Composé selon la revendication 1, dans lequel L représente une simple liaison covalente, ou une liaison covalente qui est linéaire ou ramifiée, cyclique ou hétérocyclique, saturée ou insaturée, ayant 1-20 atomes de non hydrogène choisis dans le groupe consistant en C, N, P, O et S ; et est composé de toute combinaison de liaisons éther, thioéther, amine, ester, carboxamide, sulfonamide, hydrazide et de liaisons aromatiques ou hétéro-aromatiques.

3. Composé selon la revendication 1, dans lequel -Rx est choisi dans le groupe consistant en un acrylamide, un ester activé d'un acide carboxylique, un ester carboxylique, un acyl azide, un acyl nitrile, un aldéhyde, un halogénure d'alkyle, un anhydride, une aniline, une amine, un halogénure d'aryle, un azide, une aziridine, un boronate, un diazoalcane, un haloacétamide, une halotriazine, une hydrazine, un imido ester, un isocyanate, un isothiocyanate, un maléimide, un phosphoramidite, un complexe de platine réactif, un halogénure de silyle, un halogénure de sulfonyle, un thiol et un groupe photoactivable, et facultativement dans lequel -Rx est choisi dans le groupe consistant en acide carboxylique, ester succinimidylique d'un acide carboxylique, hydrazide, amine et un maléimide.

4. Composé selon la revendication 1, dans lequel -S_{c} est choisi dans le groupe consistant en un acide aminé, un peptide, une protéine, un polysaccharide, un nucléoside, un nucléotide, un oligonucléotide, un acide nucléique, un haptène, un psoralène, un médicament, une hormone, un lipide, un assemblage lipidique, un polymère de synthèse, une microparticule polymère, une cellule biologique ou un virus, et facultativement dans lequel -S_{c} est choisi dans le groupe consistant en un anticorps ou un fragment de celui-ci, une avidine ou une streptavidine, une biotine, une protéine de composant sanguin, un dextrane, une enzyme, un inhibiteur d'enzyme, une hormone, une protéine de liaison aux IgG, une protéine fluorescente, un facteur de croissance, une lectine, un lipopolysaccharide, un microorganisme, une protéine de liaison à un métal, une fraction chélatant un métal, une microparticule non biologique, une toxine peptidique, une protéine de liaison à la phosphatidylsérine, une protéine structurale, un médicament à petites molécules ou un tyramide.

5. Composé selon la revendication 1, dans lequel, dans le groupe R¹, ledit alkyle en C₁-C₆ est méthyle ou éthyle.

6. Composé selon la revendication 5, dans lequel R¹⁶ est choisi parmi H, alkyle en C₁-C₆, benzyle, un groupe estérifiant biologiquement compatible et un sel biologiquement compatible.

7. Composé selon la revendication 6, dans lequel R¹⁶ représente méthyle.

8. Composé selon la revendication 1, dans lequel R¹⁷ et R¹⁸ représentent chacun méthyle.

9. Composé selon la revendication 1 ayant la formule : 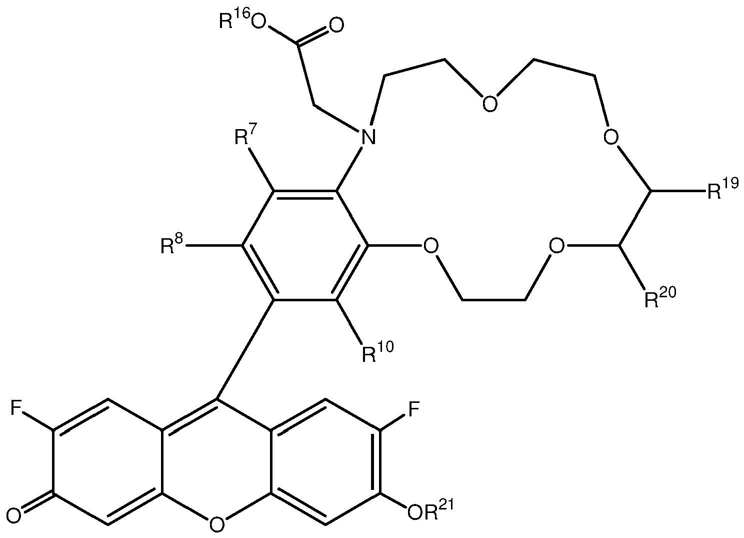 dans laquelle
- R⁷, R⁸, R¹⁰, R¹⁶, R¹⁷, R¹⁸, L, R_{X}, S_{c}, COLORANT, R¹⁹ et R²⁰ sont tels que définis dans la revendication 1 ;
- R²¹ est choisi dans le groupe consistant en H, alkyle en C₁-C₁₈, arylalkyle en C₇-C₁₈ et un groupe lipophile dans lequel chaque alkyle est facultativement substitué par -(C=O)-R¹⁵, -(C=O)-O-R¹⁶ ou alcoxy en C₁-C₆ ;
- R¹⁵ est choisi dans le groupe consistant en H, alkyle en C₁-C₆, -L-Rₓ, -L-S_{c} et -L-COLORANT ;
- et facultativement dans laquelle R¹⁶ représente méthyle, un groupe estérifiant biologiquement compatible ou un sel biologiquement compatible.

10. Composé selon l'une quelconque des revendications précédentes, dans lequel -COLORANT est choisi dans le groupe consistant en xanthène, borapolyazaindacène, carbocyanine, benzofurane, quinazolinone, indole, un benzazole, oxazine et coumarine.

11. Composé selon la revendication 10, dans lequel ladite fraction -COLORANT est indépendamment substituée par un groupe lipophile.

12. Composé selon la revendication 11, dans lequel ledit groupe lipophile est un AM ester ou un ester acétate.

13. Composition comprenant :
a. un composé selon l'une quelconque des revendications 1-12 ; et
b. un ion métallique qui est capable d'être chélaté par ledit composé.

14. Composition selon la revendication 13, dans laquelle l'ion métallique est Na⁺, Li⁺, K⁺, Ca²⁺, Zn²⁺ ou Rb⁺, par exemple est Na⁺.

15. Procédé de liaison et de détection d'ions cibles dans une cellule vivante, comprenant :
a) la mise en contact d'un échantillon de cellules vivantes avec un composé éther couronne selon l'une quelconque des revendications 1-12 à la condition que ledit composé comprenne une fraction COLORANT et au moins un groupe lipophile ;
b) l'incubation dudit échantillon et dudit composé chélate éther couronne pendant un temps suffisant pour permettre audit composé de chélater ledit ion métallique cible ; et,
c) l'éclairement dudit échantillon avec une longueur d'onde appropriée, ce par quoi ledit ion cible est détecté dans une cellule vivante.

16. Procédé selon la revendication 15, dans lequel ladite fraction COLORANT est substituée par un groupe lipophile.

17. Procédé selon la revendication 16, dans lequel ledit groupe lipophile est un AM ester ou un ester acétate.

18. Procédé selon l'une quelconque des revendications 15 à 17, dans lequel l'ion métallique est Na⁺, Li⁺, K⁺, Ca²⁺, Zn²⁺ ou Rb⁺, par exemple est Na⁺.

19. Kit de liaison d'un ion métallique dans un échantillon, comprenant :
- un composé selon l'une quelconque des revendications 1-12 ; et,
- comprenant au moins un composant choisi dans le groupe consistant en un standard d'étalonnage d'un ion métallique, un ionophore, un standard fluorescent, une solution tampon aqueuse et un solvant organique.

20. Kit selon la revendication 19, dans lequel l'ion métallique est Na⁺, Li⁺, K⁺, Ca²⁺, Zn²⁺ ou Rb⁺, par exemple est Na⁺.

21. Utilisation d'un composé selon l'une quelconque des revendications 1 à 12 en tant que chélateur d'ions métalliques.

22. Utilisation selon la revendication 21, dans laquelle le composé est utilisé en tant que chélateur d'ions métalliques *in vivo* et il comprend une fraction COLORANT substituée par un groupe lipophile, par exemple un AM ester ou un ester acétate.

23. Utilisation selon les revendications 21 et 22, dans laquelle l'ion métallique est Na⁺, Li⁺, K⁺, Ca²⁺, Zn²⁺ ou Rb⁺, par exemple est Na⁺.
